(19) Europäisches Patentamt
European Patent Office
Office européen des brevets

(11) **EP 4 435 497 A2**

(12) **EUROPÄISCHE PATENTANMELDUNG**

(43) Veröffentlichungstag:
**25.09.2024   Patentblatt 2024/39**

(51) Internationale Patentklassifikation (IPC):
***G02C 7/02*** *(2006.01)*

(21) Anmeldenummer: 24186008.9

(52) Gemeinsame Patentklassifikation (CPC):
**G02C 7/027; G02C 7/028; G16H 20/40;
G16H 50/50;** A61B 3/0025; A61B 3/1005;
A61B 3/107

(22) Anmeldetag: **24.01.2018**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **27.01.2017   DE 102017000772
23.08.2017   DE 102017007975
23.08.2017   DE 102017007990**

(62) Dokumentnummer(n) der früheren Anmeldung(en)
nach Art. 76 EPÜ:
**18706394.6 / 3 574 367**

(71) Anmelder: **Rodenstock GmbH
80687 München (DE)**

(72) Erfinder:
• **TRUMM, Stephan
80999 München (DE)**
• **BECKEN, Wolfgang
82061 Neuried (DE)**

• **ALTHEIMER, Helmut
87650 Baisweil-Lauchdorf (DE)**
• **MUSCHIELOK, Adam
83714 Miesbach (DE)**
• **BÉNARD, Yohann
81541 München (DE)**
• **ESSER, Gregor
80686 München (DE)**
• **MÜLLER, Werner
75443 Ötisheim (DE)**

(74) Vertreter: **Müller-Boré & Partner
Patentanwälte PartG mbB
Friedenheimer Brücke 21
80639 München (DE)**

Bemerkungen:
Diese Anmeldung ist am 02.07.2024 als
Teilanmeldung zu der unter INID-Code 62 erwähnten
Anmeldung eingereicht worden.

(54) **BELEGUNG EINES AUGENMODELLS ZUR OPTIMIERUNG VON BRILLENGLÄSERN MIT MESSDATEN**

(57)    Computerimplementiertes Verfahren zum Ermitteln individueller Aberrationsdaten zumindest eines Auges eines Brillenträgers für die Berechnung oder Optimierung und Herstellung eines Brillenglases für das zumindest eine Auge des Brillenträgers umfassend: Bereitstellen einer gemessenen Hornhauttopographie des zumindest einen Auges des Brillenträgers; Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

FIG 1

**Beschreibung**

Die vorliegende Erfindung betrifft in einem ersten Ansatz ein Verfahren, eine Vorrichtung und ein entsprechendes Computerprogrammerzeugnis zum Ermitteln relevanter individueller Parameter zumindest eines Auges eines Brillenträgers für die Berechnung oder Optimierung eines Brillenglases für das zumindest eine Auge des Brillenträgers bzw. ein entsprechendes Verfahren, eine Vorrichtung und ein Computerprogrammerzeugnis zur Berechnung (Optimierung) und Herstellung eines Brillenglases mit Hilfe eines teilweise individuellen Augenmodells. In einem zweiten Ansatz betrifft die vorliegende Erfindung ein Verfahren, eine Vorrichtung und ein entsprechendes Computerprogrammerzeugnis zur Berechnung (Optimierung) und Herstellung eines Brillenglases mit Hilfe eines teilweise individuellen Augenmodells.

Für die Herstellung bzw. Optimierung von Brillengläsern, insbesondere von individuellen Brillengläsern wird jedes Brillenglas so gefertigt, dass für jede gewünschte Blickrichtung oder jeden gewünschten Objektpunkt eine möglichst gute Korrektur eines Refraktionsfehlers des jeweiligen Auges des Brillenträgers erreicht wird. Im Allgemeinen gilt ein Brillenglas für eine gegebene Blickrichtung dann als vollkorrigierend, wenn die Werte für Sphäre, Zylinder und Achse der Wellenfront beim Passieren der Scheitelpunktkugel mit den Werten für Sphäre, Zylinder und Achse der Verordnung für das fehlsichtige Auge übereinstimmen. Bei der Refraktionsbestimmung für ein Auge eines Brillenträgers werden dioptrische Werte (insbesondere Sphäre, Zylinder, Achslage - also insbesondere sphäro-zylindrische Abweichungen) für eine weite (in der Regel unendliche) Entfernung und gegebenenfalls (für Mehrstärkengläser bzw. Gleitsichtgläser) eine Addition oder eine vollständige Nahrefraktion für eine nahe Entfernung (z.B. nach DIN 58208) bestimmt. Bei modernen Brillengläsern können zusätzlich auch von der Norm abweichende Objektentfernungen, die bei der Refraktionsbestimmung verwendet wurden, angegeben werden. Damit ist die Verordnung (insbesondere Sphäre, Zylinder, Achslage und gegebenenfalls Addition oder Nachrefraktion) festgelegt, die an einen Brillenglashersteller übermittelt wird. Kenntnisse über eine spezielle bzw. individuelle Anatomie des jeweiligen Auges oder die tatsächlich im Einzelfall vorliegenden Brechwerte des fehlsichtigen Auges sind dafür nicht erforderlich.

Eine vollständige Korrektur für alle Blickrichtungen gleichzeitig ist aber im Normalfall nicht möglich. Daher werden die Brillengläser derart gefertigt, dass sie vor allem in den hauptsächlichen Nutzungsbereichen, insbesondere in den zentralen Durchblicksbereichen eine gute Korrektur von Fehlsichtigkeiten des Auges und nur geringe Abbildungsfehler bewirken, während in peripheren Bereichen größere Abbildungsfehler zugelassen werden.

Um ein Brillenglas derart fertigen zu können, erfolgt zunächst eine Berechnung der Brillenglasflächen bzw. zumindest einer der Brillenglasflächen derart, dass dadurch die gewünschte Verteilung der unvermeidlichen Abbildungsfehler bewirkt wird. Diese Berechnung und Optimierung erfolgt üblicherweise mittels eines iterativen Variationsverfahrens durch Minimieren einer Zielfunktion. Als Zielfunktion wird insbesondere eine Funktion F mit folgendem funktionalen Zusammenhang zur sphärischen Wirkung S, zum Betrag der zylindrischen Wirkung Z und zur Achslage des Zylinders $\alpha$ (auch als "SZA"-Kombination bezeichnet) berücksichtigt und minimiert:

$$F = \sum_{i=1}^{m} \left[ g_{i,S\Delta} \left( S_{\Delta,i} - S_{\Delta,i,Soll} \right)^2 + g_{i,Z\Delta} \left( Z_{\Delta,i} - Z_{\Delta,i,Soll} \right)^2 + ... \right]$$

Dabei werden in der Zielfunktion F an den Bewertungsstellen *i* des Brillenglases zumindest die tatsächlichen Refraktionsdefizite der sphärischen Wirkung $S_{\Delta,i}$ und der zylindrischen Wirkung $Z_{\Delta,i}$ sowie Sollvorgaben für die Refraktionsdefizite der sphärischen Wirkung $S_{\Delta,i,Soll}$ und der zylindrischen Wirkung $Z_{\Delta,i,Soll}$ berücksichtigt.

Bereits in DE 103 13 275 wurde erkannt, dass es vorteilhaft ist, die Sollvorgaben nicht als absolute Werte der zu optimierenden Eigenschaften sondern als deren Abweichungen von der Verordnung, also als geforderte lokale Fehlanpassung anzugeben. Dies hat den Vorteil, dass die Sollvorgaben unabhängig von der Verordnung ( $Sph_V, Zyl_V, Achse_V, Pr_V, B_V$ ) sind und die Sollvorgaben nicht für jede individuelle Verordnung geändert werden müssen. Als "Ist"-Werte der zu optimierenden Eigenschaften fließen in die Zielfunktion somit auch nicht absolute Werte dieser optischen Eigenschaften, sondern die Abweichungen von der Verordnung ein. Dies hat den Vorteil, dass die Sollvorgaben unabhängig von der Verordnung vorgegeben werden können und nicht für jede individuelle Verordnung geändert werden müssen.

Die jeweiligen Refraktionsdefizite an den jeweiligen Bewertungsstellen werden vorzugsweise mit Gewichtungsfaktoren $g_{iS\Delta}$ bzw. $g_{iZ,\Delta}$ berücksichtigt. Dabei bilden die Sollvorgaben für die Refraktionsdefizite der sphärischen Wirkung $S_{\Delta,i,Soll}$ und/oder der zylindrischen Wirkung $Z_{\Delta,i,Soll}$ insbesondere zusammen mit den Gewichtungsfaktoren $g_{i,S\Delta}$ bzw. $g_{i,Z\Delta}$ das sogenannte Brillenglasdesign. Darüber hinaus können insbesondere auch weitere Residuen, insbesondere weitere zu optimierende Größen, wie z.B. Koma und/oder sphärische Aberration und/oder Prisma und/oder Vergrößerung und/oder anamorphotische Verzerrung, usw., berücksichtigt werden, was insbesondere durch den Ausdruck "+..." in der oben genannten Formel für die Zielfunktion F angedeutet ist.

In manchen Fällen kann es zu einer deutlichen Verbesserung insbesondere einer individuellen Anpassung eines Brillenglases beitragen, wenn bei der Optimierung des Brillenglases nicht nur Abbildungsfehler bis zur zweiten Ordnung (Sphäre, Betrag des Astigmatismus und Achslage), sondern auch höherer Ordnung (z.B. Koma, Dreiblattfehler, sphärische Aberration) berücksichtigt werden.

Es ist aus dem Stand der Technik bekannt, für optische Elemente und insbesondere für Brillengläser, die durch mindestens zwei brechende, refraktive Grenzflächen begrenzt sind, die Form einer Wellenfront zu bestimmen. Dies kann beispielsweise durch numerische Berechnung einer ausreichenden Anzahl an Nachbarstrahlen erfolgen, verbunden mit einem anschließenden Fit der Wellenfront durch Zernike-Polynome. Ein anderer Ansatz beruht auf einer lokalen Wellenfrontdurchrechnung bei der Refraktion (siehe WO 2008/089999 A1). Hierbei wird pro Durchblickpunkt nur ein einziger Strahl berechnet (der Hauptstrahl) und begleitend die Ableitungen der Pfeilhöhen der Wellenfront nach den transversalen (zum Hauptstrahl senkrechten) Koordinaten. Diese Ableitungen können bis zu einer bestimmten Ordnung gebildet werden, wobei die zweiten Ableitungen die lokalen Krümmungseigenschaften der Wellenfront (wie z.B. Brechwert, Astigmatismus) beschreiben und die höheren Ableitungen mit den Abbildungsfehlern höherer Ordnung zusammenhängen.

Bei einer Durchrechnung von Licht durch ein Brillenglas werden die lokalen Ableitungen der Wellenfronten an einer geeigneten Position im Strahlverlauf berechnet, um sie dort mit erwünschten Werten, die aus der Refraktion des Brillenglasträgers hervorgehen, zu vergleichen. Als solche Position, an der eine Auswertung der Wellenfronten stattfindet, wird in der Regel die Scheitelpunktkugel oder beispielsweise die Hauptebene des Auges bei der entsprechenden Blickrichtung herangezogen. Dabei wird angenommen, dass eine sphärische Wellenfront vom Objektpunkt ausgeht und bis zur ersten Brillenglasfläche propagiert. Dort wird die Wellenfront gebrochen und propagiert anschließend zur zweiten Brillenglasfläche, wo sie wieder gebrochen wird. Die letzte Propagation findet dann von der zweiten Grenzfläche bis zur Scheitelpunktkugel (oder der Hauptebene des Auges) statt, wo die Wellenfront mit vorgegebenen Werten für die Korrektion der Refraktion des Auges des Brillenträgers verglichen wird.

Um diesen Vergleich auf Basis der ermittelten Refraktionsdaten des jeweiligen Auges durchzuführen, wird der Auswertung der Wellenfront an der Scheitelpunktkugel ein etabliertes Modell des fehlsichtigen Auges unterstellt, in welchem einem rechtsichtigen Grundauge eine Fehlsichtigkeit (Refraktionsdefizit) überlagert wird. Dies hat sich besonders bewährt, da hierfür weitergehende Kenntnisse über die Anatomie bzw. Optik des jeweiligen Auges (z.B. Verteilung der Brechwerte, Augenlänge, Längenametropie und/oder Brechwertametropie) nicht erforderlich sind. Ausführliche Beschreibungen dieses Modells aus Brillenglas und Refraktionsdefizit sind beispielsweise in Dr. Roland Enders "Die Optik des Auges und der Sehhilfen", Optische Fachveröffentlichung GmbH, Heidelberg, 1995, Seiten 25 ff. und in Diepes, Blendowske "Optik und Technik der Brille", Optische Fachveröffentlichung GmbH, Heidelberg, 2002, Seiten 47 ff. enthalten. Als bewährtes Modell wird insbesondere das darin beschriebene Korrektionsmodell nach REINER verwendet.

Dabei wird als Refraktionsdefizit der Mangel oder der Überschuss an Brechwert des optischen Systems des fehlsichtigen Auges im Vergleich zu einem gleich langen rechtsichtigen Auge (Restauge) angesehen. Der Brechwert des Refraktionsdefizits ist insbesondere annähernd gleich der Fernpunktrefraktion mit negativem Vorzeichen. Für eine vollständige Korrektur der Fehlsichtigkeit bilden das Brillenglas und das Refraktionsdefizit zusammen ein Fernrohrsystem (afokales System). Das Restauge (fehlsichtiges Auge ohne eingefügtes Refraktionsdefizit) wird als rechtsichtig angenommen. Ein Brillenglas gilt damit als vollkorrigierend für die Ferne, wenn sein bildseitiger Brennpunkt mit dem Fernpunkt des fehlsichtigen Auges und damit auch mit dem objektseitigen Brennpunkt des Refraktionsdefizits zusammenfällt.

Aufgabe der Erfindung ist es, die Berechnung oder Optimierung eines Brillenglases, vorzugsweise eines progressiven Brillenglases, zu verbessern, wobei das Brillenglas bereits mit einfachen Messungen individueller, optischer und Augen-anatomischer Daten sehr wirkungsvoll an die individuellen Anforderungen des Brillenträgers angepasst wird. Diese Aufgabe wird durch ein computerimplementiertes Verfahren, eine Vorrichtung, ein Computerprogrammerzeugnis, ein Speichermedium und ein entsprechendes Brillenglas mit den in den unabhängigen Ansprüchen angegebenen Merkmalen gelöst. Bevorzugte Ausführungsformen sind Gegenstand der abhängigen Ansprüche.

Somit betrifft die Erfindung insbesondere die folgenden, ausführlicher beschriebenen beiden Ansätze:

Erster Ansatz

In den folgenden Absätzen werden, soweit nicht ausdrücklich etwas anderes erwähnt ist, zunächst Aspekte des ersten Ansatzes der Erfindung beschrieben:

Im ersten Ansatz bietet die Erfindung gemäß einem ersten Aspekt somit ein computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers. Dabei bietet die Erfindung in einem Aspekt insbesondere ein Verfahren zum Berechnen oder Optimieren eines Brillenglases für zumindest ein Auge eines Brillenträgers anhand der ermittelten individuellen Parameter. Dazu werden zunächst individuelle Refraktionsdaten des zumindest einen Auges des Brillenträgers bereitgestellt. Diese individuellen Refraktionsdaten basieren dabei auf einer individuellen Refraktionsbestimmung. Die Refraktionsdaten umfassen dabei zumindest die sphärische und astigmatische Fehlsichtigkeit des Auges. In einer bevorzugten Ausführungsform beschreiben die erfassten Refraktionsdaten auch Abbildungsfehler höherer Ordnung (HOA). Vorzugsweise werden die Refraktionsdaten (insbesondere soweit sie Abbildungsfehler höherer Ordnung umfassen auch aberrometrische Daten genannt) beispielsweise vom Augenoptiker mittels eines Autorefraktometers oder eines Aberrometers gemessen (objektive Refraktionsdaten). Alternativ oder zusätzlich kann auch eine subjektiv bestimmte Refraktion herangezogen werden. Die Refraktionsdaten werden anschließend vorzugsweise an einen Brillenglashersteller übermittelt und/oder einem Berechnungs- bzw. Optimierungsprogramm zur Verfügung gestellt. Sie stehen damit zur Verfügung, um für das erfindungsgemäße Verfahren erfasst, insbesondere in digitaler Form ausgelesen und/oder empfangen zu werden.

Vorzugsweise umfasst das Bereitstellen der individuellen Refraktionsdaten ein Bereitstellen bzw. Ermitteln der Vergenzmatrix $S_M$ der Fehlsichtigkeit des zumindest einen Auges. Dabei beschreibt die Vergenzmatrix eine Wellenfront vor dem Auge des von einem Punkt auf der Netzhaut auslaufenden bzw. in einem Punkt auf der Netzhaut konvergierenden Lichts. Messtechnisch können solche Refraktionsdaten beispielsweise dadurch ermittelt werden, dass mittels eines Lasers ein Punkt auf der Netzhaut des Brillenträgers beleuchtet wird, von welchem aus sich dann Licht ausbreitet. Während das Licht von dem beleuchteten Punkt aus zunächst im Glaskörper des Auges im Wesentlichen kugelförmig divergiert, kann sich die Wellenfront beim Durchlaufen des Auges insbesondere an optischen Grenzflächen im Auge (z.B. der Augenlinse und/oder der Hornhaut) verändern. Durch eine Messung der Wellenfront vor dem Auge sind somit die Refraktionsdaten des Auges messbar.

Außerdem umfasst das Verfahren gemäß dem ersten Aspekt der Erfindung ein Festlegen eines individuellen Augenmodells, welches zumindest gewisse Vorgaben über geometrische und optische Eigenschaften eines Modellauges individuell festlegt. So werden in dem erfindungsgemäßen individuellen Augenmodell zumindest eine Form (Topographie) einer Hornhautvorderfläche des Modellauges, ein Hornhaut-Linsen-Abstand $d_{CL}$ (dieser Abstand zwischen der Hornhaut und einer Linsenvorderfläche des Modellauges wird auch Vorderkammertiefe bezeichnet), Parameter der Linse des Modellauges, welche insbesondere die optische Wirkung der Linse des Modellauges zumindest teilweise festlegen, und ein Linsen-Netzhaut-Abstand $d_{LR}$ (dieser Abstand zwischen der Linse, insbesondere der Linsenrückfläche, und der Netzhaut des Modellauges wird auch als Glaskörperlänge bezeichnet) in bestimmter Weise, nämlich derart festlegt, dass das Modellauge die bereitgestellten individuellen Refraktionsdaten aufweist, d.h. dass eine im Modellauge von einem Punkt der Netzhaut des Modellauges auslaufende Wellenfront mit der für das reale Auge des Brillenträger ermittelte (z.B. gemessene oder anderweitig ermittelte) Wellenfront (bis zu einer gewünschten Genauigkeit) übereinstimmt. Als Parameter der Linse des Modellauges (Linsenparameter) können beispielsweise entweder geometrische Parameter (Form der Linsenflächen und deren Abstand) und vorzugsweise Materialparameter (z.B. Brechungsindizes der einzelnen Komponenten des Modellauges) so vollständig festgelegt werden, dass diese eine optische Wirkung der Linse zumindest teilweise festlegen. Alternativ oder zusätzlich können als Linsenparameter auch Parameter festgelegt werden, die die optische Wirkung der Linse des Modellauges direkt beschreiben.

So wird im einfachsten Fall eines Augenmodells die Refraktion des Auges durch das optische System bestehend aus der Hornhautvorderfläche, der Augenlinse und der Netzhaut bestimmt. In diesem einfachen Modell legen die Lichtbrechung an der Hornhautvorderfläche und die Brechkraft der Augenlinse (vorzugsweise einschließlich der sphärischen und astigmatischen Aberrationen und Aberrationen höherer Ordnung) zusammen mit deren Positionierung relativ zur Netzhaut die Refraktion des Modellauges fest.

Dabei werden die einzelnen Größen (Parameter) des Modellauges anhand von individuellen Messwerten für das Auge des Brillenträgers und/oder anhand von Standardwerten und/oder anhand der bereitgestellten individuellen Refraktionsdaten entsprechend festgelegt. Insbesondere können manche der Parameter (z.B. die Topographie der Hornhautvorderfläche und/oder die Vorderkammertiefe und/oder zumindest eine Krümmung einer Linsenfläche, usw.) direkt als individuelle Messwerte bereitgestellt werden. Andere Werte können auch - insbesondere dann, wenn es sich um Parameter handelt, deren individuelle Messung sehr aufwendig ist - aus Werten von Standardmodellen für ein menschliches Auge übernommen werden. Insgesamt müssen aber nicht alle (geometrischen) Parameter des Modellauges aus individuellen Messungen oder aus Standardmodellen vorgegeben werden. Vielmehr wird im Rahmen der Erfindung für einen oder mehrere (freie) Parameter eine individuelle Anpassung durch Berechnung unter Berücksichtigung der vorgegebenen Parameter derart vorgenommen, dass das dann resultierende Modellauge die bereitgestellten individuellen Refraktionsdaten aufweist. Je nach Anzahl der in den bereitgestellten individuellen Refraktionsdaten enthaltenen Parameter können entsprechend viele (freie) Parameter des Augenmodells individuell angepasst (gefittet) werden. Abweichend von einem beispielsweise in WO 2013/104548 A1 vorgeschlagenen Modell, wird im Rahmen der vorliegenden Erfindung dabei zumindest der Linsen-Netzhaut-Abstand durch Berechnen festgelegt.

Für die Berechnung bzw. Optimierung des Brillenglases werden eine erste Fläche und eine zweite Fläche des Brillenglases insbesondere als Startflächen mit einer vorgegebenen (individuelle) Position relativ zum Modellauge vorgegeben. In einer bevorzugten Ausführungsform wird nur eine der beiden Flächen optimiert. Vorzugsweise handelt es sich hierbei um die Rückfläche des Brillenglases. Vorzugsweise wird dabei sowohl für die Vorderfläche als auch für die Rückfläche des Brillenglases eine entsprechende Startfläche vorgegeben. In einer bevorzugten Ausführungsform wird während des Optimierungsverfahrens aber nur eine Fläche iterativ verändert bzw. optimiert. Die andere Fläche des Brillenglases kann zum Beispiel eine einfache sphärische oder rotationssymmetrische asphärische Fläche sein. Allerdings ist es auch möglich, beide Flächen zu optimieren.

Ausgehend von den beiden vorgegebenen Flächen umfasst das Verfahren zum Berechnen oder Optimieren ein Ermitteln des Verlaufs eines Hauptstrahls durch zumindest einen Durchblickspunkt *(i)* zumindest einer zu berechnenden oder optimierenden Fläche des Brillenglases in das Modellauge. Der Hauptstrahl beschreibt den geometrischen Strahlverlauf ausgehend von einem Objektpunkt durch die beiden Brillenglasflächen, die Hornhautvorderfläche und die Linse des Modellauges vorzugsweise bis zur Netzhaut des Modellauges.

Außerdem umfasst das Verfahren zum Berechnen oder Optimieren gemäß dem ersten Aspekt der Erfindung ein Auswerten einer Aberration einer entlang des Hauptstrahls aus einer auf die erste Fläche des Brillenglases auftreffenden sphärischen Wellenfront resultierenden Wellenfront an einer Bewertungsfläche insbesondere vor oder innerhalb des Modellauges im Vergleich zu einer in einem Punkt auf der Netzhaut des Augenmodells konvergierenden Wellenfront (Referenzlicht).

Insbesondere wird dazu eine auf die erste Fläche (Vorderfläche) des Brillenglases entlang des Hauptstrahls auftreffende, sphärische Wellenfront ($w_0$) vorgegeben. Diese sphärische Wellenfront beschreibt das von einem Objektpunkt ausgehende Licht (Objektlicht). Die Krümmung der sphärischen Wellenfront beim Auftreffen auf die erste Fläche des Brillenglases entspricht dem Kehrwert des Objektabstandes. Vorzugsweise umfasst das Verfahren somit ein Vorgeben eines Objektabstandsmodells, welches jeder Blickrichtung oder jedem Durchblickspunkt der zumindest einen zu optimierenden Fläche des Brillenglases eine Objektentfernung zuordnet. Damit wird vorzugsweise die individuelle Gebrauchssituation, in der das herzustellende Brillenglas zum Einsatz kommen soll, beschrieben.

Die auf das Brillenglas auftreffende Wellenfront wird nun an der Vorderfläche des Brillenglases vorzugsweise zum ersten Mal gebrochen. Anschießend propagiert die Wellenfront entlang des Hauptstrahls innerhalb des Brillenglases von der Vorderfläche zur Rückfläche, wo sie zum zweiten Mal gebrochen wird. Vorzugsweise propagiert die durch das Brillenglas transmittierte Wellenfront nun entlang des Hauptstrahls weiter bis zur Hornhautvorderfläche des Auges, wo sie vorzugsweise wiederum gebrochen wird. Vorzugsweise wird die Wellenfront nach einer weiteren Propagation innerhalb des Auges bis zur Augenlinse auch dort wiederum gebrochen, um schließlich vorzugsweise bis zur Netzhaut des Auges zu propagieren. Je nach optischen Eigenschaften der einzelnen optischen Elemente (Brillenglasflächen, Hornhautvorderfläche, Augenlinse) führt jeder Brechungsvorgang auch zu einer Deformation der Wellenfront.

Um eine exakte Abbildung des Objektpunktes auf einen Bildpunkt auf der Netzhaut zu erreichen, müsste die Wellenfront die Augenlinse vorzugsweise als konvergierende sphärische Wellenfront verlassen, deren Krümmung genau dem Kehrwert des Abstandes zur Netzhaut entspricht. Ein Vergleich der vom Objektpunkt auslaufenden Wellenfront mit einer (im Idealfall einer perfekten Abbildung) in einem Punkt auf der Netzhaut konvergierenden Wellenfront (Referenzlicht) erlaubt somit die Auswertung einer Fehlanpassung. Dieser Vergleich und damit die Auswertung der Wellenfront des Objektlichts in dem individuellen Augenmodell können dabei an unterschiedlichen Stellen entlang des Verlaufs des Hauptstrahls insbesondere zwischen der zweiten Fläche des optimierenden Brillenglases und der Netzhaut erfolgen. Insbesondere kann damit die Bewertungsfläche an unterschiedlichen Positionen, insbesondere zwischen der zweiten Fläche des Brillenglases und der Netzhaut liegen. Entsprechend weit wird die Brechung und Propagation des vom Objektpunkt auslaufenden Lichts im individuellen Augenmodell vorzugsweise für jeden Durchblickspunkt berechnet. Die Bewertungsfläche kann sich entweder auf den tatsächlichen Strahlengang beziehen oder auf einen virtuellen Strahlengang, wie er beispielsweise zur Konstruktion der Austrittspupille AP benutzt wird. Im Fall des virtuellen Strahlenganges muss das Licht nach der Brechung durch die Rückfläche der Augenlinse zurückpropagiert werden bis zu einer gewünschten Ebene (bevorzugt bis zur Ebene der AP), wobei der dabei benutzte Brechungsindex dem Medium des Glaskörpers entsprechen muss und nicht etwa der Augenlinse. Falls die Bewertungsfläche hinter der Linse bzw. nach der Brechung an der Linsenrückfläche des Modellauges vorgesehen wird, oder falls die Bewertungsfläche durch Rückpropagation entlang eines virtuellen Strahlenganges erreicht wird (wie im Fall der AP), dann kann die resultierende Wellenfront des Objektlichts vorzugsweise einfach mit einer sphärischen Wellenfront des Referenzlichts verglichen werden. Hierzu umfasst das Verfahren somit vorzugsweise ein Vorgeben einer auf die erste Fläche des Brillenglases auftreffenden sphärischen Wellenfront, ein Ermitteln einer durch die Wirkung zumindest der ersten und zweiten Fläche des Brillenglases, der Hornhautvorderfläche und der Linse des Modellauges aus der sphärischen Wellenfront resultierenden Wellenfront in dem zumindest einen Auge, und eine Auswertung der Aberration der resultierenden Wellenfront im Vergleich zu einer auf die Netzhaut konvergierenden sphärischen Wellenfront.

Falls hingegen eine Bewertungsfläche innerhalb der Linse oder zwischen der Linse des Modellauges und dem zu berechnenden bzw. optimierenden Brillenglas vorgesehen sein soll, wird als Referenzlicht einfach eine umgekehrte Propagation von einem Punkt auf der Netzhaut durch die einzelnen Komponenten des Modellauges bis hin zur Bewertungsfläche simuliert, um dort einen Vergleich des Objektlichts mit dem Referenzlicht vorzunehmen.

Wie allerdings bereits eingangs erwähnt, ist eine vollständige Korrektur der Refraktion des Auges gleichzeitig für alle Blickrichtungen des Auges, also für alle Durchblickspunkte der zumindest einen zu optimierenden Brillenglasfläche, im Allgemeinen nicht möglich. Je nach Blickrichtung wird somit vorzugsweise eine absichtliche Fehlanpassung des Brillenglases vorgegeben, welche je nach Anwendungssituation insbesondere in den hauptsächlich genutzten Bereichen des Brillenglases (z.B. zentrale Durchblickspunkte) gering, in den wenig genutzten Bereichen (z.B. periphere Durchblickspunkte) etwas höher sind. Diese Vorgehensweise ist dem Prinzip nach aus herkömmlichen Optimierungsverfahren bereits bekannt.

Um das Brillenglas zu optimieren, wird nun die zumindest eine zu berechnende oder optimierende Fläche des Brillenglases solange iterativ variiert, bis eine Aberration der resultierenden Wellenfront einer vorgegebenen Sollaberration entspricht, also insbesondere um vorgegebene Werte der Aberration von der Wellenfront des Referenzlichts (z.B. einer sphärischen Wellenfront, deren Krümmungsmittelpunkt auf der Netzhaut liegt) abweicht. Die Wellenfront des Referenzlichts wird hier auch als Referenzwellenfront bezeichnet. Vorzugsweise umfasst das Verfahren dazu ein Minimieren einer Zielfunktion F, insbesondere analog zu der eingangs bereits beschriebenen Zielfunktion. Weitere bevorzugte Zielfunktionen, insbesondere bei Berücksichtigung von Abbildungsfehlern höherer Ordnung sind auch weiter unten noch beschrieben. Falls eine Propagation des Objektlichts bis zur Netzhaut berechnet wird, kann eine Auswertung dort anstelle eines Vergleichs von Wellenfront-Parametern beispielsweise mittels einer sogenannten "point spread function" vorgenommen werden.

Im Rahmen der vorliegenden Erfindung wurde somit vorgeschlagen, insbesondere für die Berechnung bzw. Optimierung eines Brillenglases ein derart individuelles Augenmodell festzulegen, welches bis zur Netzhaut dadurch individuell an den einzelnen Brillenträger angepasst ist, dass zumindest die Glaskörperlänge des Modellauges in Abhängigkeit von anderen individuell ermittelten insbesondere gemessenen Daten des Auges individuell berechnet wird. Dieser Parameter muss damit weder a-prior festgelegt werden, noch muss er direkt gemessen werden. Im Rahmen der vorliegenden Erfindung stellte sich heraus, dass dies deshalb eine bemerkenswerte Verbesserung der individuellen Anpassung bei vergleichsweise geringem Aufwand brachte, weil sich die Wellenfrontdurchrechnung als sehr empfindlich von diesem Längenparameter abhängig herausstellte.

Die individuelle Berechnung des Augenmodells insbesondere der Linse-Netzhautabstandes (Glaskörperlänge) kann dabei insbesondere bereits in einem Aberrometer oder einem Topografen mit entsprechend erweiterter Funktionalität vorgenommen werden. Vorzugsweise erfolgt dabei eine individuelle Ermittlung einer Augenlänge. Besonders bevorzugt wird dem Benutzer die berechnete Glaskörperlänge und/oder die ermittelte (berechnete) Augenlänge angezeigt. Hierzu weist eine entsprechende Vorrichtung (insbesondere ein Aberrometer bzw. Topograf) eine entsprechende Anzeigeeinrichtung auf.

Vorzugsweise wird dabei die Hornhautvorderfläche individuell gemessen und die Augenlinse des individuellen Augenmodells entsprechend berechnet, um die individuell bestimmten Refraktionsdaten zu erfüllen. Dabei wird in einer bevorzugten Ausführungsform die Hornhautvorderfläche (bzw. ihre Krümmung) entlang der Hauptschnitte individuell gemessen (Topometrie). In einer weiteren bevorzugten Ausführungsform wird die Topographie der Hornhautvorderfläche (d.h. die vollständige Beschreibung der Fläche) individuell gemessen. In einer weiteren bevorzugten Ausführungsform erfolgt das Festlegen des Hornhaut-Linsen-Abstandes anhand von individuellen Messwerten für den Hornhaut-Linsen-Abstand.

Besonders bevorzugt umfasst das Festlegen der Parameter der Linse des Modellauges ein Festlegen folgender Parameter:

-- eine Form der Linsenvorderfläche;
-- eine Linsendicke; und
-- eine Form der Linsenrückfläche.

Auch wenn es für die Nutzung der Erfindung nicht essenziell ist, kann durch dieses genauere Modell der Linse die individuelle Anpassung nochmals verbessert werden.

In diesem Fall erfolgt in einer besonders bevorzugten Ausführungsform das Festlegen der Linsendicke und der Form der Linsenrückfläche anhand von vorgegebenen Werten (Standardwerten, beispielsweise aus der Fachliteratur), wobei weiter bevorzugt das Festlegen der Form der Linsenvorderfläche umfasst:

- Bereitstellen von Standardwerten für eine mittlere Krümmung der Linsenvorderfläche; und
- Berechnen der Form der Linsenvorderfläche unter Berücksichtigung der bereitgestellten individuellen Refraktionsdaten.

In einer weiteren bevorzugten Ausführungsform des detaillierteren Linsenmodells umfasst das Festlegen der Form der Linsenvorderfläche:

- Bereitstellen eines individuellen Messwerts einer Krümmung in einem Normalschnitt der Linsenvorderfläche.

In diesen Fall ist es besonders bevorzugt, wenn außerdem das Festlegen der Linsendicke und der Form der Linsenrückfläche anhand von Standardwerten erfolgt, und noch weiter bevorzugt das Festlegen der Form der Linsenvorderfläche umfasst:

- Berechnen der Form der Linsenvorderfläche unter Berücksichtigung der bereitgestellten individuellen Refraktionsdaten und des bereitgestellten individuellen Messwerts der Krümmung in einem Normalschnitt der Linsenvorderfläche.

Alternativ oder zusätzlich zur Form der Linse bzw. der Linsenflächen kann das Festlegen der Linsenparameter ein Festlegen einer optischen Wirkung der Linse umfassen. Insbesondere wird dabei zumindest eine Position zumindest einer Hauptebene und eine sphärische Wirkung (bzw. zumindest eine Brennweite) der Linse des Modellauges festgelegt. Besonders bevorzugt wird auch eine zylindrische Wirkung (Betrag und Achslage) der der Linse des Modellauges festgelegt. In einer weiteren bevorzugten Ausführungsform können auch optische Abbildungsfehler höherer Ordnungen der Linse des Modellauges festgelegt werden.

Vorzugsweise liegt die Bewertungsfläche zwischen der Hornhautvorderfläche und der Netzhaut. In einer besonders bevorzugten Ausführungsform liegt die Bewertungsfläche zwischen der Linse und der Netzhaut des Modellauges. In einer anderen besonders bevorzugten Ausführungsform liegt die Bewertungsfläche an der Austrittspupille (AP) des Modellauges. Dabei kann die Austrittspupille vor der Linsenrückfläche des Modellauges liegen. Bei diesen Positionierungen kann eine besonders genaue, individuelle Anpassung des Brillenglases erreicht werden.

In einem weiteren Aspekt bietet die Erfindung eine Vorrichtung zum Ermitteln relevanter individueller Parameter zumindest eines Auges eines Brillenträgers für die Berechnung oder Optimierung eines Brillenglases für das zumindest eine Auge des Brillenträgers. Dabei bietet die Erfindung in einem Aspekt insbesondere eine Vorrichtung zum Berechnen oder Optimieren eines Brillenglases für zumindest ein Auge eines Brillenträgers anhand der ermittelten individuellen Parameter. Die Vorrichtung zum Ermitteln relevanter individueller Parameter umfasst:

- eine Datenschnittstelle zum Bereitstellen von individuellen Refraktionsdaten des zumindest einen Auges des Brillenträgers; und
- ein Modellierungsmodul zum Festlegen eines individuellen Augenmodells, welches zumindest die Parameter

-- Form einer Hornhautvorderfläche eines Modellauges;
-- Hornhaut-Linsen-Abstand;
-- Parameter der Linse des Modellauges; und
-- Linsen-Netzhaut-Abstand;

derart anhand von individuellen Messwerten für das Auge des Brillenträgers und/oder von Standardwerten und/oder anhand der bereitgestellten individuellen Refraktionsdaten festlegt werden, dass das Modellauge die bereitgestellten individuellen Refraktionsdaten aufweist, wobei zumindest das Festlegen des Linsen-Netzhaut-Abstands durch Berechnen erfolgt.

Vorzugsweise ist das Modellierungsmodul ausgelegt zum Ermitteln einer Augenlänge des Modellauges unter Berücksichtigung des berechneten Linsen-Netzhaut-Abstands. Vorzugsweis umfasst die Vorrichtung außerdem eine Anzeigeeinrichtung zum Anzeigen des berechneten Linsen-Netzhaut-Abstands und/oder der ermittelten Augenlänge. Besonders bevorzugt ist die Vorrichtung als Aberrometer und/oder als Topograf ausgebildet.

Darüber hinaus umfasst die Vorrichtung zum Berechnen oder Optimieren eines Brillenglases außerdem insbesondere:

- eine Flächenmodelldatenbank zum Vorgeben einer ersten Fläche und einer zweiten Fläche für das zu berechnende bzw. optimierende Brillenglas;
- ein Hauptstrahlermittlungsmodule zum Ermitteln des Verlaufs eines Hauptstrahls durch zumindest einen Durchblickspunkt ($i$) zumindest einer zu berechnenden oder optimierenden Fläche des Brillenglases in das Modellauge;
- ein Auswertemodul zum Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront; und
- ein Optimierungsmodul iteratives Variieren der zumindest einen zu berechnenden oder optimierenden Fläche des Brillenglases bis die ausgewertete Aberration einer vorgegebenen Sollaberration entspricht.

Außerdem bietet die Erfindung ein Computerprogrammerzeugnis, insbesondere in Form eines Speichermediums oder eines Datenstroms, welches Programmcode enthält, der ausgelegt ist, wenn geladen und ausgeführt auf einem Computer, ein Verfahren zum Ermitteln relevanter individueller Parameter zumindest eines Auges eines Brillenträgers und/oder ein Verfahren zum Berechnen oder Optimieren eines Brillenglases gemäß der vorliegenden Erfindung insbesondere in einer bevorzugten Ausführungsform durchzuführen.

Zweiter Ansatz

In den folgenden Absätzen werden, soweit nicht ausdrücklich etwas anderes erwähnt ist, Aspekte des zweiten Ansatzes der Erfindung beschrieben:

Im zweiten Ansatz bietet die Erfindung somit gemäß einem ersten Aspekt ein computerimplementiertes Verfahren zum Ermitteln individueller Aberrationsdaten zumindest eines Auges eines Brillenträgers, insbesondere für eine Verwendung zum Berechnen oder Optimieren eines Brillenglases für das zumindest eine Auge des Brillenträgers. Dazu wird zunächst eine gemessene Hornhauttopographie des zumindest einen Auges des Brillenträgers bereitgestellt. Vorzugsweise wird diese Hornhauttopographie direkt mittels eines entsprechenden Topographen gemessen. Alternativ können solche gemessenen Daten auch abgespeichert werden, um sie beispielsweise später für die weitere Verarbeitung bereitstellen zu können.

Ausgehend von der gemessenen Hornhauttopographie werden individuelle Abbildungseigenschaften der Hornhaut des Auges, welche zumindest Abbildungsfehler höherer Ordnung der Hornhaut, $HOA_C$, beschreiben, ermittelt. Diese individuellen Abbildungseigenschaften der Hornhaut können aus der Hornhauttopographie und unter Berücksichtigung eines Brechungsindexübergangs zwischen Luft und Hornhaut ermittelt werden. Die individuellen Abbildungseigenschaften können in diesem Zuge in einer gewünschten Parameterdarstellung in Form unterschiedlicher Komponenten ermittelt und gegebenenfalls abgespeichert werden. Individuelle Abbildungseigenschaften beschreiben insbesondere unterschiedliche Komponenten der optischen Lichtbrechung, wie z.B. einen sphärischen Anteil, astigmatische Anteile und Anteile von Abbildungsfehlern höherer Ordnung (d.h. größer als zweiter Ordnung); Erfindungsgemäß werden zumindest (einige) Abbildungsfehler höherer Ordnung der Hornhaut (als zumindest ein Teil der individuellen Abbildungseigenschaften der Hornhaut) ermittelt.

Unter Berücksichtigung der ermittelten individuellen Abbildungseigenschaften der Hornhaut des Auges, insbesondere unter Berücksichtigung der Abbildungsfehler höherer Ordnung der Hornhaut, $HOA_C$, werden dann erfindungsgemäß zumindest Abbildungsfehler höherer Ordnung des Auges, $HOA_{Auge}$, ermittelt. Insbesondere werden diese Abbildungsfehler höherer Ordnung des Auges, $HOA_{Auge}$, als Aberration einer Wellenfront an der Hornhaut dargestellt oder ermittelt, die nach einer Brechung an der Hornhautvorderfläche mit der gemessenen Topographie (und gegebenenfalls einer weiteren Propagation durch das Auge) zu einem Punkt auf der Netzhaut konvergiert. Dabei werden die Abbildungsfehler höherer Ordnung des Auges unter Berücksichtigung der ermittelten individuellen Abbildungseigenschaften der Hornhaut des Auges, also unter Berücksichtigung der individuellen Messung der Hornhauttopographie, nicht aber unter Berücksichtigung individueller Messungen von Aberrationen (höherer Ordnung) andere Komponenten des Auges (z.B. der Augenlinse) ermittelt.

Zwar ist die Berücksichtigung von Abbildungsfehlern höherer Ordnung bei der Berechnung oder Optimierung von Brillengläsern der Qualität der individuellen Anpassung eines Brillenglases durchaus zuträglich. Allerdings ist es oft aufwändig, die Abbildungsfehler höherer Ordnung eines Auges individuell zu messen. Beispielsweise steht nicht jedem Optiker ein entsprechendes Aberrometer zur Verfügung, mit dem diese Abbildungsfehler direkt und einfach erfasst werden können. Mit der vorliegenden Erfindung ist es aber dennoch möglich, eine Berechnung oder Optimierung eines Brillenglases unter Berücksichtigung von HOA zur Verfügung zu stellen, ohne die HOA des gesamten Auges direkt und individuell messen zu müssen. Dazu können zwar in einem nachfolgend beschriebenen Augenmodell Abbildungsfehler höherer Ordnung für Grenzflächen und optische Eigenschaften einzelner Komponenten im Modellauge berücksichtigt werden. Die individuelle Ermittlung der HOA des gesamten Auges und gegebenenfalls eine Belegung der Parameter des Augenmodells erfolgt aber nicht anhand von individuellen Messungen der HOA des gesamten Auges, sondern anhand von individuellen Messung der HOA der Hornhaut des Auges. Insbesondere umfasst das Verfahren dazu ein Bereitstellen von individuellen Abbildungsfehlern der Hornhaut des Auges des Brillenträgers, welche Abbildungsfehler höherer Ordnung (HOA) der Hornhaut des Auges beschreiben. Insbesondere kann hierzu einfach die Form der Hornhautvorderfläche gemessen werden, was in der Regel wesentlich einfacher möglich ist, als die HOA des gesamten Auges direkt zu messen.

In einer bevorzugten Ausführungsform umfasst das Ermitteln der individuellen Abbildungseigenschaften der Hornhaut des Auges ein Ermitteln von Werten der Abbildungsfehler höherer Ordnung der Hornhaut, $HOA_C$, wobei das Ermitteln der Abbildungsfehler des Auges vorzugsweise ein Ermitteln der Abbildungsfehler höherer Ordnung des Auges, $HOA_{Auge}$, gemäß $HOA_{Auge} = HOA_C + \Delta HOA_{C,Auge}$ mit einer vorgegebenen oder individuell bestimmten Verschiebung höherer Ordnung, $\Delta HOA_{C,Auge}$, insbesondere mit $\Delta HOA_{C,Auge} = 0$, umfasst. Damit lassen sich die Abbildungsfehler des gesamten Auges oder eine gute Näherung davon sehr schnell und wirkungsvoll mit einfachen Mitteln bestimmen.

Unabhängig von dem konkreten, funktionalen Zusammenhang fungiert im Allgemeinen vorzugsweise $\Delta HOA_{C,Auge}$ als effektive Größe zur Beschreibung der Abbildungsfehler höherer Ordnung des Rest-Auges, insbesondere falls nicht alle Abbildungsfehler höherer Ordnung des Gesamtauges durch die Abbildungsfehler höherer Ordnung der Hornhaut erklärbar bzw. beschreibbar sind. Insbesondere besitzt im Allgemeinen im Rest-Auge die Linsenvorderfläche und/oder die Linsenrückflächen Abbildungsfehler höherer Ordnung. Wie diese sich mit den Abbildungsfehlern höherer Ordnung der Hornhaut zu den Abbildungsfehlern des Gesamtauges zusammensetzen, hängt insbesondere auch von Dimensionen des Auges, z.B. den nachfolgend noch beschriebenen Längenparametern $d_{CL}$, $d_L$ und/oder $d_{LR}$ des Auges ab. Solche Zusammenhänge könnten beispielsweise aus G. Esser, W. Becken, W. Müller, P. Baumbach, J. Arasa and D. Uttenweiler, Derivation of the Refraction Equations for Higher Order Aberrations of Local Wavefronts at Oblique Incidence", JOSA A Vol. 27, No. 2, p 218-37 (2010) und/oder aus G. Esser, W. Becken, W. Müller, P. Baumbach, J. Arasa and D. Uttenweiler, Derivation of the Propagation Equations for Higher Order Aberrations of Local Wavefronts", JOSA A Vol. 28, No. 12, p 2442-58 (2011) übernommen werden.

In einer Ausführungsform umfasst das Verfahren zum Ermitteln individueller Aberrationsdaten ein Ermitteln oder Bereitstellen von Korrekturwerten $\Delta HOA_{C,Auge,Linsenvorderfläche}$ von Abbildungsfehlern höherer Ordnung durch die Linsenvorderfläche, ein Ermitteln oder Bereitstellen von Korrekturwerten $\Delta HOA_{C,Auge,Linsenrückfläche}$ von Abbildungsfehlern höherer Ordnung durch die Linsenrückfläche und ein Ermitteln oder Bereitstellen von Längenparametern des Auges, wobei das Ermitteln der individuellen Abbildungseigenschaften der Hornhaut des Auges ein Ermitteln von Werten der Abbildungsfehler höherer Ordnung der Hornhaut, $HOA_C$, umfasst und das Ermitteln der Abbildungsfehler des Auges vorzugsweise ein Ermitteln der Abbildungsfehler höherer Ordnung des Auges, $HOA_{Auge}$, gemäß

$$HOA_{Auge} = f\left(HOA_C, \Delta HOA_{C,Auge,Linsenvorderfläche}, \Delta HOA_{C,Auge,Linsenrückfläche}, Längenparameter\right)$$

umfasst.

Will man nicht detailliert die Komponenten des Auges nach brechenden Flächen aufschlüsseln, dann kann man näherungsweise auch alle Parameter des Restauges zusammenfassen zu

$$\Delta HOA_{C,Auge} = h\left(\Delta HOA_{C,Auge,Linsenvorderfläche}, \Delta HOA_{C,Auge,Linsenrückfläche}, Längenparameter\right)$$

und dann die Abbildungsfehler höherer Ordnung des Gesamtauges allgemein schreiben als

$$HOA_{Auge} = g\left(HOA_C, \Delta HOA_{C,Auge}\right)$$

.

Dabei ist zu berücksichtigen, dass alle Parameter außer $HOA_C$ nicht notwendigerweise individuell gemessen werden müssen, sondern auch der Literatur entnommen oder modellbasiert, auch in Abhängigkeit anderer Größen (z.B. Augenlänge aus LOAs aus der subjektiven Refraktion und ggf. $LOA_C$), angenommen werden können.

In einer weiteren bevorzugten Ausführungsform umfasst das Ermitteln der individuellen Abbildungseigenschaften der Hornhaut des Auges ein Ermitteln von Refraktionswerten niedriger Ordnung der Hornhaut, $LOA_C$, wobei das Ermitteln der Abbildungsfehler des Auges ein Ermitteln von Abbildungsfehlern niedriger Ordnung des Auges, $LOA_{Auge}$, gemäß $LOA_{Auge} = LOA_C + \Delta LOA_{C,Auge}$ mit einer vorgegebenen oder individuell bestimmten Verschiebung niedriger Ordnung, $\Delta LOA_{C,Auge}$, umfasst.

Diese bevorzugte Ausführungsform bezieht sich beispielsweise auf die Situation, dass auch die LOA des Auges nicht direkt gemessen werden (z.B. per Refraktion), sondern wie die HOA aus der Topographiemessung geschätzt bzw. genähert werden. Sowohl LOA aus auch HOA des Auges sind in einer bevorzugten Ausführungsform direkt durch die entsprechenden Cornea-Aberrationen gegeben bis auf einen Shift $\Delta LOA_{C,Auge}$ bzw. $\Delta HOA_{C,Auge}$. Dieser kann konstant sein oder von weiteren Parametern abhängen. Bevorzugt ist $\Delta HOA_{C,Auge} = 0$ für alle Ordnungen n>2, und $\Delta LOA_{C,Auge,M}$ = $-LOA_{C,M,Std}$, $\Delta LOA_{C,Auge,J0} = 0$ , $\Delta LOA_{C,Auge,J45} = 0$, wobei $\Delta LOA_{C,Auge,M}$ , $\Delta LOA_{C,Auge,J0}$ , $\Delta LOA_{C,Auge,J45}$ diejenigen Shifts sind, die sich auf die $M$-Komponente, die $J_0$-Komponente bzw. die $J_{45}$-Komponente der Aberrationen des Auges beziehen, und wobei $LOA_{C,M,Std}$ der Standardwert der $M$-Komponente der Cornea ist, der bevorzugt $\Delta LOA_{C,M,Std}$ = $43.08 dpt$ ist.

Somit umfasst in einer bevorzugten Ausführungsform das Ermitteln von Refraktionswerten niedriger Ordnung der Hornhaut, $LOA_C$ , ein Ermitteln astigmatischer Anteile der Refraktion der Hornhaut, $LOA_{C,J0}$ und $LOA_{C,J45}$, wobei das Ermitteln von Abbildungsfehlern niedriger Ordnung des Auges, $LOA_{Auge}$, ein Ermitteln astigmatischer Anteile der Abbildungsfehler niedriger Ordnung des Auges, $LOA_{Auge,J0}$ und $LOA_{C,Auge,J45}$, gemäß $LOA_{Auge,J0} = LOA_{C,J0}$ und $LOA_{Auge,J45} = LOA_{C,J45}$ umfasst. Alternativ oder zusätzlich umfass das Ermitteln von Refraktionswerten niedriger Ordnung der Hornhaut, $LOA_C$ , vorzugsweise ein Ermitteln eines sphärischen Anteils der Refraktion der Hornhaut, $LOA_{C,M}$, wobei das Ermitteln von Abbildungsfehlern niedriger Ordnung des Auges, $LOA_{Auge}$ , vorzugsweise ein Ermitteln eines sphärischen Anteils der Abbildungsfehler niedriger Ordnung des Auges, $LOA_{Auge,M}$ , gemäß $LOA_{Auge,M} = LOA_{C,M} - LOA_{C,M,Std}$ mit einem vorgegebenen Standardwert $LOA_{C,M,Std}$ umfasst.

Weiterhin bevorzugt sind Shifts, die selbst eine Funktion der Cornea-Topographie sind. Besonders bevorzugt ist ein Shift, bei dem die $M$-Komponente der LOA des Auges eine lineare Funktion $\Delta LOA_{C,Auge,M}(LOA_{C,M}) = \Delta LOA_{C,Auge,M}$ $(LOA_{C,M,Std}) + \alpha(LOA_{C,M} - LOA_{C,M,Std})$ der M-Komponente der LOA der Cornea ist, wobei bevorzugt $5 < \alpha < 15$ ist. Somit umfasst vorzugsweise das Ermitteln von Refraktionswerten niedriger Ordnung der Hornhaut, $LOA_C$ ein Ermitteln eines sphärischen Anteils der Refraktion der Hornhaut, $LOA_{C,M}$, wobei das Ermitteln von Abbildungsfehlern niedriger Ordnung des Auges, $LOA_{Auge}$, ein Ermitteln eines sphärischen Anteils der Abbildungsfehler niedriger Ordnung des Auges, $LOA_{Auge,M}$, gemäß $LOA_{Auge,M} = LOA_{C,M} + \Delta LOA_{C,Auge,M}(LOA_{C,M})$ mit einer linearen Funktion $\Delta LOA_{C,Auge,M}(LOA_{C,M})$ $= \Delta LOA_{C,Auge,M}(LOA_{C,M,Std}) + \alpha(LOA_{C,M} - LOA_{C,M,Std})$ mit einem vorgegebenen Standardwert $LOA_{C,M,Std}$ und einem vorgegebenen Wert $\alpha$, vorzugsweise im Bereich $5 < \alpha < 15$, umfasst.

In einer weiteren bevorzugten Ausführungsform umfasst das Ermitteln der Abbildungsfehler des Auges ein Ermitteln von Abbildungsfehlern niedriger Ordnung des Auges, $LOA_{Auge}$, anhand von individuellen Refraktionsmessungen an dem zumindest einen Auge erfolgt. Dazu werden somit insbesondere individuelle Refraktionsdaten des zumindest einen Auges des Brillenträgers bereitgestellt. Diese individuellen Refraktionsdaten basieren dabei auf einer individuellen Refraktionsbestimmung. Die Refraktionsdaten umfassen dabei insbesondere die sphärische und astigmatische Fehlsichtigkeit des Auges. Die individuellen Refraktionsdaten enthalten jedoch keine individuellen Abbildungsfehler höherer Ordnung (HOA) des Auges.

Die Unterscheidung zwischen Abbildungsfehlern niedriger Ordnung (LOA) und höherer Ordnung (HOA) erfolgt im Rahmen der vorliegenden Beschreibung in der auf dem Fachgebiet üblichen Weise, so dass Abbildungsfehler niedriger Ordnung die Abbildungsfehler bis 2. Ordnung der Taylor- oder Zernike-Entwicklung bezeichnen (insbesondere Prisma, Sphäre, Zylinder, Achslage), während Abbildungsfehler höherer Ordnung die Abbildungsfehler ab 3. Ordnung in Taylor- oder Zernike-Entwicklung bezeichnen.

Vorzugsweise werden die Refraktionsdaten beispielsweise vom Augenoptiker mittels eines Autorefraktometers gemessen (objektive Refraktionsdaten). Alternativ oder zusätzlich kann auch eine subjektiv bestimmte Refraktion herangezogen werden. Die Refraktionsdaten werden anschließend vorzugsweise an einen Brillenglashersteller übermittelt und/oder einem Berechnungs- bzw. Optimierungsprogramm zur Verfügung gestellt. Sie stehen damit auch zur Verfügung, um für das erfindungsgemäße Verfahren erfasst, insbesondere in digitaler Form ausgelesen und/oder empfangen zu werden.

Vorzugsweise umfasst das Bereitstellen der individuellen Refraktionsdaten ein Bereitstellen bzw. Ermitteln der Vergenzmatrix $S_M$ der Fehlsichtigkeit des zumindest einen Auges für Abbildungsfehler niedriger Ordnung, insbesondere bis 2. Ordnung in Taylor- oder Zernike-Darstellung. Dabei beschreibt die Vergenzmatrix eine Wellenfront vor dem Auge des von einem Punkt auf der Netzhaut auslaufenden bzw. in einem Punkt auf der Netzhaut konvergierenden Lichts. Messtechnisch können solche Refraktionsdaten beispielsweise dadurch ermittelt werden, dass mittels eines Lasers ein Punkt auf der Netzhaut des Brillenträgers beleuchtet wird, von welchem aus sich dann Licht ausbreitet. Während das Licht von dem beleuchteten Punkt aus zunächst im Glaskörper des Auges im Wesentlichen kugelförmig divergiert, kann sich die Wellenfront beim Durchlaufen des Auges insbesondere an optischen Grenzflächen im Auge (z.B. der Augenlinse und/oder der Hornhaut) verändern. Durch eine Messung der Wellenfront vor dem Auge sind somit die Refraktionsdaten des Auges messbar.

Die Vergenzmatrix hängt mit der bereits erwähnten $M$-Komponente, der $J_0$-Komponente und der $J_{45}$-Komponente in bekannter Weise insbesondere gemäß

$$\mathbf{S} = \begin{pmatrix} M + J_0 & J_{45} \\ J_{45} & M - J_0 \end{pmatrix}$$

zusammen. Vorzugsweise werden somit die LOA des Auges als $M, J_0, J_{45}$ insbesondere in der Cornea-Ebene bereitgestellt (z.B. per Refraktion und anschließende HSA-Umrechnung).

Die HOA des Auges sind, wie bereits beschrieben, vorzugsweise direkt durch die entsprechenden Cornea-Aberrationen gegeben bis auf einen Shift $\Delta HOA_{C,Auge}$. Dieser kann konstant sein oder von weiteren Parametern abhängen. Bevorzugt ist $\Delta HOA_{C,Auge} = 0$ für alle Ordnungen n>2.

In weiteren Ausführungsformen wird vorzugsweise ein Augenmodell eingeführt. In einem einfachen Fall wird die Augenlinse dabei nicht durch zwei Flächen modelliert, sondern durch eine einzige Linse, deren LOA und HOA durch $LOA_L$ bzw. $HOA_L$ gegeben sind. Man kann sich diesen einfachen Fall aus den nachfolgend noch beschriebenen Ausführungsformen erzeugen, indem man dort setzt: $d_L = 0$, $L = L_1 + L_2$. Die Funktion, wie die Linse dann aus den $LOA_C$ hervorgeht, entspricht den Formeln (4) und (5) aus der Beschreibung WO 2013/104548 A1, wenn man dort auch $d_L = 0$, $L = L_1 + L_2$ setzt, lautet

$$\mathbf{L}(M, J_0, J_{45}, LOA_C, d_{CL}, d_{LR}) = \frac{n_{LR}}{d_{LR}} \mathbf{1} - \frac{\mathbf{S} + \mathbf{C}}{1 - d_{CL}/n_{CL}(\mathbf{S} + \mathbf{C})} \quad ,$$

$$\mathbf{S} = \begin{pmatrix} M + J_0 & J_{45} \\ J_{45} & M - J_0 \end{pmatrix}$$

wobei wiederum die Matrixdarstellung der Refraktion ist und c die entsprechende Matrixdarstellung für $LOA_C$.

Die HOA des Gesamtauges werden dann gemäß einer Funktion $f(LOA_C,HOA_C,L,HOA_L,d_{CL},d_{LR})$ ermittelt, die dadurch zustande kommt, dass eine Kugelwelle beginnend mit einem Punkt auf der Netzhaut rückwärts durch das Auge propagiert und gebrochen wird. Die HOA der resultierenden Wellenfront insbesondere in der Ebene der Cornea sind dann die HOA des Gesamtauges.

Somit umfasst das Ermitteln von Abbildungsfehlern des Auges vorzugsweise:

- Festlegen eines individuellen Augenmodells, in welchem eine Form einer Hornhautvorderfläche eines Modellauges der gemessenen Hornhauttopographie entspricht und in welchem außerdem zumindest

    - - ein Hornhaut-Linsen-Abstand;
    - - Parameter einer Linse des Modellauges; und
    - - ein Linsen-Netzhaut-Abstand

derart anhand von individuellen Messwerten für das Auge des Brillenträgers und/oder von Standardwerten und/oder anhand der ermittelten Abbildungsfehler niedriger Ordnung des Auges festgelegt werden, dass das Modellauge die ermittelten Abbildungsfehler niedriger Ordnung des Auges aufweist; und

- Ermitteln einer Aberration einer nach einer Brechung an der Hornhautvorderfläche des Modellauges und einer Propagation durch das Modellauge in einem Punkt auf der Netzhaut konvergierenden Wellenfront.

Besonders bevorzugt umfasst das Festlegen der Parameter der Linse des Modellauges ein Festlegen folgender Parameter:

    -- Form der Linsenvorderfläche;
    -- Linsendicke; und
    -- Form der Linsenrückfläche.

Vorzugsweise werden dabei neben der individuellen Hornhauttopographie auch die Refraktion $M,J_0,J_{45}$ (bzw.

$$\mathbf{S} = \begin{pmatrix} M + J_0 & J_{45} \\ J_{45} & M - J_0 \end{pmatrix}$$

deren Matrixdarstellung ) und Längenparameter $d_{CL},d_L,d_{LR}$ des Auges bereitgestellt. Damit werden vorzugsweise die LOA der einen Linsenfläche aus den LOA der anderen Linsenfläche berechnet, indem die Forderung gestellt wird, dass das aus den Komponenten aufgebaute Gesamtauge dann die gegebene Refraktion hat, d.h. eine Wellenfront, die der Refraktion entspricht, in 2. Ordnung durch das Auge propagiert und gebrochen als Kugelwelle auf der Netzhaut konvergiert.

Die HOA des Gesamtauges lassen sich dann als Funktion $f(LOA_C,HOA_C,L_1,HOA_{L1},L_2,HOA_{L2},d_{CL},d_L,d_{LR})$ ausdrücken, die dadurch zustande kommt, dass eine Kugelwelle beginnend mit einem Punkt auf der Netzhaut rückwärts durch das Auge propagiert und gebrochen wird. Die HOA der resultierenden Wellenfront in der Ebene der Cornea sind dann die HOA des Gesamtauges.

In bevorzugten Ausführungsformen sind die Längenparameter konstant. In weiterhin bevorzugten Varianten sind sie selbst Schätzwerte $d_{CL},d_L,d_{LR}$ als Funktion der Refraktion, der LOA der Cornea oder Kombinationen hiervon, die bekannte Korrelationen aus der Bevölkerung ausnutzen (z.B. dass Myopie mit großer Augenlänge korreliert). Beispiele hierfür sind $d_{CL}(M,C),d_{LR}(M,C)$.

Besonders bevorzugt werden Längenparameter, beispielsweise $d_{LR}$, wie weiter unten noch beispielhaft beschrieben berechnet. Auch hier ergibt sich eine Funktion $d_{LR}(M,C)$, wobei diese dann aber nicht aus Korrelationen innerhalb der Bevölkerung bei gegebener Topographiemessung stammt, sondern direkt aus der Topographiemessung unter bestimmten Annahmen über die Linsenflächen berechnet wird.

Vorzugsweise erfolgt das Festlegen der Linsendicke und des Linsen-Netzhaut-Abstands anhand von vorgegebenen Standardwerten, wobei das Festlegen der Form der Linsenvorderfläche und der Linsenrückfläche anhand von vorgegebenen Standardwerten für die Abbildungsfehler höherer Ordnung der jeweiligen Fläche erfolgt. Besonders bevorzugt werden die Standardwerte der Abbildungsfehler höherer Ordnung der Linsenvorderfläche und/oder der Linsenrückfläche auf null gesetzt.

Vorzugsweise umfasst das Ermitteln der individuellen Abbildungseigenschaften der Hornhaut des Auges ein Ermitteln von Refraktionswerten niedriger Ordnung der Hornhaut, $LOA_C$. Insbesondere in diesem Fall erfolgt vorzugsweise das Festlegen des Linsen-Netzhaut-Abstands und/oder das Festlegen der Linsendicke und/oder das Festlegen der Form der Linsenvorderfläche und/oder der Linsenrückfläche anhand der ermittelten Refraktionswerte niedriger Ordnung der Hornhaut, $LOA_C$. Dabei können vorzugsweise die anderen der genannten Werte insbesondere anhand von Standardwerten erfolgen.

Vorzugsweise umfasst das Ermitteln der Abbildungsfehler des Auges ein Ermitteln von Abbildungsfehlern niedriger Ordnung des Auges, $LOA_{Auge}$. Insbesondere in diesem Fall erfolgt vorzugsweise das Festlegen des Linsen-Netzhaut-Abstands und/oder das Festlegen der Linsendicke und/oder das Festlegen der Form der Linsenvorderfläche und/oder der Linsenrückfläche anhand der ermittelten Abbildungsfehler niedriger Ordnung des Auges, $LOA_{Auge}$. Dabei können vorzugsweise die anderen der genannten Werte insbesondere anhand von Standardwerten erfolgen.

Dabei wird vorzugsweise eine Korrelation zwischen der Sphäre des Auges und dem Linsen-Netzhaut-Abstand $d_{LR}$ beispielsweise aus statistischen Untersuchungen der Bevölkerung bereitgestellt. Damit wird anhand eines individuell ermittelten Wertes der Sphäre auf einen individuellen Wert $d_{LR}$ des Linsen-Netzhaut-Abstand geschlossen.

In einer anderen bevorzugten Ausführungsform wird eine Korrelation zwischen der Sphäre des Auges und der Gesamtlänge des Auges beispielsweise aus statistischen Untersuchungen der Bevölkerung bereitgestellt. Damit wird anhand eines individuell ermittelten Wertes der Sphäre auf einen individuellen Wert der Gesamtlänge des Auges geschlossen. Zur Bestimmung des individuellen Linsen-Netzhaut-Abstands werden dann vorzugsweise individuelle Werte der Vorderkammertiefe $d_{CL}$ und der Linsendicke $d_L$ subtrahiert.

In einer weiteren bevorzugten Ausführungsform werden die LOA der Cornea und die individuellen Werte der Vorderkammertiefe $d_{CL}$ und der Linsendicke $d_L$ ermittelt und vorzugsweise zusammen mit Standardwerten für die Linsenkrümmungen dazu verwendet, die Länge $d_{LR}$ so zu bestimmen, dass eine Wellenfront aus dem Unendlichen unter diesen Annahmen genau (bzgl. ihrer LOA) auf der Netzhaut konvergiert.

Vorzugsweise umfasst das Verfahren somit ein Festlegen eines individuellen Augenmodells, welches zumindest gewisse Vorgaben über geometrische und optische Eigenschaften eines Modellauges individuell festlegt. So wird in dem individuellen Augenmodell zumindest eine Form (Topographie) einer Hornhautvorderfläche des Modellauges anhand der individuellen Topographiemessung festgelegt. Vorzugsweise werden außerdem ein Hornhaut-Linsen-Abstand $d_{CL}$ (dieser Abstand zwischen der Hornhaut und einer Linse oder einer Linsenvorderfläche des Modellauges wird auch Vorderkammertiefe bezeichnet), Parameter der Linse des Modellauges, welche insbesondere die optische Wirkung der Linse des Modellauges zumindest teilweise festlegen, und ein Linsen-Netzhaut-Abstand $d_{LR}$ (dieser Abstand zwischen der Linse, insbesondere der Linsenrückfläche, und der Netzhaut des Modellauges wird auch als Glaskörperlänge bezeichnet) in bestimmter Weise, nämlich derart festlegt, dass das Modellauge die bereitgestellten individuellen Refraktionsdaten aufweist, d.h. dass eine im Modellauge von einem Punkt der Netzhaut des Modellauges auslaufende Wellenfront mit der für das reale Auge des Brillenträger ermittelte (z.B. gemessene oder anderweitig ermittelte) Wellenfront (bis zu einer gewünschten Genauigkeit) übereinstimmt. Optische Eigenschaften und brechende Flächen im Augenmodell werden dabei derart festgelegt, dass sie auch (zumindest einen) Abbildungsfehler höherer Ordnung beschreiben.

Als Parameter der Linse des Modellauges (Linsenparameter) können beispielsweise entweder geometrische Parameter (Form der Linsenflächen und deren Abstand) und vorzugsweise Materialparameter (z.B. Brechungsindizes der einzelnen Komponenten des Modellauges) so vollständig festgelegt werden, dass diese eine optische Wirkung der Linse zumindest teilweise festlegen. Alternativ oder zusätzlich können als Linsenparameter auch Parameter festgelegt werden, die die optische Wirkung der Linse des Modellauges direkt beschreiben.

So wird in einem einfachen Fall eines Augenmodells die Refraktion des Auges durch das optische System bestehend aus der Hornhautvorderfläche, der Augenlinse und der Netzhaut bestimmt. In diesem einfachen Modell legen die Lichtbrechung an der Hornhautvorderfläche und die Brechkraft der Augenlinse (einschließlich der sphärischen und astigmatischen Aberrationen und zumindest einer Aberrationen höherer Ordnung) zusammen mit deren Positionierung relativ zur Netzhaut die Refraktion des Modellauges fest.

Dabei werden die einzelnen Größen (Parameter) des Modellauges anhand von individuellen Messwerten für das Auge des Brillenträgers und/oder anhand von Standardwerten und/oder anhand der bereitgestellten individuellen Refraktionsdaten entsprechend festgelegt. Insbesondere können manche der Parameter (z.B. die Vorderkammertiefe und/oder zumindest eine Krümmung einer Linsenfläche, usw.) direkt als individuelle Messwerte bereitgestellt werden. Andere Werte können auch - insbesondere dann, wenn es sich um Parameter handelt, deren individuelle Messung sehr aufwendig ist - aus Werten von Standardmodellen für ein menschliches Auge übernommen werden. Insgesamt müssen aber nicht alle (geometrischen) Parameter des Modellauges aus individuellen Messungen oder aus Standardmodellen vorgegeben werden. Vielmehr kann für einen oder mehrere (freie) Parameter eine individuelle Anpassung durch Berechnung unter Berücksichtigung der vorgegebenen Parameter derart vorgenommen, dass das dann resultierende Modellauge die bereitgestellten individuellen Refraktionsdaten aufweist. Je nach Anzahl der in den bereitgestellten individuellen Refraktionsdaten enthaltenen Parameter können entsprechend viele (freie) Parameter des Augenmodells individuell angepasst (gefittet) werden.

Nachfolgend beschriebene Details eines Modellauges, welches für eine Berechnung oder Optimierung eines Brillenglases insbesondere für eine Strahl- und Wellenfrontdurchrechnung, genutzt werden kann, finden vorzugsweise in analoger Weise auch Anwendung für ein Modellauge zur Ermittlung der individueller Aberrationsdaten des zumindest einen Auges des Brillenträgers.

In einem Aspekt bietet die Erfindung die Möglichkeit, die ermittelten individuellen Aberrationsdaten zu nutzen, um optimierte sphärozylindrische Werte für zumindest ein Auge eines Brillenträgers zu ermitteln. Diese dienen dann vorzugsweise zur Auswahl einer Vorder- und/oder Rückfläche für ein zu fertigendes Brillenglas. Es ist auch möglich, ausgehend von vorgegebenen Vorder- und/oder Rückflächen anhand der optimierten sphärozylindrischen Werte eine Modifikation der Vorder- und/oder der Rückfläche als solche oder der relativen Lage zueinander vorzunehmen, um dann basierende auf der Modifikation ein Brillenglas zu fertigen.

Vorzugsweise umfasst ein computerimplementiertes Verfahren zum Ermitteln optimierter sphärozylindrischer Werte für zumindest ein Auge eines Brillenträgers:

- Ermitteln von subjektiven sphärozylindrischen Refraktionswerten;
- Ermitteln von objektiven sphärozylindrischen Refraktionswerten, welches umfasst:

  -- Bereitstellen individueller Aberrationsdaten, welche mittels eines Verfahrens gemäß der vorliegenden Erfindung, insbesondere in einer der hier beschriebenen bevorzugten Ausführungsformen, ermittelt wurden;
  -- Ermitteln einer Referenzwellenfront an einer Bewertungsfläche anhand der bereitgestellten individuellen Aberrationsdaten des Auges;
  -- Vorgeben einer Startvorgabe für eine zu optimierende Wellenfront, welche zu optimierende, objektive sphärozylindrische Refraktionswerte beschreibt, an der Bewertungsfläche;
  -- Ermitteln einer Differenzwellenfront aus der zu optimierenden Wellenfront und der Referenzwellenfront;
  -- Bewerten der Differenzwellenfront anhand einer vorgestimmten Metrik (z.B. gemäß WO 2008/089999 A1);
  -- Ermitteln der zu optimierenden Wellenfront derart, dass die Bewertung der Differenzwellenfront vorbestimmte Sollkriterien erfüllt; und
  -- Ermitteln der objektiven sphärozylindrischen Refraktionswerte aus der ermittelten zu optimierenden Wellenfront; und

- Ermitteln der optimierten sphärozylindrischen Werte als gewichteter Mittelwert aus den ermittelten subjektiven sphärozylindrischen Refraktionswerten und den ermittelten objektiven sphärozylindrischen Refraktionswerten.

Alternativ kann anstelle der Wellenfront auch die Point-Spread-Function einer entsprechenden Abbildung betrachtet werden.

Basierend auf diesen optimierten sphärozylindrischen Werten bietet die Erfindung somit in einem Aspekt ein computerimplementiertes Verfahren zum Berechnen oder Optimieren eines Brillenglases für zumindest ein Auge eines Brillenträgers umfassend:

- Ermitteln der optimierten sphärozylindrischen Werte für zumindest ein Auge eines Brillenträgers auf Basis individueller Aberrationsdaten insbesondere in einer der hier beschriebenen Weisen;
- Ermitteln einer Kombination aus Vorder- und Rückfläche (sphärisch, zylindrisch, asphärisch, atorisch, progressiv, ...) auf Basis der ermittelten optimierten sphärozylindrischen Werte; und
- gegebenenfalls Modifizieren der ermittelten Vorderfläche und/oder Rückfläche auf Basis der Basis der ermittelten optimierten sphärozylindrischen Werte.

Alternativ kann das Verfahren umfassen:

- Ermitteln der optimierten sphärozylindrischen Werte für zumindest ein Auge eines Brillenträgers auf Basis individueller Aberrationsdaten insbesondere in einer der hier beschriebenen Weisen;
- Festlegen einer Kombination aus Vorder- und Rückfläche als Startfläche; und
- Modifizieren der Vorderfläche und/oder Rückfläche auf Basis der Basis der ermittelten optimierten sphärozylindrischen Werte.

In einem weiteren Aspekt betrifft die Erfindung ein computerimplementiertes Verfahren zum Berechnen oder Optimieren eines Brillenglases für zumindest ein Auge eines Brillenträgers umfassend ein Bereitstellen individueller Aberrationsdaten, welche mittels eines hier beschriebenen erfindungsgemäßen Verfahrens zum Ermitteln individueller Aberrationsdaten zumindest eines Auges eines Brillenträgers, insbesondere in einer der hier beschriebenen bevorzugten Ausführungsformen, ermittelt wurden. In einer bevorzugten Ausführungsform umfasst das Verfahren zum Berechnen oder Optimieren eines Brillenglases ein entsprechendes Verfahren zum Ermitteln individueller Aberrationsdaten zumindest eines Auges eines Brillenträgers.

Außerdem umfasst das Verfahren zum Berechnen oder Optimieren eines Brillenglases insbesondere

- Ermitteln einer Referenzaberration an einer Bewertungsfläche anhand der bereitgestellten individuellen Aberrationsdaten des Auges;
- Vorgeben einer ersten Fläche und einer zweiten Fläche für das zu berechnende bzw. optimierende Brillenglas;
- Ermitteln des Verlaufs eines Hauptstrahls durch zumindest einen Durchblickspunkt (*i*) zumindest einer zu berechnenden oder optimierenden Fläche des Brillenglases;
- Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration; und
- iteratives Variieren der zumindest einen zu berechnenden oder optimierenden Fläche des Brillenglases bis die ausgewertete Aberration einer vorgegebenen Sollaberration entspricht.

Vorzugsweise liegt die Bewertungsfläche an der Scheitelpunktkugel.

Für die Berechnung bzw. Optimierung des Brillenglases werden somit eine erste Fläche und eine zweite Fläche des Brillenglases insbesondere als Startflächen mit einer vorgegebenen (individuelle) Position relativ zum Modellauge vorgegeben. In einer bevorzugten Ausführungsform wird nur eine der beiden Flächen optimiert. Vorzugsweise handelt es sich hierbei um die Rückfläche des Brillenglases. Vorzugsweise wird dabei sowohl für die Vorderfläche als auch für die Rückfläche des Brillenglases eine entsprechende Startfläche vorgegeben. In einer bevorzugten Ausführungsform wird während des Optimierungsverfahrens aber nur eine Fläche iterativ verändert bzw. optimiert. Die andere Fläche des Brillenglases kann zum Beispiel eine einfache sphärische oder rotationssymmetrische asphärische Fläche sein. Allerdings ist es auch möglich, beide Flächen zu optimieren.

Ausgehend von den beiden vorgegebenen Flächen umfasst das Verfahren ein Ermitteln des Verlaufs eines Hauptstrahls durch zumindest einen Durchblickspunkt (*i*) zumindest einer zu berechnenden oder optimierenden Fläche des Brillenglases in das Modellauge. Der Hauptstrahl beschreibt den geometrischen Strahlverlauf ausgehend von einem Objektpunkt durch die beiden Brillenglasflächen und zumindest die Hornhautvorderfläche, vorzugsweise auch durch die Linse des Modellauges insbesondere bis zur Netzhaut des Modellauges.

Außerdem umfasst das Verfahren ein Auswerten einer Aberration einer entlang des Hauptstrahls propagierenden, aus einer auf die erste Fläche des Brillenglases auftreffenden sphärischen Wellenfront resultierenden Wellenfront an einer Bewertungsfläche innerhalb des Modellauges im Vergleich zu einer in einem Punkt auf der Netzhaut des Augenmodells konvergierenden Wellenfront (Referenzwellenfront oder Referenzlicht). Vorzugsweise umfasst das Auswerten der Aberration dabei ein Vergleichen zumindest einer Aberration höherer Ordnung (HOA). Dazu wird also sowohl die Propagation und Brechung der auf die erste Fläche des Brillenglases treffenden sphärischen Wellenfront auf dem Weg zum Auge beziehungsweise im Auge einschließlich zumindest eines Abbildungsfehlers höherer Ordnung berechnet, also auch die Referenzwellenfront mit dem zumindest einen Abbildungsfehler höherer Ordnung bereitgestellt.

Insbesondere wird dazu eine auf die erste Fläche (Vorderfläche) des Brillenglases entlang des Hauptstrahls auftreffende, sphärische Wellenfront ($w_0$) vorgegeben. Diese sphärische Wellenfront beschreibt das von einem Objektpunkt ausgehende Licht (Objektlicht). Die Krümmung der sphärischen Wellenfront beim Auftreffen auf die erste Fläche des Brillenglases entspricht dem Kehrwert des Objektabstandes. Vorzugsweise umfasst das Verfahren somit ein Vorgeben eines Objektabstandsmodells, welches jeder Blickrichtung oder jedem Durchblickspunkt der zumindest einen zu optimierenden Fläche des Brillenglases eine Objektentfernung zuordnet. Damit wird vorzugsweise die individuelle Gebrauchssituation, in der das herzustellende Brillenglas zum Einsatz kommen soll, beschrieben.

Die auf das Brillenglas auftreffende Wellenfront wird nun an der Vorderfläche des Brillenglases vorzugsweise zum ersten Mal gebrochen. Anschießend propagiert die Wellenfront entlang des Hauptstrahls innerhalb des Brillenglases von der Vorderfläche zur Rückfläche, wo sie zum zweiten Mal gebrochen wird. Anschließend propagiert die durch das Brillenglas transmittierte Wellenfront nun entlang des Hauptstrahls weiter bis zur Hornhautvorderfläche des Auges, wo sie wiederum gebrochen wird. Vorzugsweise wird die Wellenfront nach einer weiteren Propagation innerhalb des Auges bis zur Augenlinse auch dort wiederum gebrochen. In der Realität propagiert das Objektlicht nach der Brechung an der Augenlinse weiter bis zur Netzhaut des Auges. Je nach optischen Eigenschaften der einzelnen optischen Elemente (Brillenglasflächen, Hornhautvorderfläche, Augenlinse) führt jeder Brechungsvorgang auch zu einer Deformation der Wellenfront, wobei erfindungsgemäß zumindest ein Abbildungsfehler höherer Ordnung berücksichtigt wird.

Um eine exakte Abbildung des Objektpunktes auf einen Bildpunkt auf der Netzhaut zu erreichen, müsste die Wellenfront die Augenlinse vorzugsweise als konvergierende sphärische Wellenfront verlassen, deren Krümmung genau dem Kehrwert des Abstandes zur Netzhaut entspricht. Ein Vergleich der vom Objektpunkt auslaufenden Wellenfront mit einer (im Idealfall einer perfekten Abbildung) in einem Punkt auf der Netzhaut konvergierenden Wellenfront (Referenzlicht) erlaubt somit die Auswertung einer Fehlanpassung. Dieser Vergleich und damit die Auswertung der Wellenfront des Objektlichts in dem individuellen Augenmodell erfolgt an einer Bewertungsfläche vorzugsweise innerhalb des Modellauges und besonders bevorzugt noch vor der Propagation des Objektlichts von der Augenlinse (z.B. Linsenrückfläche oder Austrittspupille) zur Netzhaut. Um den Vergleich und damit die Auswertung der Wellenfront des Objektlichts durchführen zu können, wird eine entsprechende Referenzwellenfront ermittelt. Die Referenzwellenfront beschreibt dabei eine in einem Punkt auf der Netzhaut des individuellen Augenmodells konvergierende Wellenfront.

Falls die Bewertungsfläche beispielsweise an der Linsenrückfläche der Linse, insbesondere nach der Brechung an der Linsenrückfläche des Modellauges vorgesehen wird, kann die resultierende Wellenfront des Objektlichts vorzugsweise einfach mit einer sphärischen Wellenfront des Referenzlichts verglichen werden. Hierzu umfasst das Verfahren somit vorzugsweise ein Vorgeben einer auf die erste Fläche des Brillenglases auftreffenden sphärischen Wellenfront, ein Ermitteln einer durch die Wirkung zumindest der ersten und zweiten Fläche des Brillenglases, der Hornhautvorderfläche und der Linse des Modellauges aus der sphärischen Wellenfront resultierenden Wellenfront in dem zumindest einen Auge, und eine Auswertung der Aberration der resultierenden Wellenfront im Vergleich zu einer auf die Netzhaut konvergierenden sphärischen Wellenfront.

Falls hingegen eine Bewertungsfläche innerhalb der Linse oder zwischen der Hornhautvorderfläche und der Linse des Modellauges vorgesehen sein soll, wird als Referenzlicht einfach eine umgekehrte Propagation von einem Punkt auf der Netzhaut durch die einzelnen Komponenten des Modellauges bis hin zur Bewertungsfläche simuliert, um dort einen Vergleich des Objektlichts mit dem Referenzlicht vorzunehmen.

Wie allerdings bereits eingangs erwähnt, ist eine vollständige Korrektion der Refraktion des Auges gleichzeitig für alle Blickrichtungen des Auges, also für alle Durchblickspunkte der zumindest einen zu optimierenden Brillenglasfläche, im Allgemeinen nicht möglich. Je nach Blickrichtung wird somit vorzugsweise eine absichtliche Fehlanpassung des Brillenglases vorgegeben, welche je nach Anwendungssituation insbesondere in den hauptsächlich genutzten Bereichen des Brillenglases (z.B. zentrale Durchblickspunkte) gering, in den wenig genutzten Bereichen (z.B. periphere Durchblickspunkte) etwas höher sind. Diese Vorgehensweise ist dem Prinzip nach aus herkömmlichen Optimierungsverfahren bereits bekannt.

Um das Brillenglas zu optimieren, wird nun die zumindest eine zu berechnende oder optimierende Fläche des Brillenglases solange iterativ variiert, bis eine Aberration der resultierenden Wellenfront einer vorgegebenen Sollaberration entspricht, also insbesondere um vorgegebene Werte der Aberration von der Wellenfront des Referenzlichts (z.B. einer sphärischen Wellenfront, deren Krümmungsmittelpunkt auf der Netzhaut liegt) abweicht. Die Wellenfront des Referenzlichts wird hier auch als Referenzwellenfront bezeichnet. Vorzugsweise umfasst das Verfahren dazu ein Minimieren einer Zielfunktion F, insbesondere analog zu der eingangs bereits beschriebenen Zielfunktion, wobei darin - wie weiter unten noch beschrieben - Abbildungsfehler höherer Ordnung berücksichtigt werden.

Im Rahmen eines bevorzugten Aspekts der vorliegenden Erfindung wurde somit vorgeschlagen, für die Berechnung bzw. Optimierung eines Brillenglases ein derart individuelles Augenmodell festzulegen, welches vorzugsweise bis zur Netzhaut individuell an den einzelnen Brillenträger angepasst ist. An diesem individuellen Augenmodell wird dann eine numerische Strahl- und Wellenfrontberechnung derart durchgeführt, dass diese durch die Bewertungsfläche vorzugsweise in zwei Abschnitte unterteilt wird, von denen ein erster Abschnitt für jeden Durchblickspunkt der zumindest einen zu berechnenden bzw. optimierenden Fläche des Brillenglases eine Berechnung des Objektlicht bis zum individuellen Modellauge oder in das individuelle Modellauge hinein umfasst, während ein zweiter Abschnitt das Ermitteln der dem individuellen Augenmodell entsprechenden Referenzwellenfront umfasst. Sowohl die Berechnung des Objektlichts als auch die Ermittlung der Referenzwellenfront erfolgt unter Berücksichtigung von Abbildungsfehlern höherer Ordnung der jeweiligen Wellenfront und der brechenden Grenzflächen im Augenmodell. Dazu wird das Augenmodell anhand der bereitgestellten, insbesondere gemessenen Abbildungsfehler höherer Ordnung der Hornhaut des Auges des Brillenträgers mit entsprechenden Werten der einzelnen Parameter zur Beschreibung der Flächenformen (z.B. Linsenvorderfläche und/oder Linsenrückfläche) und gegebenenfalls unter Berücksichtigung von Standardwerten für einige dieser Parameter belegt.

Damit wird für jeden Durchblickspunkt insbesondere eine Propagation des Objektlichts einschließlich von HOA berechnet. Auch die Referenzwellenfront wird vorzugsweise ausgehend von der Netzhaut des Modellauges (in Rückwärtspropagation) bis zur Bewertungsfläche hin ebenfalls einschließlich von HOA berechnet. An der Bewertungsfläche werden die beiden Wellenfront einschließlich HOA verglichen.

Im Rahmen der vorliegenden Erfindung stellte sich heraus, dass die Berücksichtigung von HOA insbesondere in einem Augenmodell selbst dann zu einer deutlichen Verbesserung der individuellen Anpassung führt, wenn die HOA des gesamten Auges nicht vollständig individuell gemessen werden, sondern im Augenmodell aus individuellen Messungen nur der HOA der Augenhornhaut (z.B. Form der Hornhautvorderfläche) und unter Standardannahmen für die HOA des Auges und/oder der Augenlinse abgeleitet werden. Damit ist es nicht erforderlich, die HOA des gesamten Auges zu messen, sondern man kann bereits anhand der weniger aufwendig zu ermittelnden Form der Hornhautvorderfläche eine sehr gute individuelle Anpassung des Brillenglases erreichen.

Insbesondere wird im Rahmen der Erfindung die Hornhautvorderfläche individuell gemessen und vorzugsweise die Augenlinse des individuellen Augenmodells entsprechend berechnet und/oder zumindest teilweise anhand von Standartwerten festgelegt, um zumindest die individuell bestimmten Refraktionsdaten zu erfüllen. Dabei wird in einer bevorzugten Ausführungsform die Hornhautvorderfläche (bzw. ihre Krümmung) entlang der Hauptschnitte individuell gemessen (Topometrie). Das Festlegen der Form der Hornhautvorderfläche des Modellauges anhand dieser Messungen erfolgt dabei derart, dass dadurch die Abbildungsfehler der Hornhaut des Auges einschließlich der HOA der Hornhaut beschrieben werden. In einer weiteren bevorzugten Ausführungsform wird die Topographie der Hornhautvorderfläche (d.h. die vollständige Beschreibung der Fläche) individuell gemessen. Das Festlegen der Form der Hornhautvorderfläche des Modellauges anhand dieser Messungen erfolgt dabei derart, dass dadurch die Abbildungsfehler der Hornhaut des Auges einschließlich der HOA der Hornhaut beschrieben werden.

In einer weiteren bevorzugten Ausführungsform erfolgt auch das Festlegen des Hornhaut-Linsen-Abstandes anhand von individuellen Messwerten für den Hornhaut-Linsen-Abstand.

Besonders bevorzugt umfasst das Festlegen der Parameter der Linse des Modellauges ein Festlegen folgender Parameter:

-- eine Form der Linsenvorderfläche einschließlich zumindest eines Abbildungsfehlers höherer Ordnung der Linsenvorderfläche;
-- eine Linsendicke; und
-- eine Form der Linsenrückfläche einschließlich zumindest eines Abbildungsfehlers höherer Ordnung der Linsenrückfläche.

Vorzugsweise erfolgt das Festlegen der Linsendicke und des Linsen-Netzhaut-Abstands anhand von vorgegebenen Standardwerten erfolgt. Dabei umfasst das Verfahren vorzugsweise außerdem ein Vorgeben von Standardwerten für Abbildungsfehler höherer Ordnung (HOA) des Auges sowie Standardwerten für die Form der Linsenrückfläche, einschließlich von Aberrationen höherer Ordnung der Linsenrückfläche. Auf Basis dieser vorgegebenen Werte und der bereitgestellten individuellen Refraktionsdaten des Auges und Aberrationen der Hornhaut und der Linsenrückfläche wird vorzugsweise die Form der Linsenvorderfläche einschließlich von Abbildungsfehlern höherer Ordnung der Linsenvorderfläche durch Berechnen ermittelt.

In einer alternativen, bevorzugten Implementierung wird anstelle der Form der Linsenrückfläche (einschließlich HOA) eine Form der Linsenvorderfläche einschließlich von HOA der Linsenvorderfläche als Standardwerte vorgegeben. Auf Basis der ansonsten vorgegebenen Werte und der bereitgestellten individuellen Refraktionsdaten des Auges und Aberrationen der Hornhaut und der Linsenvorderfläche wird vorzugsweise die Form der Linsenrückfläche einschließlich von Abbildungsfehlern höherer Ordnung der Linsenrückfläche durch Berechnen ermittelt.

In einer weiteren alternativen, bevorzugten Implementierung erfolgt das Festlegen der Linsendicke anhand von vorgegebenen Standardwerten, wobei das Verfahren außerdem ein Vorgeben von Standardwerten für Abbildungsfehler höherer Ordnung (HOA) des Auges umfasst. Insbesondere wird somit eine Vergenzmatrix $\mathbf{S}_M$ einschließlich Abbildungsfehler niedriger und höherer Ordnung bereitgestellt, wobei die Abbildungsfehler niedriger Ordnung den individuellen Refraktionsdaten des Auges und die Abbildungsfehler höherer Ordnung den vorgegebenen Standardwerten entsprechen. Vorzugsweise wird darüber hinaus für zumindest eine der Linsenvorderfläche und der Linsenrückfläche die Form einschließlich von Abbildungsfehlern höherer Ordnung anhand von vorgegebenen Standardwerten einschließlich bereitgestellt. Für die andere Linsenfläche wird vorzugsweise zumindest ein Parameter eines Abbildungsfehlers niedriger Ordnung, insbesondere eine Krümmung in einem Normalschnitt insbesondere durch individuelle Messung bereitgestellt. Auf Basis dieser Daten werden dann der Linsen-Netzhaut-Abstand sowie die übrigen Parameter der anderen Linsenfläche durch Berechnen ermittelt.

In einer weiteren alternativen, bevorzugten Implementierung erfolgt das Festlegen der Linsendicke und des Linsen-Netzhaut-Abstands anhand von vorgegebenen Standardwerten. Darüber hinaus erfolgt besonders bevorzugt das Festlegen der Form der Linsenvorderfläche und der Linsenrückfläche anhand von vorgegebenen Standardwerten für die Abbildungsfehler höherer Ordnung der jeweiligen Fläche. Lediglich für die Hornhaut des Auges des Brillenträgers werden die HOA individuell gemessen und im Augenmodell berücksichtigt. Besonders bevorzugt werden dabei die Standardwerte der Abbildungsfehler höherer Ordnung der Linsenvorderfläche und der Linsenrückfläche auf null gesetzt.

In einer bevorzugten Ausführungsform liegt die Bewertungsfläche an der Austrittspupille des Modellauges. Vorzugsweise liegt die Bewertungsfläche an einer Grenzfläche des Modellauges, insbesondere innerhalb des Modellauges, insbesondere an der Linsenrückfläche oder an der Linsenvorderfläche oder an der Hornhaut beziehungsweise an einer Fläche (Grenzfläche) der Hornhaut (z.B. Hornhautrückfläche). Besonders bevorzugt umfasst dabei das Auswerten der Aberration der entlang des Hauptstrahls propagierenden Wellenfront an der Bewertungsfläche ein Berechnen einer Brechung der Wellenfront an der Grenzfläche, an welcher die Bewertungsfläche liegt. Der Wechsel von Propagationsschritten und Brechungsschritten in der numerischen Beschreibung und Berechnung des Verlaufs des Objektlichts endet somit mit einem Brechungsschritt, während der nachfolgende Propagationsschritt bereits Teil der Simulation der Referenzwellenfunktion darstellt. Gerade diese Vorgehensweise hat sich als besonders bevorzugt herausgestellt. Insbesondere stellt die Berechnung der Propagation der Wellenfronten hohe Anforderungen an numerische Berechnungseinheiten und benötigt vergleichsweise viel Prozesszeit. Durch das Abbrechen der Berechnung des Objektlichts nach einer Brechung, muss die nachfolgende Lichtpropagation nicht für jeden Durchblickspunkt und jeden Iterationsschritt erneut berechnet werden. Stattdessen kann für jeden Iterationsschritt dieselbe Referenzwellenfunktion verwendet werden, wobei dennoch eine ausgesprochen gute individuelle Anpassung des Brillenglases erreicht wird, zumindest soweit die Referenzwellenfunktion vorzugsweise auf dem individuellen Augenmodell basiert.

In einem weiteren Aspekt betrifft die Erfindung eine Vorrichtung zum Ermitteln individueller Aberrationsdaten zumindest eines Auges eines Brillenträgers umfassend:

- eine Datenschnittstelle zum Bereitstellen einer gemessenen Hornhauttopographie des zumindest einen Auges des Brillenträgers (oder eine Messvorrichtung zum Messen der Hornhauttopographie des zumindest einen Auges);
- ein Hornhautauswertemodul zum Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und
- ein Berechnungsmodul zum Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

In einem weiteren Aspekt betrifft die Erfindung eine Vorrichtung zum Berechnen oder Optimieren eines Brillenglases für zumindest ein Auge eines Brillenträgers umfassend:

- eine Datenschnittstelle zum Bereitstellen individueller Aberrationsdaten, welche mittels eines Verfahrens gemäß der vorliegenden Erfindung insbesondere in einer der hier beschriebenen bevorzugten Ausführungsformen, ermittelt wurden;
- ein Modellierungsmodul zum Ermitteln einer Referenzaberration an einer Bewertungsfläche anhand der bereitgestellten individuellen Aberrationsdaten des Auges;
- eine Flächenmodelldatenbank zum Vorgeben einer ersten Fläche und einer zweiten Fläche für das zu berechnende bzw. optimierende Brillenglas;
- ein Hauptstrahlermittlungsmodul zum Ermitteln des Verlaufs eines Hauptstrahls durch zumindest einen Durchblickspunkt (*i*) zumindest einer zu berechnenden oder optimierenden Fläche des Brillenglases;
- ein Auswertemodul zum Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration; und
- ein Optimierungsmodul zum iterativen Variieren der zumindest einen zu berechnenden oder optimierenden Fläche des Brillenglases bis die ausgewertete Aberration einer vorgegebenen Sollaberration entspricht.

Weitere Aspekte

In den folgenden Absätzen werden, soweit nicht ausdrücklich etwas anderes erwähnt ist, Aspekte beschrieben, die sowohl für den ersten als auch für den zweiten Ansatz der Erfindung relevant sind:
Außerdem bietet die Erfindung ein Verfahren zum Herstellen eines Brillenglases umfassend:

Berechnen oder Optimieren eines Brillenglases nach dem Verfahren zum Berechnen oder Optimieren eines Brillenglases gemäß einem der vorliegenden Erfindung insbesondere in einer bevorzugten Ausführungsform; und
Fertigen des so berechneten oder optimierten Brillenglases.

Außerdem bietet die Erfindung eine Vorrichtung zum Herstellen eines Brillenglases umfassend:

Berechnungs- oder Optimierungsmittel, welche ausgelegt sind, das Brillenglas nach einem Verfahren zum Berechnen oder Optimieren eines Brillenglases gemäß der vorliegenden Erfindung insbesondere in einer bevorzugten Ausführungsform zu berechnen oder zu optimieren;
Bearbeitungsmittel, welche ausgelegt sind, das Brillenglas fertig zu bearbeiten.

Außerdem bietet die Erfindung eine Verwendung eines nach dem Herstellungsverfahren gemäß der vorliegenden Erfindung insbesondere in einer bevorzugten Ausführungsform hergestellten Brillenglases in einer vorgegebenen durchschnittlichen oder individuellen Gebrauchsstellung des Brillenglases vor den Augen eines bestimmten Brillenträgers zur Korrektur einer Fehlsichtigkeit des Brillenträgers.

Bevorzugte Ausführungsformen der Erfindung werden nachfolgend zumindest teilweise unter Bezugnahme auf die beigefügte Zeichnung beispielhaft erläutert. Dabei zeigt:

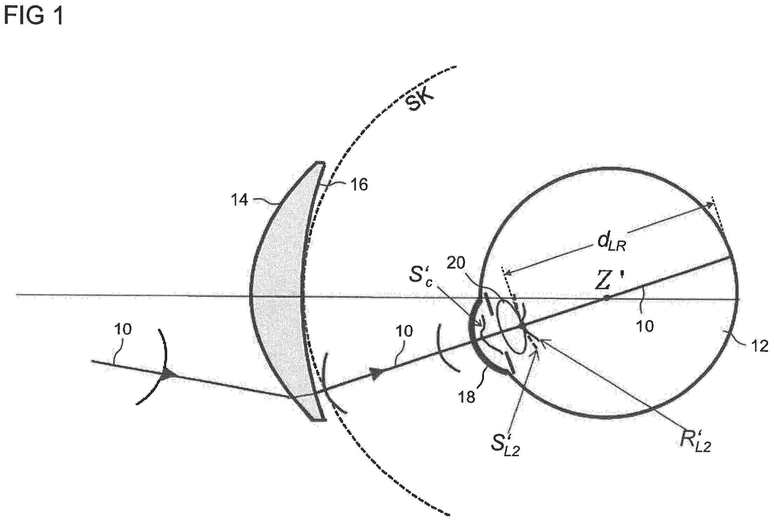

Fig. 1 eine schematische Darstellung des physiologischen und physikalischen Modells eines Brillenglases und eines Auges zusammen mit einem Strahlverlauf in einer vorgegebenen Gebrauchsstellung

Erster Ansatz

In den folgenden Absätzen werden, soweit nicht ausdrücklich etwas anderes erwähnt ist, zunächst Details zu beispielhaften und bevorzugten Implementierungen des ersten Ansatzes der Erfindung beschrieben:
Fig. 1 zeigt eine schematische Darstellung des physiologischen und physikalischen Modells eines Brillenglases und eines Auges in einer vorgegebenen Gebrauchsstellung zusammen mit einem beispielhaften Strahlverlauf, welches einer individuellen Brillenglasberechnung bzw. -optimierung gemäß einer bevorzugten Ausführungsform der Erfindung zugrunde liegt.

Hierbei wird pro Durchblickpunkt des Brillenglases vorzugsweise nur ein einziger Strahl berechnet (der Hauptstrahl 10, der vorzugsweise durch den Augendrehpunkt Z' läuft), begleitend aber außerdem auch die Ableitungen der Pfeilhöhen der Wellenfront nach den transversalen (zum Hauptstrahl senkrechten) Koordinaten. Diese Ableitungen werden bis zur gewünschten Ordnung berücksichtigt, wobei die zweiten Ableitungen die lokalen Krümmungseigenschaften der Wellenfront beschreiben und die höheren Ableitungen mit den Abbildungsfehlern höherer Ordnungen zusammenhängen.

Bei der Durchrechnung von Licht durch das Brillenglas bis in das Auge 12 gemäß dem individuell bereitgestellten Augenmodell werden die lokalen Ableitungen der Wellenfronten im Endeffekt an einer geeigneten Position im Strahlverlauf ermittelt, um sie dort mit einer Referenzwellenfront zu vergleichen, welche in einem Punkt auf der Retina des Auges 12 konvergiert. Insbesondere werden die beiden Wellenfronten (d.h. die vom Brillenglas kommende Wellenfront und die Referenzwellenfront) in einer Bewertungsfläche miteinander verglichen.

Unter "Position" ist dabei nicht einfach nur ein bestimmter Wert der z-Koordinate (in Lichtrichtung) gemeint, sondern ein solcher Koordinatenwert in Kombination mit der Angabe aller Flächen, durch die vor Erreichen der Bewertungsfläche gebrochen wurde. In einer bevorzugten Ausführungsform wird durch alle brechenden Flächen einschließlich der Linsenrückfläche gebrochen. In diesem Fall dient als Referenzwellenfront vorzugsweise eine sphärische Wellenfront, deren Krümmungsmittelpunkt auf der Retina des Auges 12 liegt.

Besonders bevorzugt wird ab dieser letzten Brechung nicht weiter propagiert, so dass der Krümmungsradius dieser Referenzwellenfront gerade dem Abstand zwischen Linsenrückfläche und Retina entspricht. In einer außerdem bevorzugten Ausführungsform wird nach der letzten Brechung noch propagiert, und zwar bevorzugt bis zur Austrittspupille AP des Auges 12. Diese liegt beispielsweise im Abstand $d_{AR} = d_{LR}^{(b)} = d_{LR} - d_{LR}^{(a)} > d_{LR}$ vor der Retina und damit sogar vor der Linsenrückfläche, so dass die Propagation in diesem Fall eine Rückpropagation ist (die Bezeichnungen $d_{LR}^{(a)}$, $dLR$ werden weiter unten noch bei der Aufzählung der Schritte 1-6 beschrieben). Auch in diesem Fall ist die Referenzwellenfront sphärisch mit Krümmungsmittelpunkt auf der Retina, besitzt aber Krümmungsradius $1/d_{AR}$.

Hierzu wird angenommen, dass eine sphärische Wellenfront $w_0$ vom Objektpunkt ausgeht und bis zur ersten Brillenglasfläche 14 propagiert. Dort wird sie gebrochen und anschließend propagiert sie bis zur zweiten Brillenglasfläche 16, wo sie wieder gebrochen wird. Die vom Brillenglas austretenden Wellenfront $w_{g1}$ propagiert anschließend entlang des Hauptstrahls in Richtung des Auges 12 (propagierte Wellenfront $w_{g2}$) bis sie auf die Cornea 18 trifft, wo sie wiederum gebrochen wird (Wellenfront $w_c$). Nach einer weiteren Propagation innerhalb der Augenvorderkammer bis zur Augenlinse 20, wird die Wellenfront auch von der Augenlinse 20 wiederum gebrochen, wodurch beispielsweise an der Rückfläche der Augenlinse 20 oder an der Austrittspupille des Auges die resultierenden Wellenfront $w_e$ entsteht. Diese wird mit der sphärischen Referenzwellenfront $w_s$ verglichen und die Abweichungen werden für alle Durchblickspunkte in der Zielfunktion (vorzugsweise mit entsprechenden Gewichtungen für die einzelnen Durchblickspunkte) bewertet.

Somit wird die Fehlsichtigkeit nicht mehr nur durch eine dünne sphäro-zylindrische Linse beschrieben, wie dies in vielen herkömmlichen Verfahren üblich war, sondern vorzugsweise werden die Hornhauttopographie, die Augenlinse, die Abstände im Auge und die Deformation der Wellenfront (einschließlich der Abbildungsfehler niedriger Ordnung - also Sphäre, Zylinder und Achslage - sowie vorzugsweise auch einschließlich der Abbildungsfehler höherer Ordnung) im Auge direkt berücksichtigt. Dabei wird im erfindungsgemäßen Augenmodell die Glaskörperlänge $d_{LR}$ individuell berechnet.

Vorzugsweise liefert eine Aberrometermessung die individuellen Wellenfrontdeformationen des realen fehlsichtigen Auges für Ferne und Nähe (Abweichungen, keine absoluten Brechwerte) und die individuellen mesopischen und photopischen Pupillendurchmesser. Aus einer Messung der Hornhauttopographie (flächenhafte Vermessung der Cornea-Vorderfläche) erhält man vorzugsweise eine individuelle reale Hornhautvorderfläche, die im Allgemeinen nahezu 75% des Gesamtbrechwertes des Auges ausmacht. In einer bevorzugten Ausführungsform ist es nicht notwendig, die Cornea-Rückfläche zu vermessen. Sie wird vorzugsweise wegen des geringen Brechzahlunterschiedes zum Kammerwasser und wegen der geringen Hornhautdicke in guter Näherung nicht durch eine separate brechende Fläche, sondern durch eine Anpassung des Brechungsindex der Cornea beschrieben.

Generell sollen in dieser Beschreibung fett gesetzte Kleinbuchstaben Vektoren und fett gesetzte Großbuchstaben Matrizen bezeichnen, wie z.B. die (2 x 2)-Vergenzmatrizen bzw. -Brechwertmatrizen

$$\mathbf{S} = \begin{pmatrix} S_{xx} & S_{xy} \\ S_{xy} & S_{yy} \end{pmatrix}, \qquad \mathbf{C} = \begin{pmatrix} C_{xx} & C_{xy} \\ C_{xy} & C_{yy} \end{pmatrix}, \qquad \mathbf{L} = \begin{pmatrix} L_{xx} & L_{xy} \\ L_{xy} & L_{yy} \end{pmatrix}, \qquad \mathbf{1} = \begin{pmatrix} 1 & 0 \\ 0 & 1 \end{pmatrix}$$

und kursiv gesetzte wie d skalare Größen.

Weiterhin sollen fette, kursiv gesetzte Großbuchstaben Wellenfronten oder Flächen als Ganzes bezeichnen. So ist z.B. S die Vergenzmatrix der gleichnamigen Wellenfront S, nur dass *S* außer den Aberrationen 2-ter Ordnung, die in S zusammengefasst sind, auch die Gesamtheit aller Aberrationen höherer Ordnung (=HOA für engl. *Higher Order Aberrations*) der Wellenfront mit umfasst. Mathematisch gesehen steht *S* für die Menge aller Parameter, die notwendig sind, um eine Wellenfront (ausreichend genau) in Bezug auf ein gegebenes Koordinatensystem zu beschreiben. Vorzugsweise steht *S* für einen Satz von Zernike-Koeffizienten mit einem Pupillenradius oder einen Satz aus Koeffizienten einer Taylorreihe. Besonders bevorzugt steht *S* für die Menge aus einer Vergenzmatrix S zur Beschreibung der Wellenfronteigenschaften 2. Ordnung und einem Satz von Zernike-Koeffizienten (mit einem Pupillenradius), der zur Beschreibung aller restlichen Wellenfronteigenschaften außer der 2.Ordnung dient oder einem Satz von Koeffizienten gemäß einer Zerlegung nach Taylor. Für Flächen statt Wellenfronten gelten analoge Aussagen.

Unter anderem können folgende Daten im Prinzip direkt gemessen werden:

- Die Wellenfront $S_M$, die durch den Laserspot auf der Retina und den Durchgang durch das Auge erzeugt wird (aus aberrometrischer Messung)

- Form der Hornhautvorderfläche *C* (durch Hornhaut-Topographie)

- Abstand zwischen Hornhaut und Linsenvorderfläche $d_{CL}$ (durch Pachymetrie). Diese Größe kann auch indirekt durch die Messung des Abstandes zwischen der Hornhaut und der Regenbogenhaut ermittelt werden, dabei können gegebenenfalls Korrekturwerte angewendet werden. Derartige Korrekturen können der Abstand zwischen der Linsenvorderfläche und der Regenbogenhaut aus bekannten Augenmodellen (z.B. Literaturwerte) sein.

- Krümmung der Linsenvorderfläche in einer Richtung $L_{1xx}$ (durch Pachymetrie) Dabei kann ohne Einschränkung der Allgemeinheit die x-Ebene beispielsweise derart definiert werden, dass dieser Schnitt in der x-Ebene liegt. Wird das Koordinatensystem so definiert, dass diese Ebene schief liegt, muss die Ableitung durch die Funktionen des entsprechenden Winkels ergänzt werden. Es wird nicht gefordert, dass es sich dabei um einen Hauptschnitt handelt. Beispielsweise kann es sich um den Schnitt in der horizontalen Ebene handeln.

Weiterhin können folgende Daten - je nach Ausführungsform - entweder gemessen oder der Literatur entnommen werden:

- Dicke der Linse $d_L$

- Krümmung der Linsenrückfläche in derselben Richtung wie die Linsenvorderfläche $L_{2,xx}$ (durch Pachymetrie)

Damit gibt es für die Linsenrückfläche folgende Möglichkeiten:

- Messung von $L_{2,xx}$ ($L_{2,M}$) und Annahme einer Rotationssymmetrie $L_{2,xx} = L_{2,yy} = L_2 = L_{2,M}$ und $L_{2,xy} = L_{2,yx} = 0$

- Entnahme von $L_{2,xx}$ aus der Literatur ($L_{2,Lit}$) und Annahme einer Rotationssymmetrie $L_{2,xx} = L_{2,yy} = L_2 = L_{2,M}$ und $L_{2,xy} = L_{2,yx} = 0$

- Entnahme der vollständigen (unsymmetrischen) Form $L_2$ aus der Literatur ($L_{2,Lit}$)

- Messung von $L_{2,xx}$ ($L_{2,M}$) und Annahme eines Zylinders oder einer anderweitig spezifizierten Asymmetrie $a_{Lit}$ aus der Literatur

$L_{2,xx} = L_{2,M}$ und $L_{2,xy} = L_{2,yx} = f(L_{2,xx}, a_{Lit})$ sowie $L_{2,yy} = g(L_{2,xx}, a_{Lit})$

Folgende Daten können der Literatur entnommen werden:

- Brechungsindices $n_{CL}$ von Hornhaut und Augenvorderkammer sowie des Kammerwassers $n_{LR}$ und der der Linse $n_L$

Damit verbleiben insbesondere der Abstand $d_{LR}$ zwischen Linsenrückfläche und Netzhaut sowie die Komponenten $L_{1,yy}$ und $L_{1,xy} = L_{1,yx}$ der Linsenvorderfläche als unbekannte Parameter. Zur Vereinfachung des Formalismus kann ersterer auch als Vergenzmatrix $\mathbf{D}_{LR} = D_{LR} \cdot \mathbf{1}$ mit $D_{LR} = n_{LR}/d_{LR}$ geschrieben werden. Weiterhin wird generell die Größe $\tau$ verwendet, die definiert ist als $\tau = d/n$ (wobei für den Brechungsindex als $n$ immer der entsprechende Index zu verwenden ist wie für $d$ und $\tau$, z.B. als $\tau_{LR} = d_{LR}/n_{LR}$, $\tau_{CL} = d_{CL}/n_{CL}$).

Die Modellierung des Durchgangs der Wellenfront durch das erfindungsgemäß verwendete Augenmodell, also nach dem Durchgang durch die Flächen des Brillenglases, kann in einer bevorzugten Ausführungsform, in der die Linse über eine Vorder- und eine Rückfläche beschrieben wird, wie folgt beschrieben werden, wobei die Transformationen der Vergenzmatrizen explizit angegeben wird:

1. Brechung der Wellenfront $\mathbf{S}$ mit der Vergenzmatrix $\mathbf{S}$ an der Hornhaut $\mathbf{C}$ mit der Flächenbrechwertmatrix C zur Wellenfront $\mathbf{S'}_c$ mit Vergenzmatrix $\mathbf{S'}_c = \mathbf{S} + \mathbf{C}$
2. Propagation um die Vorderkammertiefe $d_{CL}$ (Abstand zwischen Hornhaut und Linsenvorderfläche) zur Wellenfront $\mathbf{S}_{L1}$ mit Vergenzmatrix $\mathbf{S}_{L1} = \mathbf{S'}_C/(1 - \tau_{CL} \cdot \mathbf{S'})$

$$\mathbf{S}_{L1} = \frac{\mathbf{S'}_C}{(1 - \tau_{CL} \cdot \mathbf{S'}_C)}$$

3. Brechung an der Linsenvorderfläche $\mathbf{L}_1$ mit der Flächenbrechwertmatrix $\mathbf{L}_1$ zur Wellenfront $\mathbf{S'}_{L1}$ mit der Vergenzmatrix $\mathbf{S'}_{L1} = \mathbf{S}_{L1} + \mathbf{L}_1$
4. Propagation um die Linsendicke $d_L$ zur Wellenfront $\mathbf{S}_{L2}$ mit Vergenzmatrix $\mathbf{S}_{L2} = \mathbf{S'}_{L1}/(1 - -r \cdot \mathbf{S'}_{L1})$
5. Brechung an der Linsenrückfläche $\mathbf{L}_2$ mit der Flächenbrechwertmatrix $\mathbf{L}_2$ zur Wellenfront $\mathbf{S'}_{L2}$ mit Vergenzmatrix $\mathbf{S'}_{L2} = \mathbf{S}_{L2} + \mathbf{L}_2$
6. Propagation um den Abstand zwischen Linse und Netzhaut $d_{LR}$ zur Wellenfront $\mathbf{S}_R$ mit der Vergenzmatrix $\mathbf{S}_R = \mathbf{S'}_{L2}/(1 - \tau_{LR} \cdot \mathbf{S'}_{L2})$

Jeder der Schritte 2, 4, 6, bei denen die Abstände $\tau_{CL}$, $\tau_{CL}$, bzw. $\tau_{CL}$ propagiert wird, kann dabei aufgeteilt werden in zwei Teilpropagationen 2a,b), 4a,b) bzw. 6a,b) nach dem folgenden Schema, das für den Schritt 6a,b) explizit lautet:

6a. Propagation um den Abstand $d_{LR}^{(a)}$ $d_{LR}$ zwischen Linse und Zwischenebene zur Wellenfront $\mathbf{S}_{LR}$ mit der Vergenzmatrix $\mathbf{S}_{LR} = \mathbf{S'}_{L2}/(1 - \tau_{LR}^{(a)}\mathbf{S'}_{L2})$

6b. Propagation um den Abstand $d_{LR}^{(b)}$ zwischen Zwischenebene und Netzhaut zur Wellenfront $\mathbf{S}_R$ mit der Vergenzmatrix $\mathbf{S}_R = \mathbf{S}_{LR}/(1 - \tau_{LR}^{(b)}\mathbf{S}_{LR})$

Dabei können $\tau_{LR}^{(a)} = d_{LR}^{(a)}/n_{LR}^{(a)}$ und $\tau_{LR}^{(b)} = d_{LR}^{(b)}/n_{LR}^{(b)}$ positiv oder negativ sein, wobei stets $n_{LR}^{(a)} = n_{LR}^{(b)} = n_{LR}$ und $\tau_{LR}^{(a)} + \tau_{LR}^{(b)} = \tau_{LR}$ sein soll. In jedem Fall lassen sich Schritt 6a und 6b durch

$$\mathbf{S}_R = \mathbf{S'}_{L2}/(1 - (\tau_{LR}^{(a)} + \tau_{LR}^{(b)})\mathbf{S'}_{L2}) = \mathbf{S'}_{L2}/(1 - \tau_{LR}\mathbf{S'}_{L2})$$

wieder zusammenfassen. Die Aufteilung in Schritt 6a und 6b bietet aber Vorteile, und bevorzugt kann die Zwischenebene in die Ebene der Austrittspupille AP gelegt werden, die vorzugsweise vor der Linsenrückfläche liegt. In diesem Fall ist $\tau_{LR}^{(a)} < 0$ und $\tau_{LR}^{(b)} > 0$ .

Analog zur Aufteilung von Schritt 6 in 6a,b) kann auch die Aufteilung der Schritte 2,4 erfolgen.

Entscheidend für die Wahl der Bewertungsfläche der Wellenfront ist also nicht nur die absolute Lage in Bezug auf die z-Koordinate (in Lichtrichtung), sondern auch die Anzahl der Flächen, durch die bis zur Bewertungsfläche schon gebrochen wurde. So kann ein- und dieselbe Ebene mehrmals durchlaufen werden. Beispielhaft wird die Ebene der AP (die normalerweise zwischen der Linsenvorderfläche und der Linsenrückfläche liegt) vom Licht formal zum ersten Mal durchlaufen nach einem gedachten Schritt 4a, bei dem von der Linsenvorderfläche um die Länge $\tau_L^{(a)} > 0$ propagiert wird. Zum zweiten Mal wird die gleiche Ebene erreicht nach Schritt 6a, wenn nach der Brechung durch die Linsenrückfläche wieder zur AP-Ebene zurückpropagiert wird, d.h. $\tau_{LR}^{(a)} = -\tau_L + \tau_L^{(a)} = -\tau_L^{(b)} < 0$ , was gleichbedeutend ist mit

$\tau_{LR}^{(a)} = \tau_{LR} - \tau_{LR}^{(b)} < 0$ . Bei den Wellenfronten $S_{AP}$, die sich im Text auf die AP beziehen, soll (falls nicht explizit anders erwähnt) vorzugsweise immer die Wellenfront $S_{AP} = S_{LR}$ gemeint sein, die Ergebnis von Schritt 6a ist."

Auf diese Schritte 1 bis 6 wird im weiteren Verlauf der Beschreibung immer wieder Bezug genommen. Sie beschreiben einen bevorzugten Zusammenhang zwischen der Vergenzmatrix S einer Wellenfront $S$ an der Hornhaut und den Vergenzmatrizen aller daraus hervorgehenden Zwischenwellenfronten an den brechenden Zwischenflächen des Auges, insbesondere der Vergenzmatrix $S'_{L2}$ einer Wellenfront $S'_{L2}$ nach der Augenlinse (oder sogar einer Wellenfront $S_R$ an der Netzhaut). Diese Zusammenhänge können sowohl dazu verwendet werden, a-priori nicht bekannte Parameter (z.B. $d_{LR}$ oder $L_1$) zu berechnen und so das Modell entweder individuell oder generisch mit Werten zu belegen, als auch um mit dann belegten Modellen die Ausbreitung der Wellenfront im Auge zur Optimierung von Brillengläsern zu simulieren.

In einer bevorzugten Ausführungsform werden die Flächen und Wellenfronten bis in zweiter Ordnung behandelt, wozu eine Darstellung durch Vergenzmatrizen ausreichend ist. Eine später noch beschriebene andere bevorzugte Ausführungsform berücksichtigt und nutzt auch höhere Ordnungen von Abbildungsfehlern.

In einer Beschreibung in zweiter Ordnung besitzt das Augenmodell in einer bevorzugten Ausführungsform zwölf Parameter als Freiheitsgrade des Modells, die belegt werden müssen. Diese umfassen vorzugsweise die drei Freiheitsgrade der Flächenbrechwertmatrix C der Cornea $C$ , die jeweils drei Freiheitsgrade der Flächenbrechwertma-trizen $L_1$ und $L_2$ für die Linsenvorder- bzw. Rückfläche, sowie jeweils einen für die Längenparameter Vorderkammertiefe $d_{CL}$ , Linsendicke $d_L$ und die Glaskörperlänge $d_{LR}$.

Belegungen dieser Parameter können im Prinzip auf mehrere Weisen erfolgen:

i) Direkte, also individuelle Messung eines Parameters

ii) A priori gegebener Wert eines Parameters, z.B. als Literaturwert oder aus einer Schätzung, beispielsweise durch Vorliegen eines Messwertes für eine andere Größe, die anhand einer vorangegangenen Populationsanalyse auf bekannte Weise mit dem zu bestimmenden Parameter korreliert

iii) Berechnung aus Konsistenzbedingungen, z.B. Verträglichkeit mit einer bekannten Refraktion

Die gesamte Anzahl $df_2$ von Freiheitsgraden des Augenmodells in zweiter Ordnung (*df* steht für ‚degree of freedom', Index ‚2' für 2.Ordnung) setzt sich also zusammen aus

$$df_2 = df_2(i) + df_2(ii) + df_2(iii)$$

Liegen beispielsweise für alle zwölf Modellparameter direkte Messwerte vor, dann ist $df_2(i)$ = 12, $df_2(ii)$ = 0 und $df_2(iii)$ = 0, was nachfolgend der Einfachheit halber durch die Schreibweise $df_2$ = 12 + 0 + 0 ausgedrückt wird. In einem solchen Fall ist auch die objektive Refraktion des betreffenden Auges festgelegt, so dass eine objektive Refraktionsbestimmung nicht mehr zusätzlich durchgeführt werden müsste.

Ein zentraler Aspekt der Erfindung betrifft aber gerade das Ziel, nicht alle Parameter direkt messen zu müssen. So ist es insbesondere deutlich einfacher, die Refraktion des betreffenden Auges zu messen bzw. objektiv und/oder subjektiv zu bestimmen, als alle Parameter des Modellauges individuell zu vermessen. Vorzugsweise liegt somit zumindest eine Refraktion, also Messdaten zur Wellenfront $S_M$ des Auges bis zur zweiten Ordnung vor, die den Daten der Vergenzmatrix $S_M$ entsprechen. Bei einer Belegung des Augenmodells auf Basis rein objektiv gemessener Daten können diese Werte aberrometrische Messungen oder autorefraktometrischen Messungen entnommen werden, bzw. laut (ii) anderweitig gegebener Daten belegt werden. Eine Berücksichtigung subjektiver Verfahren (d.h. subjektive Refraktion), sei es als Ersatz der objektiven Messung der Refraktion oder durch die Kombination beider Ergebnisse, wird später noch beschrieben. Die drei Bedingungen der Übereinstimmung mit den drei unabhängigen Parametern der Vergenzmatrix $\mathbf{S}_M$ erlauben es damit, drei Parameter des Augenmodells abzuleiten, was in der oben eingeführten Notation $df_2(iii)$ = 3 entspricht.

Die Erfindung nutzt also die Möglichkeit, in Fällen, in denen nicht alle Modellparameter direkten Messungen zugänglich sind, oder diese Messungen sehr aufwändig wären, die fehlenden Parameter sinnvoll zu belegen. Liegen beispielsweise höchstens für neun Modellparameter direkte Messwerte vor ($df_2(i) \leq 9$), dann kann man die genannten Bedingungen der Refraktion benutzen, um drei der Modellparameter zu berechnen ($df_2(iii)$ = 3). Falls genau $df_2(i)$ = 9 gilt, dann sind alle zwölf Modellparameter durch die Messungen und die Berechnung eindeutig bestimmt, und es gilt ($df_2(ii)$ = 0). Ist dagegen $df_2(i) < 9$ , dann ist $df_2(ii) = 9 - df_2(i) > 0$ , d.h. das Modell ist unterbestimmt in dem Sinne, dass $df_2(ii)$ Parameter a priori festgelegt werden müssen.

Mit dem Bereitstellen einer individuellen Refraktion, also Messdaten zur Wellenfront $\mathbf{S}_M$ des Auges insbesondere bis zur zweiten Ordnung liegen die nötigen Daten der Vergenzmatrix $\mathbf{S}_M$ entsprechend vor. Gemäß einem in WO 2013/104548 A1 beschriebenen herkömmlichen Verfahren werden insbesondere die Parameter {$\mathbf{C}, d_{CL}, \mathbf{S}_M$} gemessen. Hingegen werden herkömmlicherweise unter anderem die beiden Längenparameter $d_L$ und $d_{LR}$ (bzw. $D_{LR}$) a priori festgelegt (z.B. durch Literaturwerte oder Schätzung). In WO 2013/104548 A1 werden insbesondere die beiden Fälle unterschieden, in denen entweder $\mathbf{L}_2$ a priori festgelegt und $\mathbf{L}_1$ daraus berechnet wird, oder umgekehrt. Als Berechnungsvorschrift offenbart die genannte Offenlegungsschrift hierzu Gl.(4) bzw. Gl.(5). Für beide Fälle gilt $df_2 = 4 + 5 + 3$.

In der Bezeichnungsweise der oben genannten Schritte 1 bis 6 geschieht die Anpassung von $\mathbf{L}_1$ an die Messungen insbesondere dadurch, dass man zum einen die gemessene Vergenzmatrix $\mathbf{S}_M$ mittels der Schritte 1, 2 durch die ebenfalls gemessene Matrix C hindurch rechnet und bis zur objektseitigen Seite der Linsenvorderfläche propagiert. Zum anderen rechnet man von einer gedachten punktförmigen Lichtquelle auf der Netzhaut eine Kugelwelle mittels der rückwärts durchlaufenen Schritte 6, 5, 4 von hinten nach vorne, indem man diese Kugelwelle an der zuvor festgelegten Flächenbrechwertmatrix $\mathbf{L}_2$ der Linsenrückfläche bricht und die dann erhaltene Wellenfront von der Linsenrückfläche bis zur bildseitigen Seite der Linsenvorderfläche propagiert. Die Differenz der so bestimmten Vergenzmatrizen $\mathbf{S}_{L1}$ und $\mathbf{S'}_{L1}$, die objektseitig bzw. bildseitig der Linsenvorderfläche liegen muss durch die Matrix $\mathbf{L}_1$ bewirkt worden sein, denn bei der aberrometrischen Messung geht die gemessene Wellenfront aus einer Wellenfront hervor, die von einem Punkt auf der Netzhaut ausgeht, und deshalb aufgrund der Umkehrbarkeit der Strahlengänge identisch mit derjenigen einfallenden Wellenfront ist ($\mathbf{S} = \mathbf{S}_M$), die auf diesen Punkt der Netzhaut zusammenläuft. Dies führt zu Gleichung (4) in der genannten Offenlegungsschrift:

$$\mathbf{L}_1\left(D_{LR}\right) = \frac{D_{LR} \cdot \mathbf{1} - \mathbf{L}_2}{1 + \tau_L \cdot \left(D_{LR} \cdot \mathbf{1} - \mathbf{L}_2\right)} - \frac{\mathbf{S}_M + \mathbf{C}}{1 - \tau_{CL}\left(\mathbf{S}_M + \mathbf{C}\right)} \qquad (1a)$$

Der andere Fall in der genannten Offenlegungsschrift betrifft die Anpassung der Matrix $\mathbf{L}_2$ an die Messungen, nachdem die Matrix $\mathbf{L}_1$ festgelegt worden ist. Ein Unterschied besteht jetzt lediglich darin, dass die gemessene Wellenfront $\mathbf{S}_M$ den Schritten 1, 2, 3, 4 unterzogen wird und die angenommene Wellenfront aus der punktförmigen Lichtquelle nur dem Schritt 6, und dass der zur Anpassung der Linsenrückfläche $\mathbf{L}_2$ zu erfolgende fehlende Schritt jetzt Schritt 5 ist, entsprechend Gl.(5) der genannten Offenlegungsschrift:

$$\mathbf{L}_2 = \mathbf{D}_{LR} - \left(\frac{\mathbf{S}_M + \mathbf{C}}{1 - \tau_{CL}\left(\mathbf{S}_M + \mathbf{C}\right)} + \mathbf{L}_1\right)\left(\mathbf{1} - \tau_L\left(\frac{\mathbf{S}_M + \mathbf{C}}{1 - \tau_{CL}\left(\mathbf{S}_M + \mathbf{C}\right)} + \mathbf{L}_1\right)\right)^{-1} \qquad (1b)$$

Der zentrale Gedanke der Erfindung besteht darin, zumindest den Längenparameter $d_{LR}$ (bzw. $D_{LR}$) aus anderen gemessenen Daten und a-priori-Annahmen zu anderen Freiheitsgraden zu berechnen und nicht wie herkömmlich selbst a-priori anzunehmen. Im Rahmen der vorliegenden Erfindung stellte sich heraus, dass dies deshalb eine bemerkenswerte Verbesserung der individuellen Anpassung bei vergleichsweise geringem Aufwand brachte, weil sich die Wellenfrontdurchrechnung als sehr empfindlich von diesem Längenparameter abhängig herausstellte. Dies bedeutet, dass es erfindungsgemäß ein Vorteil ist, wenn zumindest der Längenparameter $d_{LR}$ zu den $df_2(iii)$ = 3 Parametern gehört, die berechnet wird. Dieser Parameter ist insbesondere einer direkten Messung schlecht zugänglich, er variiert zwischen unterschiedlichen Probanden stärker und diese Variationen beeinflusst die Abbildung des Auges vergleichsweise stark.

Vorzugsweise stehen die Daten der Vergenzmatrix $\mathbf{S}_M$ und besonders bevorzugt auch die Daten zu $\mathbf{C}$ aus individuellen Messungen zur Verfügung. In einem weiteren bevorzugten Aspekt, der vorzugsweise auch in den folgenden Ausführungsformen berücksichtigt wird, wird bei einer Annahme von Daten zur Linsenrückfläche von einer sphärischen Rückfläche, d.h. einer Rückfläche ohne astigmatische Komponenten ausgegangen.

In einer bevorzugten Ausführungsform der Erfindung liegen also zur Cornea $\mathbf{C}$ Messdaten bis zur zweiten Ordnung vor, die den Daten der Flächenbrechwertmatrix $\mathbf{C}$ entsprechen. Obgleich diese Werte topographischen Messungen entnommen werden können, sind letztere nicht notwendig. Vielmehr sind topometrische Messungen ausreichend. Diese Situation entspricht dem Fall $df_2 = 3 + 6 + 3$, wobei insbesondere die Vorderkammertiefe $d_{CL}$ einer der sechs a priori festzulegenden Parameter ist.

Soweit keine weiteren individuellen Messungen vorgenommen werden, liegt eine Situation mit $df_2 = 3 + 6 + 3$ vor, Um $d_{LR}$ eindeutig bestimmen zu können, müssen also sechs Parameter aus $\{\mathbf{L}_1, \mathbf{L}_2, d_L, d_{CL}\}$ durch Annahmen bzw. Literaturwerte belegt werden. Die übrigen beiden ergeben sich zusätzlich zu $d_{LR}$ aus der Rechnung. In einer bevorzugten Ausführungsform werden die Parameter der Linsenrückfläche, die mittlere Krümmung der Linsenvorderfläche und die beiden Längenparameter $d_L$ und $d_{CL}$ a priori (als vorgegebene Standardwerte) belegt.

In einem für die Erfindung besonders wichtigen Fall ist zusätzlich die Vorderkammertiefe $d_{CL}$ also der Abstand zwischen der Hornhaut und Linsenvorderfläche, beispielsweise aus pachymetrischen oder OCT-Messungen bekannt. Damit umfassen die gemessenen Parameter $\{\mathbf{C}, d_{CL}, \mathbf{S}_M\}$. Diese Situation entspricht dem Fall $df_2 = 4 + 5 + 3$. Das Problem ist demnach mathematisch noch unterbestimmt, es müssen also fünf Parameter aus $\{\mathbf{L}_1, \mathbf{L}_2, d_L\}$ durch Annahmen bzw. Literaturwerte a priori festgelegt werden. Hierbei handelt es sich in einer bevorzugten Ausführungsform um die Parameter der Linsenrückfläche, die mittlere Krümmung der Linsenvorderfläche und die Linsendicke. Der genaue Rechenweg für diesen Fall dargestellt weiter unten noch dargelegt.

Allein für die Genauigkeit der individuellen Anpassung ist es von Vorteil, möglichst viele Parameter mit individuellen Messungen belegen zu können. In einer bevorzugten Ausführungsform wird dazu zusätzlich die Linsenkrümmung in einem Normalschnitt auf Basis einer individuellen Messung bereitgestellt. Dadurch ergibt sich dann eine Situation gemäß $df_2 = 5 + 4 + 3$, und es reicht aus, vier Parameter aus $\{L_{1yy}, \alpha_{L1}, \mathbf{L}_2, d_L\}$ a priori festzulegen. Auch hier handelt es sich in einer bevorzugten Ausführungsform wieder um die Parameter der Linsenrückfläche und die Linsendicke. Die genaue Durchrechnung wieder weiter unten noch beschrieben.

Insbesondere alternativ zum Normalschnitt der Linsenvorderfläche und besonders bevorzugt zusätzlich zur Vorderkammertiefe kann auch die Linsendicke aus einer individuellen Messung zur Verfügung gestellt werden. Dadurch entfällt die Notwendigkeit, diesen Parameter mit Modelldaten oder Schätzparametern zu belegen ($df_2 = 5 + 4 + 3$). Ansonsten gilt das oben bereits ausgeführte. Diese Ausführungsform ist besonders vorteilhaft, wenn ein Pachymeter eingesetzt wird, dessen Messtiefe die Erkennung der Linsenrückfläche erlaubt, nicht aber eine hinreichend sichere Bestimmung der Linsenkrümmungen.

Zusätzlich zur Vorderkammertiefe und einem Normalschnitt der Linsenvorderfläche können in einer bevorzugten Ausführungsform ein (z.B. Messung in zwei Normalschnitten) oder zwei weitere Parameter (Messung beider Hauptschnitte und der Achslage) der Linsenvorderfläche durch eine individuelle Messung erfasst werden. Diese zusätzlichen Informationen können insbesondere auf zwei Arten ausgenutzt werden:

- Aufgabe von a priori Annahmen: Es können ein bzw. zwei der ansonsten a priori getroffenen Annahmen aufgegeben und aus durch Berechnung bestimmt werden. In diesem Fall ergeben sich die Situationen $df_2 = 6 + 3 + 3$ bzw. $df_2 = 7 + 2 + 3$. So kann im ersten Fall die mittlere Krümmung der Rückfläche (bei Annahme einer astigmatismusfreien Rückfläche) und im zweiten Fall bei gegebener mittlerer Krümmung der Flächenastigmatismus (incl. Achslage) bestimmt werden. Alternativ kann in beiden Fällen auch die Linsendicke aus den Messungen bestimmt werden.

  Ein derartiges Vorgehen bedarf jedoch im Allgemeinen einer gewissen Umsicht, da verrauschte Messdaten leicht zu einem "Weglaufen" der freigegebenen Parameter führen können. Dadurch kann das Modell insgesamt deutlich schlechter anstatt besser werden. Eine Möglichkeit dies zu verhindern besteht darin, anatomisch sinnvolle Grenzwerte für diese Parameter vorzugeben und die Variation der Parameter auf diesen Bereich zu beschränken. Selbstverständlich können diese Grenzen auch abhängig von den gemessenen Werten vorgegeben werden.

- Verringerung der Messunsicherheit: Werden dagegen weiterhin die gleichen a priori Annahmen getroffen (vorzugsweise also $\{\mathbf{L}_2, d_L\}$), liegen die Situationen $df_2 = 6 + 4 + 3$ bzw. $df_2 = 7 + 4 + 3$ vor, das System ist also mathematisch überbestimmt.

  Anstelle einer einfachen analytischen Bestimmung von $D_{LR}$ gemäß nachfolgender Ausführungen wird $D_{LR}$ (und gegebenenfalls der noch fehlende Parameter aus $\mathbf{L}_1$) so bestimmt ("fit"), dass der Abstand zwischen dem sich aus den Gleichungen ergebenden $\mathbf{L}_1$ und dem gemessenen $\mathbf{L}_1$ (bzw. dem um den fehlenden Parameter ergänzten gemessenen $\mathbf{L}_1$) minimal wird. Durch dieses Vorgehen kann - offensichtlich - eine Verringerung der Messunsicherheit erreicht werden.

In einer weiteren bevorzugten Implementierung werden die Vorderkammertiefe, zwei oder drei Parameter der Linsenvorderfläche und die Linsendicke individuell gemessen. Die Berechnung der übrigen Größen erfolgt dabei analog, wobei die a priori Annahme der Linsendicke durch die entsprechende Messung ersetzt werden kann.

In einer weiteren bevorzugten Implementierung werden individuelle Messungen der Vorderkammertiefe, mindestens eines Parameters der Linsenvorderfläche, der Linsendicke und mindestens eines Parameter der Linsenrückfläche bereitgestellt. Hierbei handelt es sich um eine Ergänzung der oben genannten Fälle. Die jeweiligen zusätzlich gemessenen Parameter können analog den schrittweisen Erweiterungen der obigen Abschnitte erfolgen. Diese Fälle sind besonders vorteilhaft, wenn die oben erwähnten Pachymetrieeinheiten, die in einer Ebene, zwei Ebenen oder über die ganze Fläche messen in der Messtiefe entsprechend erweitert und so präzise sind, dass sich die Krümmungsdaten hinreichend genau ermitteln lassen.

Nachfolgend wird anhand einiger Beispiele gezeigt, wie die Berechnung einzelner Parameter aus den übrigen gemessenen oder a priori festgelegten Parametern und anhand der individuellen Refraktionsdaten erfolgen kann.

Beispielsweise ist in bevorzugten Ausführungsformen eine Messung der Krümmung einer Linsenfläche in einem Normalschnitt verfügbar. Da die Rückfläche in der Praxis nicht gemessen werden kann, ohne dass auch die Vorderfläche gemessen wird, und die Messung der Vorderfläche bevorzugt stattfindet, werden nachfolgend die Gleichungen für die Fälle einer in einem Normalschnitt bekannten Krümmung der Linsenvorderfläche angegeben. Ist statt eines Normalschnittes der Linsenvorderfläche ein Normalschnitt der Linsenrückfläche gegeben (z.B. entsprechende Messungen, Modellannahmen), muss in analoger Weise mit GI.(1b) verfahren werden. Ohne Beschränkung der Allgemeinheit legt man das Koordinatensystem so, dass der Normalschnitt in x -Richtung verläuft. In einem nächsten Schritt wertet man dann die Matrixgleichung (1a) in dem gegebenen Normalschnitt aus und löst nach $D_{LR}$ auf, und setzt diese Lösung anschließend wieder in GI.(1a) ein zur vollständigen Angabe von $\mathbf{L}_1$.

Setzt man die xx -Komponente von $\mathbf{L}_1(D_{LR})$ aus GI. (1) dem gemessenen Wert $L_{1,xx}$ gleich, erhält man für dieses Matrixelement eine in $D_{LR}$ quadratische Gleichung, deren positive Lösung dem Abstand zwischen Linsenrückfläche und Netzhaut entspricht:

$$D_{LR} = \frac{-b + \sqrt{b^2 - 4c}}{2a} \qquad (2)$$

Dabei gelten:

$$a = \tau_L(1 + \tau_L A)$$

$$b = 1 - \tau_L(\mathrm{tr}(\mathbf{L}_2) - AB)$$

$$\begin{aligned} c &= A - L_{2,xx} + \tau_L \det \mathbf{L}_2 \, (1 + \tau_L A) - \tau_L A \, \mathrm{tr}(\mathbf{L}_2) \\ &= A - L_{2,xx} + a \det \mathbf{L}_2 - \tau_L A \, \mathrm{tr}(\mathbf{L}_2) \end{aligned} \qquad (2a)$$

mit

$$A = -S_{M.L1.xx} - L_{1.xx}$$

$$B = 2 - \tau_L \mathrm{tr}(\mathbf{L}_2)$$

$$\det(\mathbf{L}_2) = L_{2.xx} L_{2.yy} - L_{2.xy}^2$$

$$\mathrm{tr}(\mathbf{L}_2) = L_{2.xx} + L_{2.yy} \qquad (2b)$$

sowie

$$S_{M,L1,xx} = \frac{\tau_{CL}S'^2_{M.C.xy} + S'_{M.C.xx}\cdot\left(1 - \tau_{CL}S'_{M.C.yy}\right)}{-\tau^2_{CL}S'^2_{M.C.xy} + \left(1 - \tau_{CL}S'_{M.C.xx}\right)\cdot\left(1 - \tau_{CL}S'_{M.C.yy}\right)}$$

$$S'_{M.C.xx} = S_{M.xx} + C_{xx} \quad \left(xy \text{ und } yy \text{ analog}\right)$$

(2c)

Für den Fall einer symmetrischen Linsenrückfläche ($L_2 = L_{2,xx}\cdot\mathbf{1}$) vereinfacht sich dies zu:

$$D_{LR} = L_{2,xx} + \frac{L_{1,xx} + S_{M,L1,xx}}{1 - \tau_L\cdot\left(L_{1,xx} + S_{M,L1,xx}\right)}$$

(3)

mit $S_{M,L1,xx}$ aus Gleichung (2c).

In beiden Fällen ist es damit möglich, die Linsenvorderfläche $L_1$ zu berechnen, indem man das jeweilige gewonnene $D_{LR}$ in Gleichung (1a) einsetzt:

$$\mathbf{L}_1 = \frac{\mathbf{D}_{LR} - \mathbf{L}_2}{1 + \tau_L\cdot\left(\mathbf{D}_{LR} - \mathbf{L}_2\right)} - \frac{\mathbf{S}_M + \mathbf{C}}{1 - \tau_{CL}\left(\mathbf{S}_M + \mathbf{C}\right)}$$

(4)

Das Ergebnis ist naturgemäß symmetrisch ($L_{1,xy} = L_{1,yx}$) und reproduziert für die Komponente $L_{1,xx}$ den in (2b) bzw. (3) eingesetzten Wert.

In einigen bevorzugten Ausführungsformen steht eine individuelle Messung oder eine Vorgabe einer mittleren Krümmung einer Linsenfläche zur Verfügung. Diese Situation liegt beispielsweise dann vor, wenn die mittlere Krümmung der Linsenvorderfläche gemessen werden kann oder keine Messungen an den Linsenflächen durchgeführt werden können und die mittlere Krümmung einer Linsenfläche angenommen wird (z.B. aus der Literatur entnommen). Wie soeben wird auch hier das Verfahren für die Linsenvorderfläche beschrieben und ist analog auf die Linsenrückfläche übertragbar.

Im diesem Fall einer gegebenen mittleren Sphäre $L_{1,ms}$ der Linsenvorderfläche sind die freien Parameter der Zylinder $L_{1,cyl}$ und die Achslage $\alpha_{L1}$. Mit $L_{1,diff} = L_{1,cyl}/\mathbf{2}$ wird $\mathbf{L}_1$ zu

$$\mathbf{L}_1 = \begin{pmatrix} L_{1,ms} - L_{1,diff}\cdot\cos 2\alpha_{L1} & -L_{1,diff}\cdot\sin 2\alpha_{L1} \\ -L_{1,diff}\cdot\sin 2\alpha_{L1} & L_{1,ms} + L_{1,diff}\cdot\cos 2\alpha_{L1} \end{pmatrix}$$

(5)

Auch geht man wieder von Gl.(1a) aus. Setzt man nun die Ausdrücke für $\mathbf{L}_1$ aus den Gleichungen (5) und (1a) gleich, erhält man ein Gleichungssystem aus drei Gleichungen (die beiden Nichtdiagonalelemente sind identisch) und den drei Unbekannten $L_{1,\text{diff}}$, $\alpha_{L1}$ und $D_{LR}$. Dieses hat die physikalisch relevante Lösung

$$D_{LR} = \frac{-\bar{b} + \sqrt{\bar{b}^2 - 4\bar{a}\bar{c}}}{2\bar{a}}$$

$$L_{1diff} = \pm\sqrt{\sigma^2 + \gamma^2}$$

$$\alpha_{L1} = \frac{1}{2}\arctan\left(\pm\gamma, \pm\sigma\right) + \frac{\pi}{2}$$

(6)

mit

$$\bar{a} = \tau_L(1 + \tau_L\bar{A})$$

$$\bar{b} = 1 - \tau_L \left( \operatorname{tr}(\mathbf{L}_2) - \overline{A}B \right)$$

$$\bar{c} = \frac{1}{4} \left( \overline{A}B^2 - B\,\operatorname{tr}(\mathbf{L}_2) - \bar{a}\,\operatorname{Ast}(\mathbf{L}_2)^2 \right)$$

und

$$\overline{A} = \ \overline{S}_{M,L1} - \overline{L}_{1,mess}$$

$$\operatorname{Ast}(\mathbf{L}_2) = \sqrt{\operatorname{tr}(\mathbf{L}_2)^2 - 4\det \mathbf{L}_2}$$

$$\gamma = \frac{2\left(-1 + \sqrt{\bar{b}^2 - 4\overline{a}\overline{c}}\right)\left(L_{2,xx} - L_{2,yy}\right) + \tau_L^2 \operatorname{Ast}(\mathbf{L}_2)^2 \left(S_{M,L1,xx} - S_{M,L1,yy}\right)}{2\tau_L^2 \operatorname{Ast}(\mathbf{L}_2)^2}$$

$$\sigma = \frac{2\left(-1 + \sqrt{\bar{b}^2 - 4\overline{a}\overline{c}}\right)L_{2,xy} + \tau_L^2 \operatorname{Ast}(\mathbf{L}_2)^2 S_{M,L1,xy}}{2\tau_L^2 \operatorname{Ast}(\mathbf{L}_2)^2}$$

$$(6a)$$

Auch dies lässt sich für den Fall einer rotationssymmetrischen Linsenrückfläche vereinfachen:

$$D_{LR} = L_2 + \frac{\overline{L}_{1,mess} + \overline{S}_{M,L1}}{1 - \tau_L \cdot \left(\overline{L}_{1,mess} + \overline{S}_{M,L1}\right)}$$

$$\mathbf{L}_1 = \left(\overline{L}_{1,mess} + \overline{S}_{M,L1}\right)\cdot 1 - \frac{\mathbf{S}_M + \mathbf{C}}{1 - \tau_{CL}\left(\mathbf{S}_M + \mathbf{C}\right)}$$

$$(7)$$

wobei

$$\overline{L}_{1,mess} = \frac{D_{LR} - L_2}{1 + \tau_L \cdot \left(D_{LR} - L_2\right)} - \overline{S}_{M,L1}$$

mit

$$\overline{S}_{M,L1} = \frac{S_{M,L1,xx} + S_{M,L1,yy}}{2}$$

Damit sind die einzelnen Elemente des Augenmodells vollständig berechenbar.

Bei den gegebenen (d.h. gemessenen bzw. angenommenen) Größen kann es sich außer um einen Hauptschnitt mit gegebener Winkelstellung bzw. der mittleren Krümmung auch um andere Parameter wie den stärksten Hauptschnitt, den schwächsten Hauptschnitt, den Zylinder und die Achslage handeln. Das Vorgehen ist in diesen Fällen analog zu den geschilderten Fällen.

Da inzwischen auch die HOA des Auges bei der Optimierung von Brillengläsern berücksichtigt werden, ist es vorteilhaft, bei der Belegung des Augenmodells auch die HOA der Hornhaut bzw. der Linse zu betrachten. Bei der Wahl von HOA für die Linse gilt generell, dass der Linsenvorder- bzw. Rückfläche HOA zugeordnet werden können, die auch den Brechungsindexverlauf innerhalb der Linse abbilden können.

Vorzugsweise wird der bisher dargestellte Formalismus insbesondere hinsichtlich der genannten Schritte 1 bis 6 auf die Mitbehandlung der HOA erweitert, indem außer den in Schritt 1 bis 6 explizit angegebenen Formeln für die Vergenzmatrizen die Berechnungsverfahren aus den Publikationen von G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" angewendet werden.

Generell ist das Vorgehen in Bezug auf das Abzählen von Freiheitsgraden sehr ähnlich wie oben ausgeführt. Liegen zur brechenden Fläche C der Hornhaut und zur austretenden Wellenfront $S_M$ außer Daten zu Fehlern 2. Ordnung auch Daten über deren HOA vor (entweder aus Messungen oder aus sinnvollen Annahmen), dann kann auch die Wellenfront $S_{L1}$ mit entsprechend vielen HOA rechnerisch bestimmt werden. Dies gilt unabhängig von der Darstellungsform der HOA. Besonders bevorzugt ist allerdings die Taylorreihe, denn in dieser Form gilt exakt die Aussage: Sind zu beiden Flächen *C* und $S_M$ HOA-Koeffizienten bis zur Ordnung n gegeben, dann lassen sich auch die entsprechenden HOA-Koeffizienten für $S_{L1}$ bis zur Ordnung *n* daraus rechnerisch bestimmen. Weiterhin bevorzugt ist die Zernike-Basis, denn auch hier gilt eine ähnliche Aussage. Exakt ist diese allerdings nur dann, wenn alle Zernike-Koeffizienten mit einer Ordnung > *n* verschwinden.

Vorzugsweise wird (vorab) eine Ordnung n festgelegt, bis zu der alle beteiligten Flächen und Wellenfronten behandelt werden sollen. Unabhängig von der Darstellung der HOA besitzen die Wellenfronten oder Flächen dann außer den drei Komponenten für die Fehler 2.Ordnung noch *N* Komponenten für die HOA, wobei *N* von *n* und i.a. von der Darstellungsform der HOA abhängt (In der Taylor- und Zernike-Zerlegung gilt N = (n + 1)(n + 2)/2 - 6 )

Entsprechend besitzt dann auch die Anpassungsbedingung anhand einer gemessenen Wellenfront, z.B. $S_{M,L1}$, nicht mehr nur die oben beschriebenen drei Komponenten, sondern insgesamt maximal *N* + 3 Komponenten. Diesen stehen dann entsprechend 3 (*N* + 3) + 3 = 3N + 12 Parameter gegenüber (nämlich die drei Längenparameter $d_{CL}$, $d_L$ und $d_{LR}$ (bzw. $D_{LR}$) sowie je N + 3 Komponenten von der Cornea *C* und den Linsenflächen $L_1$ und $L_2$). Das bedeutet, es gilt:

$$df_n = df_n(i) + df_n(ii) + df_n(iii)$$
$$= 3N + 12$$

mit $df_n(iii)$ = N + 3. Werden wieder bevorzugt die Vorderkammertiefe $d_{CL}$ und die Cornea C gemessen, gilt $df_n(i)$ = N + 4 und folglich $df_n(ii)$ = N + 5, entsprechend der Situation $df_n$ = (N + 4) + (N + 5) + (N + 3).

Das weitere Vorgehen kann ganz analog zum oben beschriebenen durchgeführt werden.

Bei der dem hier beschriebenen Vorgehen zu Grunde liegenden Messvorrichtung können mit der Aberrometrieeinheit die HOA der Abbildung des Auges auf die Netzhaut in Transmission erfasst werden. Ferner können mit der gleichen Vorrichtung durch die Topographieeinheit die HOA der Hornhaut-Oberfläche in Reflexion gemessen werden. Damit stehen sowohl die austretende Wellenfront $S_M$ als auch die brechende Fläche *C* der Hornhaut, inklusive der HOA bis zu einer bestimmten Ordnung n zur Verfügung. Die Wellenfront $S_M$ liefert $df_n(iii)$ = N + 3 Bedingungen zur Parameterberechnung. Wird wieder bevorzugt, außer der Cornea *C* auch die Vorderkammertiefe $d_{CL}$, gemessen, gilt $df_n(i)$ = N + 4 und folglich ist $df_n(ii)$ = N + 5, entsprechend der Situation $df_n$ = (N + 4) + (N + 5) + (N + 3)

In einer bevorzugten Ausführungsform der Erfindung können nun bei der Belegung des Modells die HOA der Linse so gewählt werden, dass bei der Propagation einer von einem Punkt der Netzhaut ausgehenden Wellenfront gemäß den Schritten 1 bis 6 in umgekehrter Reihenfolge die gemessene Wellenfront entsteht.

Erfindungsgemäß wird allerdings vorgeschlagen, dass zumindest der Längenparameter $d_{LR}$ weder a-priori vorgegeben noch individuell gemessen, sondern anhand der individuellen Refraktionsdaten und der sonstigen (vorab) festgelegten Daten berechnet wird. Dazu wird insbesondere für einen der Freiheitsgrade Linsenflächen $L_1$ oder $L_2$ zumindest ein Messwert oder eine Annahme bereitgestellt. Ist dies z.B. ein Messwert für die Krümmung von $L_1$ in einem Normalschnitt, dann kann $d_{LR}$ (bzw. $D_{LR}$) daraus durch Berechnung bestimmt werden.

Bezieht sich die Angabe in den Vergenzmatrizen auf die lokale Krümmung (dies entspricht der Angabe der HOA als Koeffizienten einer Zerlegung nach Taylor), werden dazu zuerst wie in oben bereits beschrieben $D_{LR}$ und die fehlenden Parameter der Linse bestimmt. Im Anschluss daran können dann mit dem Formalismus aus G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" schrittweise die HOA der Linse ausgehend von der zweiten bis zur n-ten Ordnung aufgebaut werden.

Werden dagegen die mittlere Krümmung über eine bestimmte Pupille verwendet, was beispielsweise bei der Darstellung nach Zernike der Fall ist, ist der Freiheitsgrad $D_{LR}$ ebenso festgelegt. In diesem Formalismus wäre aufgrund der Abhängigkeiten ein iteratives Vorgehen notwendig. Dies lässt sich jedoch durch eine Umrechnung zwischen den beiden Notationen vor Beginn der Berechnung vermeiden.

Selbst wenn weder ein Topograph noch ein Aberrometer verwendet werden, also keine individuellen Messdaten zu HOAs vorliegen, können dennoch modellbasierte Annahmen über die HOA der Hornhaut, der Linse bzw. das Auge getroffen und bei der Belegung des Augenmodells verwendet werden. Die angenommen Werte können dabei auch an Hand entsprechender Modelle in Abhängigkeit von gemessenen Daten (z.B. Refraktionswerte, Ergebnisse der Topometrie- oder Autorefraktometermessung) gewählt werden. Beispiele für die genaue Berechnung wurden bereits weiter oben beschrieben, wobei an Stelle der gemessenen Werte für die HOAs die entsprechenden Annahmen treten. Dies gilt auch wieder insbesondere für sphärische Aberrationen, da diese im Mittel über die Population deutlich von Null verschieden ist. Dabei kann diese unabhängig von den gemessenen Daten oder in Abhängigkeit von gemessenen Daten (z.B. Refraktionswerte, Ergebnisse der Topometrie- oder Autorefraktometermessung) gewählt und der Hornhaut, einer der beiden Linsenflächen oder Kombinationen zugeordnet werden.

Auf Grund der großen Bedeutung der subjektiven Refraktion ist es vorteilhaft, auch die Ergebnisse einer derartigen subjektiven Augenglasbestimmung in die Belegung des Modells für die Optimierung zumindest teilweise eingehen zu lassen. Vorzugsweise werden subjektive Refraktionsdaten in der Form Sphäre, Zylinder und Achslage bereitgestellt. Der Einfachheit halber orientiert sich die Beschreibung des Vorgehens an dieser Notation mit *sph, cyl* und *a* für die Werte von Sphäre, Zylinder und Achslage.

Werden HOA nicht berücksichtigt, kann wie folgt vorgegangen werden:

Sollen nur die Werte der subjektiven Refraktion in die Optimierung einfließen, kann auf die Messung der Wellenfront $S_M$ durch ein Aberrometer bzw. einen Autorefraktometer verzichtet werden und statt dessen die Matrix $\mathbf{S}_M$ aus den subjektiven Werten aufgebaut werden:

$$\mathbf{S}_M = \begin{pmatrix} \left(sph + \frac{1}{2} \cdot cyl\right) - \frac{1}{2} \cdot cyl \cdot \cos(2a) & -\frac{1}{2} \cdot cyl \cdot \sin(2a) \\ -\frac{1}{2} \cdot cyl \cdot \sin(2a) & \left(sph + \frac{1}{2} \cdot cyl\right) + \frac{1}{2} \cdot cyl \cdot \cos(2a) \end{pmatrix}$$

Vorzugsweise werden aber die Ergebnisse der subjektiven Refraktion mit denen der aberrometrischen bzw. autorefraktometrischen Messung kombiniert. Dazu wird auf Basis beider Datensätze eine optimierte Refraktion beispielsweise nach einem in DE 10 2007 032 564 A1 beschriebenem Verfahren bestimmt. Diese wird durch die Werte $sph_{opt}$, $cyl_{opt}$ und $a_{opt}$ beschrieben. Analog zu dem vorigen Abschnitt erhält man $\mathbf{S}_M$ als

$$\mathbf{S}_M = \begin{pmatrix} \left(sph_{opt} + \frac{1}{2} \cdot cyl_{opt}\right) - \frac{1}{2} \cdot cyl_{opt} \cdot \cos(2a_{opt}) & -\frac{1}{2} \cdot cyl_{opt} \cdot \sin(2a_{opt}) \\ -\frac{1}{2} \cdot cyl_{opt} \cdot \sin(2a_{opt}) & \left(sph_{opt} + \frac{1}{2} \cdot cyl_{opt}\right) + \frac{1}{2} \cdot cyl_{opt} \cdot \cos(2a_{opt}) \end{pmatrix}$$

Gemäß DE 10 2007 032 564 A1 müssen nicht alle Werte der subjektiven Refraktion bzw. objektiven Messung in die optimierten Refraktionswerte eingehen. So kann beispielsweise im Fall einer Bestimmung der optimierten Refraktionswerten für die Nähe oder im Fall erwarteter Instrumentenmyopie auf die Verwendung der objektiv gemessenen Sphäre bzw. des objektiv gemessenen Defokusterms verzichtet werden.

Auch bei der Einbeziehung subjektiver Refraktionsdaten können bei der Belegung des Modells wiederum auch die HOA berücksichtigt werden. Dazu ist es bei Verwendung der subjektiven Refraktionswerte erforderlich, diese in konsistenter Weise in den Datensatz einfließen zu lassen. Zur Vereinfachung der Darstellung wird im Folgenden ein Formalismus auf Basis von Zernike Koeffizienten gewählt, wobei grundsätzlich auch eine andere Basis verwendet werden kann.

Im Folgenden wird zunächst der Zusammenhang zwischen einem Satz Zernike Koeffizienten zur Darstellung von Wellenfronten ($c_{nm}$) mit $r_0$ als Radius der Wellenfront und Refraktionswerten (*sph, cyl, a*) betrachtet. Der Radius $r_0$ wird vorzugsweise entweder gemessen oder auf Basis von Modellannahmen festgelegt. Bei Verwendung der RMS-Metrik ergibt beispielsweise der bijektive Zusammenhang

$$\begin{pmatrix} c_{2,-2} \\ c_{2,0} \\ c_{2,+2} \end{pmatrix} = g_{RMS}(sph, cyl, a) = \frac{r_0^2}{2\sqrt{6}} \cdot \begin{pmatrix} \frac{1}{2} \cdot cyl \cdot \sin(2a) \\ -\frac{1}{\sqrt{2}} \cdot \left(sph + \frac{1}{2}cyl\right) \\ \frac{1}{2} \cdot cyl \cdot \cos(2a) \end{pmatrix} \quad \Leftrightarrow$$

$$\begin{pmatrix} sph \\ cyl \\ a \end{pmatrix} = f_{RMS}(c_{2,-2}, c_{2,0}, c_{2,+2}) = \begin{pmatrix} -\frac{4\sqrt{3}}{r_0^2} \cdot \left(c_{2,0} - \frac{1}{\sqrt{2}} \cdot \sqrt{c_{2,-2}^2 + c_{2,+2}^2}\right) \\ -\frac{4\sqrt{6}}{r_0^2} \cdot \sqrt{c_{2,-2}^2 + c_{2,+2}^2} \\ \frac{1}{2} \cdot \arctan(c_{2,+2}, c_{2,-2}) + \frac{\pi}{2} \end{pmatrix}$$

Dies ist jedoch nur als Beispiel für eine Metrik der generellen Form

$$\begin{pmatrix} sph \\ cyl \\ a \end{pmatrix} = f_0(c_{2,-2}, c_{2,0}, c_{2,+2}) \quad \Leftrightarrow \quad \begin{pmatrix} c_{2,-2} \\ c_{2,0} \\ c_{2,+2} \end{pmatrix} = g_0(sph, cyl, a) \tag{8}$$

zu verstehen.

Darüber hinaus gibt es Zusammenhänge, bei denen auch HOA in die Refraktionswerte eingehen. Diese Abbildung ist dann für die Berechnung der Refraktionswerte immer noch surjektiv, aber nicht mehr bijektiv, d.h. der komplette Satz aller Zernike Koeffizienten aller Abbildungsfehler lässt sich nicht eindeutig aus den Refraktionswerten reproduzieren. Die Koeffizienten der Abbildungsfehler niedriger Ordnung lassen sich jedoch auch hier wieder eindeutig bestimmen, wenn die Koeffizienten für die HOA vorgegeben werden:

$$\begin{pmatrix} sph \\ cyl \\ a \end{pmatrix} = f_1(c_{2,-2}, c_{2,0}, c_{2,+2}, c_{i,j}) \quad \Leftrightarrow \quad \begin{pmatrix} c_{2,-2} \\ c_{2,0} \\ c_{2,+2} \end{pmatrix} = g_1(sph, cyl, a, c_{i,j}) \quad (i > 2) \tag{9}$$

Analoge Berechnungen und Ableitung sind naturgemäß auch in anderen Notationen wie beispielsweise mit den in den Publikationen von G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" verwendeten lokalen Ableitungen der Wellenfronten möglich. Liegen autorefraktometrische Messung mit Daten zu HOA vor, können diese Daten oder Teile dieser Daten verwendet werden, um zusammen mit den subjektiven Refraktionswerten beispielsweise gemäß DE 10 2007 032 564 A1 einen Satz optimierter Refraktionsdaten zu bestimmen. Dabei ist die gleichzeitige Verwendung sowohl subjektiver Refraktionsdaten als auch der Messdaten nicht erforderlich. Die in diesem Abschnitt im weiteren als optimierte Refraktionswerte ($sph_{opt}$, $cyl_{opt}$ und $a_{opt}$) bezeichneten Größen können also auch direkt aus der subjektiven Refraktionsbestimmung ohne Verwendung objektiver Messgrößen übernommen werden.

Grundsätzlich müssen nicht alle Werte der subjektiven Refraktion bzw. objektiven Messung in die optimierten Refraktionswerte eingehen. So kann beispielsweise im Fall einer Bestimmung der optimierten Refraktionswerten für die Nähe oder im Fall erwarteter Instrumentenmyopie auf die Verwendung der objektiv gemessenen Sphäre bzw. des objektiv gemessenen Defokusterms verzichtet werden.

Auf Basis der optimierten Refraktionswerte wird dann eine Wellenfront (vorzugsweise dargestellt durch die Zernike-Koeffizienten $o_{i,j}$) bestimmt, die diesen optimierten Werten entspricht. Diese Wellenfront wird dann an Stelle der oben beschriebenen, gemessenen austretenden Wellenfront verwendet. Bei Verwendung einer Metrik nach Gleichung (8) können die Koeffizienten zweiter Ordnung dieser Wellenfront gemäß Gleichung (8) aus den optimierten Refraktionswerten berechnet und die Koeffizienten höherer Ordnung aus der objektiven Messung der austretenden Wellenfront dargestellt durch die Koeffizienten $m_{i,j}$, direkt übernommen werden:

$$\begin{pmatrix} o_{2,-2} \\ o_{2,0} \\ o_{2,+2} \end{pmatrix} = g_0\big(sph_{opt}, cyl_{opt}, a_{opt}\big) \quad o_{i,j} = m_{i,j} \quad (i > 2)$$

Bei Verwendung einer Metrik nach Gleichung (9) sind die Koeffizienten zweiter Ordnung der Wellenfront ($o_{i,j}$) dagegen nicht nur von der optimierten Refraktion abhängig, sondern dagegen so zu wählen, dass gilt

$$\begin{pmatrix} sph_{opt} \\ cyl_{opt} \\ a_{opt} \end{pmatrix} = f_1\big(o_{2,-2}, o_{2,0}, o_{2,+2}, c_{i,j}\big) \qquad (i > 2)$$

und hängen deswegen zusätzlich direkt von den Koeffizienten höherer Ordnung der gemessenen austretenden Wellenfront ($m_{i,j}$) ab:

$$\begin{pmatrix} o_{2,-2} \\ o_{2,0} \\ o_{2,+2} \end{pmatrix} = g_1\big(sph_{opt}, cyl_{opt}, a_{opt}, m_{i,j}\big) \quad o_{i,j} = m_{i,j}$$

Die Auswertung der Aberrationen während des Berechnungs- bzw. Optimierungsverfahrens kann an unterschiedlichen Stellen im Strahlverlauf vorgenommen werden, d.h. die Bewertungsfläche kann an unterschiedlichen Positionen vorgesehen werden. Anstatt an der Netzhaut oder an der Linsenrückfläche kann eine Bewertung der abbildenden Wellenfront auch schon an einer weiter vorne im Modellauge liegenden Fläche erfolgen. Dazu wird innerhalb des Modellauges eine Referenzwellenfront **R** definiert, die dann beispielsweise bei der Glasoptimierung verwendet wird. Diese Referenzwellenfront hat dabei die Eigenschaft, dass sie bei weiterer Propagation durch das Auge bis zur Retina zu einem punktförmigen Bild führt. Entsprechend kann die Referenzwellenfront durch Rückpropagation einer Wellenfront, die auf der Netzhaut in einen Punkt zusammenläuft, von der Netzhaut bis zur Position der Referenzwellenfront bestimmt werden. Da die gemessene Wellenfront $S_M$ genau die Wellenfront ist, die aus einer punktförmigen Lichtquelle auf der Netzhaut hervorgeht, kann stattdessen auch diese ins Augeninnere bis zur Position der Referenzwellenfront propagiert werden.

Mathematisch betrachtet sind beide Vorgehensweisen äquivalent und führen auf die gleichen Formeln für die Referenzwellenfront. Im Folgenden wird zur Ableitung der entsprechenden Referenzwellenfronten jeweils der Weg gewählt, der mit weniger Propagationsschritten auskommt und eine einfachere Darstellung ermöglicht. Nachfolgend wird beispielhaft nur die Behandlung der Komponenten des Defokus und des Astigmatismus beschrieben. Eine Ausdehnung auf HOA und die Verwendung der subjektiven Refraktion ist jedoch ebenfalls möglich und vorteilhaft.

Bei der Berücksichtigung von HOA können diese analog zur Berechnung der HOA gemäß untenstehender Ausführungen durch Brechung G. Esser et al.: "Derivation of the refraction equations for higher order aberrations of local wavefronts at oblique incidence", JOSA A, Vol. 27, No. 2 (2010) und Propagation (G. Esser et al.: "Derivation of the propagation equations for higher order aberrations of local wavefronts, JOSA A, Vol. 28, No. 11 (2011)") erfolgen.

Da die Wellenfrontpropagation ein nichtlinearer Vorgang ist, führt eine Brillenglasoptimierung, die eine abbildende Wellenfront durch Vergleich mit einer Referenzwellenfront bewertet, im allgemeinen zu verschiedenen Ergebnissen, je nachdem, an welcher Fläche innerhalb des Auges dieser Vergleich stattfindet.

In einer bevorzugten Ausführungsform wird lediglich auf den allerletzten Schritt (insbesondere Schritt 6b), also die Propagation von der AP zur Netzhaut verzichtet. Die einfallende Wellenfront wird also nach der Brechung an der Linsenrückfläche nur bis zur AP simuliert (also Berechnung von $S_{AP}$ gemäß eingangs genanntem Schritt 6a) und dort mit einer Referenzwellenfront $R_{AP}$ verglichen. Diese zeichnet sich dabei dadurch aus, dass sie bei der Propagation zur Netzhaut dort ein punktförmiges Bild ergibt. Gemäß dem oben Gesagten ist die Vergenzmatrix dieser Wellenfront gerade

$$\mathbf{R}_{AP} = \mathbf{D}_{AP} = \mathbf{D}_{LR}^{(b)} = \frac{1}{\tau_{LR}^{(b)}}\mathbf{1} = \frac{1}{\tau_{LR} - \tau_{LR}^{(a)}}\mathbf{1} = \frac{1}{1/D_{LR} - d_{LR}^{(a)}/n_{LR}}\mathbf{1}$$

mit dem aus Gleichung (2) bzw. (3) bestimmten $D_{LR}$ sowie dem negativen (akkommodationsabhängigen) Wert $d_{LR}^{(a)} < 0$, dessen Betrag den Abstand zwischen der Linsenrückfläche und der AP beschriebt.

In einer weiterhin bevorzugten Ausführungsform wird außerdem auf den vorletzten Schritt, insgesamt also die Propagation von der Linsenrückfläche zur Netzhaut verzichtet. Die einfallende Wellenfront wird also nur bis nach der Brechung an der Linsenrückfläche simuliert (also Berechnung von $S'_{L2}$ gemäß eingangs genanntem Schritt 5) und dort mit einer Referenzwellenfront $R'_{L2}$ verglichen. Diese zeichnet sich dabei dadurch aus, dass sie bei der Propagation zur Netzhaut dort ein punktförmiges Bild ergibt. Gemäß dem oben Gesagten ist die Vergenzmatrix dieser Wellenfront gerade

$$\mathbf{R}'_{L2} = \mathbf{D}'_{L2} = D_{LR} \cdot \mathbf{1}$$

mit dem aus Gleichung (2) bzw. (3) bestimmten $D_{LR}$.

Eine weitere Vereinfachung ergibt sich, wenn man den Vergleich vor die Brechung durch die Linsenrückfläche legt. In diesem Fall muss die einfallende Wellenfront nur bis $S_{L2}$ gemäß obigem Schritt 4 simuliert, also berechnet werden. Dazu wird analog zu $S'_{L2}$ eine Referenzwellenfront $R_{L2}$ definiert, die nach der Brechung an der Linsenrückfläche und der Propagation zur Netzhaut dort ein punktförmiges Bild ergibt. Diese bestimmt sich zu

$$\mathbf{R}_{L2} = \mathbf{R}'_{L2} - \mathbf{L}_2 = D_{LR} \cdot \mathbf{1} - \mathbf{L}_2$$

mit dem aus Gleichung (2) bzw. (3) bestimmten $D_{LR}$ und dem aus der Literatur bzw. aus Messungen bekanntem $L_2$.
Im Fall einer rotationssymmetrischen Linsenrückfläche vereinfacht sich dies zu

$$\mathbf{R}_{L2} = \left(D_{LR} - L_{2,xx}\right) \cdot \mathbf{1}$$

Insbesondere soweit die Linsendicke ebenfalls der Literatur entnommen wird, bietet es sich in einer weiteren bevorzugten Ausführungsform an, als nächsten Vereinfachungsschritt auf die Propagation durch die Linse zu verzichten und den Vergleich hinter der Brechung durch die Linsenvorderfläche auszuführen. In Weiterführung des oben Gesagten verwendet man dazu vorzugsweise eine Referenzwellenfront $R'_{L1}$, die aus $R_{L2}$ durch Rückwärtspropagation um die Linsendicke entsteht und folgende Vergenzmatrix besitzt:

$$\mathbf{R}'_{L1} = \mathbf{R}_{L2}/(\mathbf{1} + \tau_L \mathbf{R}_{L2})$$

mit dem aus Gleichung (2) bzw. (3) bestimmten $D_{LR}$ und dem aus der Literatur bzw. aus Messungen bekannten $\tau_L = d_L/n_L$ sowie der aus Gl.(6) bzw. (7) bestimmten Vergenzmatrix $\mathbf{R}_{L2}$.
Im Fall einer rotationssymmetrischen Linsenrückfläche vereinfacht sich dies zu

$$\mathbf{R}'_{L1} = \frac{D_{LR} - L_{2,xx}}{1 + \tau_L \cdot \left(D_{LR} - L_{2,xx}\right)} \cdot \mathbf{1}$$

Wie bei obigen Modellen gilt auch hier, dass selbst wenn die Betrachtung vor den letzten Schritten stattfindet und - je nach Notation - die Größe $D_{LR}$ nicht explizit vorkommt, diese Größe dennoch zusammen mit $d_L$ und $L_2$ zumindest implizit eingeht, da sie zusammen die Verteilung der Wirkung $L_1$ in der Linsenvorderfläche steuern.
Eine noch weitere Vereinfachung ergibt sich, wenn man den Vergleich vor die Brechung durch die Linsenvorderfläche legt. In diesem Fall muss die einfallende Wellenfront nur bis $S_{L1}$ gemäß Schritt 2 simuliert werden. Dazu wird analog zu $R'_{L1}$ eine Referenzwellenfront $R_{L1}$ definiert, die nach der Brechung an der Linsenvorderfläche und den weiteren Schritten an der Netzhaut zu einem Punkt konvergiert. Diese kann man entweder durch die Brechung von $R'_{L1}$ an $L_1$ berechnen oder direkt aus der Brechung der gemessenen Wellenfront $S_M$ an der Hornhaut $C$ und einer anschließenden Propagation um $d_{CL}$ bestimmen. In beiden Fällen erhält man

$$\mathbf{R}_{L1} = \frac{\mathbf{S}_M + \mathbf{C}}{1 - \tau_{CL} \cdot (\mathbf{S}_M + \mathbf{C})}$$

Darin gehen die Größen $D_{LR}$, $d_L$ und $\mathbf{L}_2$ nun nicht mehr ein, es ist also ausreichend, $\mathbf{S}_M$, $\mathbf{C}$ und $d_{CL}$ zu kennen.

Mit relativ wenig Rechenaufwand verbunden ist eine Ausführungsform, in der der Vergleich nach der Brechung an der Hornhaut durchgeführt wird. In diesem Fall werden nur noch $\mathbf{S}_M$ und $\mathbf{C}$ berücksichtigt:

$$\mathbf{R}'_C = \mathbf{S}_M + \mathbf{C}$$

Eine weitere sehr effiziente Möglichkeit ist der Positionierung der Bewertungsfläche an der Austrittspupille des Modellauges. Diese liegt vorzugsweise vor der Linsenrückfläche.

Das Augenmodell und die Belegung desselben kann wie folgt erweitert werden:

Grundsätzlich kann das Augenmodell zwischen Hornhaut und Vorderkammer unterschieden. Dazu wird hinter der Hornhaut Vorderfläche $\boldsymbol{C}_1$ (vormals $\boldsymbol{C}$) im Abstand $d_C$ eine Hornhautrückfläche $\boldsymbol{C}_2$ eingeführt und für Hornhaut und Vorderkammer zwei unterschiedliche Brechungsindices $n_C$ bzw. $n_{CL}$ angegeben. Ferner wird oben ausgeführte erste Schritt (Brechung der Wellenfront S an der Hornhaut $\boldsymbol{C}$ zu Wellenfront $\boldsymbol{S}'_C$ mit Vergenzmatrix $\mathbf{S'}_C = \mathbf{S} + \mathbf{C}$ ) durch folgende drei Schritte ersetzt:

1a: Brechung der Wellenfront $\boldsymbol{s}$ an der Hornhautvorderfläche $\boldsymbol{c}_1$ zur Wellenfront $\boldsymbol{S}'_{C1}$ mit der Vergenzmatrix $\mathbf{s'}_{C1}$ = $\mathbf{s} + \mathbf{c}_1$

1b: Propagation um die Dicke der Hornhaut $d_C$ zur Wellenfront $\boldsymbol{S}_{C2}$ mit Vergenzmatrix $\mathbf{s}_{C2} = \mathbf{s'}_{C1}/(\mathbf{1} - \tau_C\mathbf{s'}_{C1})$

1c: Brechung an der Hornhautrückfläche $\boldsymbol{c}_2$ zur Wellenfront $\boldsymbol{s}'_{C2}$ mit Vergenzmatrix $\mathbf{s'}_{C2} = \mathbf{s}_{C2} + \mathbf{c}_2$

$$\tau_C = \frac{d_C}{n_C}$$

wobei

Analog zu den anderen Werten können auch hier die Werte für $d_C$ und $\boldsymbol{C}_2$ jeweils gemessen, der Literatur entnommen oder abgeleitet werden. Beispielhaft seien hier einige Möglichkeiten für $\boldsymbol{C}_2$ beschrieben:

So kann - falls keine Messung an der Hornhautrückfläche vorliegt - die Form der Hornhautrückfläche aus bekannten Augenmodellen entnommen werden. Alternativ kann in diesem Fall auch die Hornhautrückfläche aus der gemessenen Form der Hornhautvorderfläche abgeleitet werden. Dazu bietet es sich an, entweder eine gleichmäßige Hornhautdicke (definiert beispielsweise als "in Richtung der Pfeilhöhe" oder "in radialer Richtung von einem "Hornhautkrümmungsmittelpunkt" ausgehend") anzunehmen. Die Dicke kann dabei entweder einer Messung entnommen, aus dieser abgeleitet oder der Literatur entnommen werden. Weiterhin können lokale Eigenschaften auch nur teilweise auf die Rückfläche übertragen werden.

Wird lediglich ein Hauptschnitt der Hornhautrückfläche gemessen, kann diese Information verwendet werden, um die vollständige Rückfläche zu rekonstruieren. Dies kann beispielsweise durch das Aufstellen einer Funktion der Dicke oder Pfeilhöhe der Hornhautrückfläche vom Radius oder der Dicke von der Pfeilhöhe der Vorderfläche geschehen.

In den meisten derartigen Fällen werden dabei die Hornhaut Vorder- und Rückfläche in demselben Normalschnitt (d.h. hier in der x-Richtung) bekannt sein.

Die Tatsache, dass es sich bei dem menschlichen Auge um ein nicht-zentriertes optisches System handelt, kann dadurch Rechnung getragen werden, dass die optischen Elemente gegen eine zentrale Achse versetzt und/oder verkippt angeordnet werden.

Dies kann sich auf die einzelnen Elemente als Ganzes (d.h. Hornhaut und Linse) oder alle brechenden Flächen einzeln (Hornhautvorderfläche, ggf. Hornhautrückfläche, Linsenvorderfläche und Linsenrückfläche) beziehen. Die entsprechenden Parameter sind jeweils beispielsweise zwei laterale Koordinaten der Verschiebung des Zentrums des Elements bzw. der Fläche von der zentralen Achse und zwei Kippwinkel. Alternativ können auch Zernikekoeffizienten erster Ordnung (Tip/Tilt) herangezogen werden.

Die relevante Größe, die von der Änderung gegenüber einem zentrierten System betroffen ist, ist der Hauptstrahl, der der Erfindung für alle Berechnungen zugrunde liegt und dem bis jetzt behandeltem zentrierten Systemen der optischen Achse entspricht. Im allgemeinen Fall ist der Hauptstrahl derjenige Strahl, der von der Retina als Zentrum der Mess-Wellenfront (bevorzugt dem Ort der Fovea) ausgeht und durch die Mitte der Eintrittspupille trifft. Anders als im zentrierten System, in dem dieser Strahl in geeigneten Koordinaten mit der globalen z-Achse des Augenmodells zusammenfällt, ist der Strahl nun nur noch abschnittsweise von Grenzfläche zu Grenzfläche gerade und trifft auch auf jeder Grenzfläche dezentriert und unter bestimmten Einfallswinkeln auf. Vor Berechnung der Wellenfronten (in zweiter Ordnung oder in höherer Ordnung) müssen der Verlauf des Hauptstrahls, die Positionen der Durchstoßpunkte und die jeweiligen Einfallswinkel bestimmt werden.

Wenn die Änderungen der einzelnen Elemente gegenüber einem zentrierten System klein sind, kann der Hauptstrahl näherungsweise durch folgende affine Gleichungen bestimmt werden. Diese entsprechen einer affin erweiterten Form der linearen Optik in Bezug auf ein globales Koordinatensystem. Jede Propagation eines Strahls mit lateraler Koordinate r und Richtungswinkel $a$ gegen die globale z-Achse um eine Länge d wird dabei durch die 2x2-Transfermatrix-Gleichung

$$\begin{pmatrix} r' \\ \alpha' \end{pmatrix} = \begin{pmatrix} 1 & d \\ 0 & 1 \end{pmatrix} \begin{pmatrix} r \\ \alpha \end{pmatrix} \tag{10a}$$

auf den propagierten Strahl mit lateraler Koordinate $r'$ und Richtungswinkel $\alpha'$ abgebildet. Die Brechung dagegen wird durch die erweiterte 2x2-Transfermatrix-Gleichung

$$\begin{pmatrix} r' \\ \alpha' \end{pmatrix} = \begin{pmatrix} 1 & 0 \\ \left(\frac{n}{n'} - 1\right)\rho & \frac{n}{n'} \end{pmatrix} \begin{pmatrix} r \\ \alpha \end{pmatrix} + \begin{pmatrix} \Delta r \\ \Delta \alpha \end{pmatrix} \tag{10b}$$

beschrieben. Dabei sind $\rho$ die Krümmung der brechenden Fläche und $n, n'$ die Brechungsindizes vor und nach der Brechung. Weiter sind $\Delta r$ und $\Delta \alpha$ Korrekturanteile der Strahlparameter, die durch die laterale Verschiebung und die Verkippung der brechenden Grenzfläche zustande kommen, und aus dem Verkippungs- und Verschiebungsparametern der Fläche etwa mit Hilfe der Prentice'schen Regel bestimmt werden können. Im Falle zylindrischer Flächen sind entsprechend die 4x4-Transfermatrix-Gleichungen zu verwenden.

Wenn die in Gl.(10a) und (10b) beschriebene Näherung nicht ausreicht, kann der Hauptstrahl, d.h. alle Durchstoßpunkte durch die Flächen numerisch bestimmt werden. In beiden Fällen bewirkt die Hauptstrahlbestimmung, dass alle Propagationsabstände, die Koordinaten der Durchstoßpunkte und die Ein- und Ausfallswinkel $\varepsilon, \varepsilon'$ an jeder Grenzfläche bestimmt werden. Im Falle der affinen Gleichungen ergeben sich $\varepsilon, \varepsilon'$ aus $\alpha, \alpha'$, und den Flächennormalen, die sich aus $r$, der Dezentration und der dioptrischen Wirkung gemäß der Prentice'schen Regel am Durchstoßpunkt bestimmen lassen. Im allgemeinen Fall ergeben sich $\varepsilon, \varepsilon'$ aus der numerischen Hauptstrahldurchrechnung und den Flächennormalen am Durchstoßpunkt $r$. Letztere können z.B. durch Ableiten der Flächendarstellung (z.B. Taylordarstellung oder Zernikedarstellung um den Punkt $r = 0$, oder B-Splines) an der Stelle $r$ berechnet werden.

Die Flächenbrechwertmatrix **C** ist im Fall der affinen Gleichungen konstant und durch das jeweilige brechende Element gegeben. Im Fall der numerischen Durchrechnung ergibt sich **C** am Durchstoßpunkt durch die lokalen zweiten Ableitungen in Bezug auf ein lokales Koordinatensystem.

Mit den derart berechneten Ein- bzw. Ausfallswinkeln $\varepsilon, \varepsilon'$ und der gegebenenfalls neu bestimmten Flächenbrechwertmatrix **C** können die Berechnungsverfahren der Erfindung wie folgt beschrieben auch dezentrierte Systeme angewandt werden:

In zweiter Ordnung tritt bei der Brechung an Stelle der Vergenzgleichung in Matrixform $S'_C = S + C$ die generalisierte Coddingtongleichung

$$\mathrm{Cos}(\varepsilon')\, S'_C\, \mathrm{Cos}(\varepsilon') = \mathrm{Cos}(\varepsilon)\, S\, \mathrm{Cos}(\varepsilon) + \nu\, C \tag{11}$$

mit

$$\nu = \frac{n'\cos\varepsilon' - n\cos\varepsilon}{n'-n}$$

$$\mathbf{Cos}(\varepsilon) = \begin{pmatrix} 1 & 0 \\ 0 & \cos(\varepsilon) \end{pmatrix} \quad \text{und} \quad \mathbf{Cos}(\varepsilon') = \begin{pmatrix} 1 & 0 \\ 0 & \cos(\varepsilon') \end{pmatrix}$$

(11a)

An Stelle der Propagationsgleichung **S' = S/(1 - τS)** mit τ = d/n tritt die Matrixgleichung

$$\mathbf{S'} = \mathbf{S}/(\mathbf{1} - \tau_{\alpha,r} \cdot \mathbf{S}) \quad \text{mit} \quad \tau_{\alpha,r} = d_{\alpha,r}/n$$

(12)

Dabei bezeichnet $d_{\alpha,r}$ den tatsächlichen räumlichen Abstand zwischen den Durchstoßpunkten der aufeinanderfolgenden Flächen.

Sollen HOA mit berücksichtigt werden, sind für Brechung und Propagation statt der Gleichungen (11) und (12) die entsprechend erweiterten Gleichungen für die jeweiligen Ordnungen aus den Publikationen von G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" zu verwenden und dazu die Koeffizienten der Taylorentwicklung der brechenden Fläche in dem Koordinatensystem des Strahleinfalls wie ebenda beschrieben zu ermitteln.

Weiterhin kann eine - ebenfalls auch verschobene bzw. verkippte - Blende eingeführt werden um die Vignettierung durch die Regenbogenhaut zu berücksichtigen

Zweiter Ansatz

In den folgenden Absätzen werden, soweit nicht ausdrücklich etwas anderes erwähnt ist, nun Details zu beispielhaften und bevorzugten Implementierungen des zweiten Ansatzes der Erfindung beschrieben:
Fig. 1 zeigt eine schematische Darstellung des physiologischen und physikalischen Modells eines Brillenglases und eines Auges in einer vorgegebenen Gebrauchsstellung zusammen mit einem beispielhaften Strahlverlauf, welches einer individuellen Brillenglasberechnung bzw. -optimierung gemäß einer bevorzugten Ausführungsform der Erfindung zugrunde liegt.

Hierbei wird pro Durchblickspunkt des Brillenglases vorzugsweise nur ein einziger Strahl berechnet (der Hauptstrahl 10, der vorzugsweise durch den Augendrehpunkt Z' läuft), begleitend aber außerdem auch die Ableitungen der Pfeilhöhen der Wellenfront nach den transversalen (zum Hauptstrahl senkrechten) Koordinaten. Diese Ableitungen werden bis zur gewünschten Ordnung berücksichtigt, wobei die zweiten Ableitungen die lokalen Krümmungseigenschaften der Wellenfront beschreiben und die höheren Ableitungen mit den Abbildungsfehlern höherer Ordnungen zusammenhängen.

Bei der Durchrechnung von Licht durch das Brillenglas bis in das Auge 12 gemäß dem individuell bereitgestellten Augenmodell werden die lokalen Ableitungen der Wellenfronten im Endeffekt an einer geeigneten Position im Strahlverlauf ermittelt, um sie dort mit einer Referenzwellenfront zu vergleichen, welche in einem Punkt auf der Retina des Auges 12 konvergiert. Insbesondere werden die beiden Wellenfronten (d.h. die vom Brillenglas kommende Wellenfront und die Referenzwellenfront) in einer Bewertungsfläche miteinander verglichen.

Unter "Position" ist dabei nicht einfach nur ein bestimmter Wert der z-Koordinate (in Lichtrichtung) gemeint, sondern ein solcher Koordinatenwert in Kombination mit der Angabe aller Flächen, durch die vor Erreichen der Bewertungsfläche gebrochen wurde. In einer bevorzugten Ausführungsform wird durch alle brechenden Flächen einschließlich der Linsenrückfläche gebrochen. In diesem Fall dient als Referenzwellenfront vorzugsweise eine sphärische Wellenfront, deren Krümmungsmittelpunkt auf der Retina des Auges 12 liegt.

Besonders bevorzugt wird ab dieser letzten Brechung nicht weiter propagiert, so dass der Krümmungsradius dieser Referenzwellenfront gerade dem Abstand zwischen Linsenrückfläche und Retina entspricht. In einer alternativen Möglichkeit wird nach der letzten Brechung noch propagiert, und zwar bevorzugt bis zur Austrittspupille AP des Auges 12. Diese liegt beispielsweise im Abstand $d_{AR} = d_{LR}^{(b)} = d_{LR} - d_{LR}^{(a)} > d_{LR}$ vor der Retina und damit sogar vor der Linsenrückfläche, so dass die Propagation in diesem Fall eine Rückpropagation ist (die Bezeichnungen $d_{LR}^{(a)}$, $d_{LR}^{(b)}$

werden weiter unten noch bei der Aufzählung der Schritte 1-6 beschrieben). Auch in diesem Fall ist die Referenzwellenfront sphärisch mit Krümmungsmittelpunkt auf der Retina, besitzt aber Krümmungsradius $1/d_{AR}$.

Hierzu wird angenommen, dass eine sphärische Wellenfront $w_0$ vom Objektpunkt ausgeht und bis zur ersten Brillenglasfläche 14 propagiert. Dort wird sie gebrochen und anschließend propagiert sie bis zur zweiten Brillenglasfläche 16, wo sie wieder gebrochen wird. Die vom Brillenglas austretenden Wellenfront $w_{g1}$ propagiert anschließend entlang des Hauptstrahls in Richtung des Auges 12 (propagierte Wellenfront $w_{g2}$) bis sie auf die Cornea 18 trifft, wo sie wiederum gebrochen wird (Wellenfront $w_c$). Nach einer weiteren Propagation innerhalb der Augenvorderkammer bis zur Augenlinse 20, wird die Wellenfront auch von der Augenlinse 20 wiederum gebrochen, wodurch beispielsweise an der Rückfläche der Augenlinse 20 oder an der Austrittspupille des Auges die resultierenden Wellenfront $w_e$ entsteht. Diese wird mit der sphärischen Referenzwellenfront $w_s$ verglichen und die Abweichungen werden für alle Durchblickspunkte in der Zielfunktion (vorzugsweise mit entsprechenden Gewichtungen für die einzelnen Durchblickspunkte) bewertet.

Somit wird die Fehlsichtigkeit nicht mehr nur durch eine dünne sphäro-zylindrische Linse beschrieben, wie dies in vielen herkömmlichen Verfahren üblich war, sondern vorzugsweise werden die Hornhauttopographie, die Augenlinse, die Abstände im Auge und die Deformation der Wellenfront (einschließlich der Abbildungsfehler niedriger Ordnung - also Sphäre, Zylinder und Achslage - sowie vorzugsweise auch einschließlich der Abbildungsfehler höherer Ordnung) im Auge direkt berücksichtigt.

Vorzugsweise liefert eine Aberrometermessung die individuellen Wellenfrontdeformationen des realen fehlsichtigen Auges für Ferne und Nähe (Abweichungen, keine absoluten Brechwerte) und die individuellen mesopischen und photopischen Pupillendurchmesser. Aus einer Messung der Hornhauttopographie (flächenhafte Vermessung der Cornea-Vorderfläche) erhält man vorzugsweise eine individuelle reale Hornhautvorderfläche, die im Allgemeinen nahezu 75% des Gesamtbrechwertes des Auges ausmacht. In einer bevorzugten Ausführungsform ist es nicht notwendig, die Cornea-Rückfläche zu vermessen. Sie wird vorzugsweise wegen des geringen Brechzahlunterschiedes zum Kammerwasser und wegen der geringen Hornhautdicke in guter Näherung nicht durch eine separate brechende Fläche, sondern durch eine Anpassung des Brechungsindex der Cornea beschrieben.

Generell sollen in dieser Beschreibung fett gesetzte Kleinbuchstaben Vektoren und fett gesetzte Großbuchstaben Matrizen bezeichnen, wie z.B. die (2 x 2) -Vergenzmatrizen bzw. -Brechwertmatrizen

$$\mathbf{S} = \begin{pmatrix} S_{xx} & S_{xy} \\ S_{xy} & S_{yy} \end{pmatrix}, \qquad \mathbf{C} = \begin{pmatrix} C_{xx} & C_{xy} \\ C_{xy} & C_{yy} \end{pmatrix}, \qquad \mathbf{L} = \begin{pmatrix} L_{xx} & L_{xy} \\ L_{xy} & L_{yy} \end{pmatrix}, \qquad \mathbf{1} = \begin{pmatrix} 1 & 0 \\ 0 & 1 \end{pmatrix}$$

und kursiv gesetzte wie d skalare Größen.

Weiterhin sollen fette, kursiv gesetzte Großbuchstaben Wellenfronten oder Flächen als Ganzes bezeichnen. So ist z.B. S die Vergenzmatrix der gleichnamigen Wellenfront S, nur dass S außer den Aberrationen 2-ter Ordnung, die in S zusammengefasst sind, auch die Gesamtheit aller Aberrationen höherer Ordnung (=HOA für engl. *Higher Order Aberrations*) der Wellenfront mit umfasst. Mathematisch gesehen steht *S* für die Menge aller Parameter, die notwendig sind, um eine Wellenfront (ausreichend genau) in Bezug auf ein gegebenes Koordinatensystem zu beschreiben. Vorzugsweise steht S für einen Satz von Zernike-Koeffizienten mit einem Pupillenradius oder einen Satz aus Koeffizienten einer Taylorreihe. Besonders bevorzugt steht S für die Menge aus einer Vergenzmatrix S zur Beschreibung der Wellenfronteigenschaften 2. Ordnung und einem Satz von Zernike-Koeffizienten (mit einem Pupillenradius), der zur Beschreibung aller restlichen Wellenfronteigenschaften außer der 2.Ordnung dient oder einem Satz von Koeffizienten gemäß einer Zerlegung nach Taylor. Für Flächen statt Wellenfronten gelten analoge Aussagen.

Unter anderem können folgende Daten im Prinzip direkt gemessen werden:

- Die Wellenfront $\boldsymbol{S}_M$, die durch den Laserspot auf der Retina und den Durchgang durch das Auge erzeugt wird (aus aberrometrischer Messung)

- Form der Hornhautvorderfläche C (durch Hornhaut-Topographie)

- Abstand zwischen Hornhaut und Linsenvorderfläche $d_{CL}$ (durch Pachymetrie). Diese Größe kann auch indirekt durch die Messung des Abstandes zwischen der Hornhaut und der Regenbogenhaut ermittelt werden, dabei können gegebenenfalls Korrekturwerte angewendet werden. Derartige Korrekturen können der Abstand zwischen der Linsenvorderfläche und der Regenbogenhaut aus bekannten Augenmodellen (z.B. Literaturwerte) sein.

- Krümmung der Linsenvorderfläche in einer Richtung $L_{1xx}$ (durch Pachymetrie) Dabei kann ohne Einschränkung der Allgemeinheit die x-Ebene beispielsweise derart definiert werden, dass dieser Schnitt in der x-Ebene liegt. Wird das Koordinatensystem so definiert, dass diese Ebene schief liegt, muss die Ableitung durch die Funktionen des entsprechenden Winkels ergänzt werden. Es wird nicht gefordert, dass es sich dabei um einen Hauptschnitt handelt. Beispielsweise kann es sich um den Schnitt in der horizontalen Ebene handeln.

Weiterhin können folgende Daten - je nach Ausführungsform - entweder gemessen oder der Literatur entnommen werden:

- Dicke der Linse $d_L$

- Krümmung der Linsenrückfläche in derselben Richtung wie die Linsenvorderfläche $L_{2,xx}$ (durch Pachymetrie)

Damit gibt es für die Linsenrückfläche folgende Möglichkeiten:

- Messung von $L_{2,xx}$ ($L_{2,M}$) und Annahme einer Rotationssymmetrie $L_{2,xx} = L_{2,yy} = L_2 = L_{2,M}$ und $L_{2,xy} = L_{2,yx} = 0$

- Entnahme von $L_{2,xx}$ aus der Literatur ($L_{2,Lit}$) und Annahme einer Rotationssymmetrie $L_{2,xx} = L_{2,yy} = L_2 = L_{2,M}$ und $L_{2,xy} = L_{2,yx} = 0$

- Entnahme der vollständigen (unsymmetrischen) Form $L_2$ aus der Literatur ($L_{2,Lit}$)

- Messung von $L_{2,xx}$ ($L_{2,M}$) und Annahme eines Zylinders oder einer anderweitig spezifizierten Asymmetrie $a_{Lit}$ aus der Literatur
  $L_{2,xx} = L_{2,M}$ und $L_{2,xy} = L_{2,yx} = f(L_{2,xx}, a_{Lit})$ sowie $L_{2,yy} = g(L_{2,xx}, a_{Lit})$

Folgende Daten können der Literatur entnommen werden:

- Brechungsindices $n_{CL}$ von Hornhaut und Augenvorderkammer sowie des Kammerwassers $n_{LR}$ und der der Linse $n_L$

Damit verbleiben insbesondere der Abstand $d_{LR}$ zwischen Linsenrückfläche und Netzhaut sowie die Komponenten $L_{1,yy}$ und $L_{1,xy} = L_{1,yx}$ der Linsenvorderfläche als unbekannte Parameter. Zur Vereinfachung des Formalismus kann ersterer auch als Vergenzmatrix $\mathbf{D}_{LR} = D_{LR} \cdot \mathbf{1}$ mit $D_{LR} = n_{LR}/d_{LR}$ geschrieben werden. Weiterhin wird generell die Größe $\tau$ verwendet, die definiert ist als $\tau = d/n$ (wobei für den Brechungsindex als $n$ immer der entsprechende Index zu verwenden ist wie für $d$ und $\tau$, z.B. als $\tau_{LR} = d_{LR}/n_{LR}$, $\tau_{CL} = d_{CL}/n_{CL}$).

Die Modellierung des Durchgangs der Wellenfront durch das erfindungsgemäß verwendete Augenmodell, also nach dem Durchgang durch die Flächen des Brillenglases, kann in einer bevorzugten Ausführungsform, in der die Linse über eine Vorder- und eine Rückfläche beschrieben wird, wie folgt beschrieben werden, wobei die Transformationen der Vergenzmatrizen explizit angegeben wird:

7. Brechung der Wellenfront S mit der Vergenzmatrix S an der Hornhaut C mit der Flächenbrechwertmatrix C zur Wellenfront $\boldsymbol{S'}_C$ mit Vergenzmatrix $\boldsymbol{S'}_C = S + C$

8. Propagation um die Vorderkammertiefe $d_{CL}$ (Abstand zwischen Hornhaut und Linsenvorderfläche) zur Wellenfront $\boldsymbol{S}_{L1}$ mit Vergenzmatrix $\boldsymbol{S}_{L1} = \boldsymbol{S'}_C/(1 - \tau_{CL} \cdot S')$

$$\mathbf{S}_{L1} = \frac{\mathbf{S}'_C}{(1 - \tau_{CL} \cdot \mathbf{S}'_C)}$$

9. Brechung an der Linsenvorderfläche $\boldsymbol{L}_1$ mit der Flächenbrechwertmatrix $\mathbf{L}_1$ zur Wellenfront $\boldsymbol{S'}_{L1}$ mit der Vergenzmatrix $\boldsymbol{S'}_{L1} = \boldsymbol{S}_{L1} + \mathbf{L}_1$

10. Propagation um die Linsendicke $d_L$ zur Wellenfront $\boldsymbol{S}_{L2}$ mit Vergenzmatrix

$$\mathbf{S}_{L2} = \mathbf{S}'_{L1}/(1 - \tau_L \cdot \mathbf{S}'_{L1})$$

11. Brechung an der Linsenrückfläche $\boldsymbol{L}_2$ mit der Flächenbrechwertmatrix $\mathbf{L}_2$ zur Wellenfront $\boldsymbol{S'}_{L2}$ mit Vergenzmatrix $\boldsymbol{S'}_{L2} = \boldsymbol{S}_{L2} + \mathbf{L}_2$

12. Propagation um den Abstand zwischen Linse und Netzhaut $d_{LR}$ zur Wellenfront $\boldsymbol{S}_R$ mit der Vergenzmatrix $\boldsymbol{S}_R = \boldsymbol{S'}_{L2}/(1 - \tau_{LR} \cdot \boldsymbol{S'}_{L2})$

Jeder der Schritte 2, 4, 6, bei denen um die Abstände $\tau_{CL}$, $\tau_{CL}$, bzw. $\tau_{CL}$ propagiert wird, kann dabei aufgeteilt werden in zwei Teilpropagationen 2a,b), 4a,b) bzw. 6a,b) nach dem folgenden Schema, das für den Schritt 6a,b) explizit lautet:

6a. Propagation um den Abstand $d_{LR}^{(a)}$ zwischen Linse und Zwischenebene zur Wellenfront $\boldsymbol{S}_{LR}$ mit der Vergenzmatrix $\mathbf{S}_{LR} = \mathbf{S}'_{L2} / (1 - \tau_{LR}^{(a)} \mathbf{S}'_{L2})$

6b. Propagation um den Abstand $d_{LR}^{(b)}$ zwischen Zwischenebene und Netzhaut zur Wellenfront $\boldsymbol{S}_R$ mit der Vergenzmatrix $\mathbf{S}_R = \mathbf{S}_{LR} / (1 - \tau_{LR}^{(b)} \mathbf{S}_{LR})$

Dabei können $\tau_{LR}^{(a)} = d_{LR}^{(a)} / n_{LR}^{(a)}$ und $\tau_{LR}^{(b)} = d_{LR}^{(b)} / n_{LR}^{(b)}$ positiv oder negativ sein, wobei stets

$n_{LR}^{(a)} = n_{LR}^{(b)} = n_{LR}$ und $\tau_{LR}^{(a)} + \tau_{LR}^{(b)} = \tau_{LR}$ sein soll. In jedem Fall lassen sich Schritt 6a und 6b durch

$\mathbf{S}_R = \mathbf{S}'_{L2} / (1 - (\tau_{LR}^{(a)} + \tau_{LR}^{(b)}) \mathbf{S}'_{L2}) = \mathbf{S}'_{L2} / (1 - \tau_{LR} \mathbf{S}'_{L2})$ wieder zusammenfassen. Die Aufteilung in Schritt 6a und 6b bietet aber Vorteile, und bevorzugt kann die Zwischenebene in die Ebene der Austrittspupille AP gelegt werden, die vorzugsweise vor der Linsenrückfläche liegt. In diesem Fall ist $\tau_{LR}^{(a)} < 0$ und $\tau_{LR}^{(b)} > 0$.

Analog zur Aufteilung von Schritt 6 in 6a,b) kann auch die Aufteilung der Schritte 2,4 erfolgen.

Entscheidend für die Wahl der Bewertungsfläche der Wellenfront ist also nicht nur die absolute Lage in Bezug auf die z-Koordinate (in Lichtrichtung), sondern auch die Anzahl der Flächen, durch die bis zur Bewertungsfläche schon gebrochen wurde. So kann ein- und dieselbe Ebene mehrmals durchlaufen werden. Beispielhaft wird die Ebene der AP (die normalerweise zwischen der Linsenvorderfläche und der Linsenrückfläche liegt) vom Licht formal zum ersten Mal durchlaufen nach einem gedachten Schritt 4a, bei dem von der Linsenvorderfläche um die Länge $\tau_L^{(a)} > 0$ propagiert wird. Zum zweiten Mal wird die gleiche Ebene erreicht nach Schritt 6a, wenn nach der Brechung durch die Linsenrückfläche wieder zur AP-Ebene zurückpropagiert wird, d.h. $\tau_{LR}^{(a)} = -\tau_L + \tau_L^{(a)} = -\tau_L^{(b)} < 0$, was gleichbedeutend ist mit

$\tau_{LR}^{(a)} = \tau_{LR} - \tau_{LR}^{(b)} < 0$. Bei den Wellenfronten $\boldsymbol{S}_{AP}$, die sich im Text auf die AP beziehen, soll (falls nicht explizit anders erwähnt) vorzugsweise immer die Wellenfront $\boldsymbol{S}_{AP} = \boldsymbol{S}_{LR}$ gemeint sein, die Ergebnis von Schritt 6a ist."

Auf diese Schritte 1 bis 6 wird im weiteren Verlauf der Beschreibung immer wieder Bezug genommen. Sie beschreiben einen bevorzugten Zusammenhang zwischen der Vergenzmatrix S einer Wellenfront S an der Hornhaut und den Vergenzmatrizen aller daraus hervorgehenden Zwischenwellenfronten an den brechenden Zwischenflächen des Auges, insbesondere der Vergenzmatrix $\boldsymbol{S}'_{L2}$ einer Wellenfront $\boldsymbol{S}'_{L2}$ nach der Augenlinse (oder sogar einer Wellenfront $\boldsymbol{S}_R$ an der Netzhaut). Diese Zusammenhänge können sowohl dazu verwendet werden, a-priori nicht bekannte Parameter (z.B. $d_{LR}$ oder $\boldsymbol{L}_1$) zu berechnen und so das Modell entweder individuell oder generisch mit Werten zu belegen, als auch um mit dann belegten Modellen die Ausbreitung der Wellenfront im Auge zur Optimierung von Brillengläsern zu simulieren.

Bevor auf die erfindungsgemäße Vorgehensweise der Berücksichtigung von Abbildungsfehlern höherer Ordnung (d.h. höher als zweiter Ordnung insbesondere in Taylor- oder Zernike-Zerlegung der Abbildungsfehler) eingegangen wird, soll im Folgenden der Einfachheit halber ein beispielhaftes Prinzip des Formalismus anhand einer Beschreibung der Flächen und Wellenfronten bis in zweiter Ordnung beschrieben werden, wozu eine Darstellung durch Vergenzmatrizen ausreichend ist. Wie anschließend dargelegt wird, kann dieser Formalismus analog auf für die Implementierung der Erfindung unter Berücksichtigung höherer Ordnungen von Abbildungsfehlern genutzt werden.

In einer Beschreibung in zweiter Ordnung besitzt das Augenmodell in einer bevorzugten Ausführungsform zwölf Parameter als Freiheitsgrade des Modells, die belegt werden müssen. Diese umfassen vorzugsweise die drei Freiheitsgrade der Flächenbrechwertmatrix C der Cornea C , die jeweils drei Freiheitsgrade der Flächenbrechwertmatrizen $\boldsymbol{L}_1$ und $\boldsymbol{L}_2$ für die Linsenvorder- bzw. Rückfläche, sowie jeweils einen für die Längenparameter Vorderkammertiefe $d_{CL}$, Linsendicke $d_L$ und die Glaskörperlänge $d_{LR}$.

Belegungen dieser Parameter können im Prinzip auf mehrere Weisen erfolgen:

iv) Direkte, also individuelle Messung eines Parameters
v) A priori gegebener Wert eines Parameters, z.B. als Literaturwert oder aus einer Schätzung, beispielsweise durch Vorliegen eines Messwertes für eine andere Größe, die anhand einer vorangegangenen Populationsanalyse auf bekannte Weise mit dem zu bestimmenden Parameter korreliert
vi) Berechnung aus Konsistenzbedingungen, z.B. Verträglichkeit mit einer bekannten Refraktion

Die gesamte Anzahl $df_2$ von Freiheitsgraden des Augenmodells in zweiter Ordnung ($df$ steht für 'degree of freedom', Index '2' für 2.Ordnung) setzt sich also zusammen aus

$$df_2 = df_2(i) + df_2(ii) + df_2(iii)$$

Liegen beispielsweise für alle zwölf Modellparameter direkte Messwerte vor, dann ist $df_2(i) = 12$ , $df_2(ii) = 0$ und $df_2(iii) = 0$, was nachfolgend der Einfachheit halber durch die Schreibweise $df_2 = 12 + 0 + 0$ ausgedrückt wird. In einem solchen Fall ist auch die objektive Refraktion des betreffenden Auges festgelegt, so dass eine objektive Refraktionsbestimmung nicht mehr zusätzlich durchgeführt werden müsste.

Für die Implementierung der vorliegenden Erfindung ist es nicht notwendig, alle Parameter direkt zu messen. So ist es unter Umständen einfacher, die Refraktion des betreffenden Auges zu messen bzw. objektiv und/oder subjektiv zu bestimmen, als alle Parameter des Modellauges individuell zu vermessen. Vorzugsweise liegt somit zumindest eine Refraktion, also Messdaten zur Wellenfront $S_M$ des Auges bis zur zweiten Ordnung vor, die den Daten der Vergenzmatrix $S_M$ entsprechen. Bei einer Belegung des Augenmodells auf Basis rein objektiv gemessener Daten können diese Werte beispielsweise autorefraktometrischen Messungen entnommen werden, bzw. laut (ii) anderweitig gegebener Daten belegt werden. Die drei Bedingungen der Übereinstimmung mit den drei unabhängigen Parametern der Vergenzmatrix $S_M$ erlauben es damit, drei Parameter des Augenmodells abzuleiten, was in der oben eingeführten Notation $df_2(iii) = 3$ entspricht.

Es ist also möglich, in Fällen, in denen nicht alle Modellparameter direkten Messungen zugänglich sind, oder diese Messungen sehr aufwändig wären, die fehlenden Parameter sinnvoll zu belegen. Liegen beispielsweise höchstens für neun Modellparameter direkte Messwerte vor ($df_2(i) \leq 9$), dann kann man die genannten Bedingungen der Refraktion benutzen, um drei der Modellparameter zu berechnen ($df_2(iii) = 3$). Falls genau $df_2(i) = 9$ gilt, dann sind alle zwölf Modellparameter durch die Messungen und die Berechnung eindeutig bestimmt, und es gilt ($df_2(ii) = 0$). Ist dagegen $df_2(i) < 9$, dann ist $df_2(ii) = 9 - df_2(i) > 0$, d.h. das Modell ist unterbestimmt in dem Sinne, dass $df_2(ii)$ Parameter a priori festgelegt werden müssen.

Mit dem Bereitstellen einer individuellen Refraktion, also Messdaten zur Wellenfront $S_M$ des Auges bis zur zweiten Ordnung liegen die nötigen Daten der Vergenzmatrix $S_M$ entsprechend vor. Gemäß einem in WO 2013/104548 A1 beschriebenen Verfahren werden insbesondere die Parameter $\{C, d_{CL}, S_M\}$ gemessen. Hingegen werden vorzugsweise unter anderem die beiden Längenparameter $d_L$ und $d_{LR}$ (bzw. $D_{LR}$) a priori festgelegt (z.B. durch Literaturwerte oder Schätzung). In WO 2013/104548 A1 werden insbesondere die beiden Fälle unterschieden, in denen entweder $L_2$ a priori festgelegt und $L_1$ daraus berechnet wird, oder umgekehrt. Als Berechnungsvorschrift offenbart die genannte Offenlegungsschrift hierzu Gl.(4) bzw. Gl.(5). Für beide Fälle gilt $df_2 = 4 + 5 + 3$.

In der Bezeichnungsweise der oben genannten Schritte 1 bis 6 geschieht die Anpassung von $L_1$ an die Messungen insbesondere dadurch, dass man zum einen die gemessene Vergenzmatrix $S_M$ mittels der Schritte 1, 2 durch die ebenfalls gemessene Matrix C hindurch rechnet und bis zur objektseitigen Seite der Linsenvorderfläche propagiert. Zum anderen rechnet man von einer gedachten punktförmigen Lichtquelle auf der Netzhaut eine Kugelwelle mittels der rückwärts durchlaufenen Schritte 6, 5, 4 von hinten nach vorne, indem man diese Kugelwelle an der zuvor festgelegten Flächenbrechwertmatrix $L_2$ der Linsenrückfläche bricht und die dann erhaltene Wellenfront von der Linsenrückfläche bis zur bildseitigen Seite der Linsenvorderfläche propagiert. Die Differenz der so bestimmten Vergenzmatrizen $S_{L1}$ und $S'_{L1}$, die objektseitig bzw. bildseitig der Linsenvorderfläche liegen muss durch die Matrix $L_1$ bewirkt worden sein, denn bei der aberrometrischen Messung geht die gemessene Wellenfront aus einer Wellenfront hervor, die von einem Punkt auf der Netzhaut ausgeht, und deshalb aufgrund der Umkehrbarkeit der Strahlengänge identisch mit derjenigen einfallenden Wellenfront ist ($S = S_M$), die auf diesen Punkt der Netzhaut zusammenläuft. Dies führt zu Gleichung (4) in der genannten Offenlegungsschrift:

$$\mathbf{L}_1\left(D_{LR}\right) = \frac{D_{LR}\cdot\mathbf{1}-\mathbf{L}_2}{1+\tau_L\cdot\left(D_{LR}\cdot\mathbf{1}-\mathbf{L}_2\right)} - \frac{\mathbf{S}_M+\mathbf{C}}{1-\tau_{CL}\left(\mathbf{S}_M+\mathbf{C}\right)}$$

(1a)

Der andere Fall in der genannten Offenlegungsschrift betrifft die Anpassung der Matrix $\mathbf{L}_2$ an die Messungen, nachdem die Matrix $\mathbf{L}_1$ festgelegt worden ist. Ein Unterschied besteht jetzt lediglich darin, dass die gemessene Wellenfront $\mathbf{S}_M$ den Schritten 1, 2, 3, 4 unterzogen wird und die angenommene Wellenfront aus der punktförmigen Lichtquelle nur dem Schritt 6, und dass der zur Anpassung der Linsenrückfläche $\mathbf{L}_2$ zu erfolgende fehlende Schritt jetzt Schritt 5 ist, entsprechend Gl.(5) der genannten Offenlegungsschrift:

$$\mathbf{L}_2 = D_{LR} - \left(\frac{\mathbf{S}_M+\mathbf{C}}{1-\tau_{CL}\left(\mathbf{S}_M+\mathbf{C}\right)}+\mathbf{L}_1\right)\left(1-\tau_L\left(\frac{\mathbf{S}_M+\mathbf{C}}{1-\tau_{CL}\left(\mathbf{S}_M+\mathbf{C}\right)}+\mathbf{L}_1\right)\right)^{-1}$$

(1b)

In einer bevorzugten Implementierung der Erfindung wird zumindest einer der Längenparameter $d_L$ und $d_{LR}$ (bzw. $D_{LR}$) aus anderen gemessenen Daten und a-priori-Annahmen zu anderen Freiheitsgraden berechnet und insbesondere nicht selbst a-priori angenommen.

Vorzugsweise stehen die Daten der Vergenzmatrix $\mathbf{S}_M$ und besonders bevorzugt auch die Daten zu C aus individuellen Messungen zur Verfügung. In einer weiteren bevorzugten Ausführungsform wird bei einer Annahme von Daten zur Linsenrückfläche von einer sphärischen Rückfläche, d.h. einer Rückfläche ohne astigmatische Komponenten ausgegangen.

In einer bevorzugten Ausführungsform der Erfindung liegen also zur Cornea C Messdaten bis zur zweiten Ordnung vor, die den Daten der Flächenbrechwertmatrix **C** entsprechen. Obgleich diese Werte topographischen Messungen entnommen werden können, sind letztere nicht notwendig. Vielmehr sind topometrische Messungen ausreichend. Diese Situation entspricht dem Fall $df_2 = 3 + 6 + 3$, wobei insbesondere die Vorderkammertiefe $d_{CL}$ einer der sechs a priori festzulegenden Parameter ist.

Soweit keine weiteren individuellen Messungen vorgenommen werden, liegt eine Situation mit $df_2 = 3 + 6 + 3$ vor. Um $d_{LR}$ eindeutig bestimmen zu können, müssen also sechs Parameter aus $\{\mathbf{L}_1, \mathbf{L}_2, d_L, d_{CL}\}$ durch Annahmen bzw. Literaturwerte belegt werden. Die übrigen beiden ergeben sich zusätzlich zu $d_{LR}$ aus der Rechnung. In einer bevorzugten Ausführungsform werden die Parameter der Linsenrückfläche, die mittlere Krümmung der Linsenvorderfläche und die beiden Längenparameter $d_L$ und $d_{CL}$ a priori (als vorgegebene Standardwerte) belegt.

In einer bevorzugten Implementierung ist zusätzlich die Vorderkammertiefe $d_{CL}$ also der Abstand zwischen der Hornhaut und Linsenvorderfläche, beispielsweise aus pachymetrischen oder OCT-Messungen bekannt. Damit umfassen die gemessenen Parameter $\{\mathbf{C}, d_{CL}, \mathbf{S}_M\}$. Diese Situation entspricht dem Fall $df_2 = 4 + 5 + 3$. Das Problem ist demnach mathematisch noch unterbestimmt, es müssen also fünf Parameter aus $\{\mathbf{L}_1, \mathbf{L}_2, d_L\}$ durch Annahmen bzw. Literaturwerte a priori festgelegt werden. Hierbei handelt es sich in einer bevorzugten Ausführungsform um die Parameter der Linsenrückfläche, die mittlere Krümmung der Linsenvorderfläche und die Linsendicke. Der genaue Rechenweg für diesen Fall dargestellt weiter unten noch dargelegt.

Allein für die Genauigkeit der individuellen Anpassung ist es von Vorteil, möglichst viele Parameter mit individuellen Messungen belegen zu können. In einer bevorzugten Ausführungsform wird dazu zusätzlich die Linsenkrümmung in einem Normalschnitt auf Basis einer individuellen Messung bereitgestellt. Dadurch ergibt sich dann eine Situation gemäß $df_2 = 5 + 4 + 3$, und es reicht aus, vier Parameter aus $\{L_{1yy}, \alpha_{L1}, \mathbf{L}_2, d_L\}$ a priori festzulegen. Auch hier handelt es sich in einer bevorzugten Ausführungsform wieder um die Parameter der Linsenrückfläche und die Linsendicke. Die genaue Durchrechnung wieder weiter unten noch beschrieben.

Insbesondere alternativ zum Normalschnitt der Linsenvorderfläche und besonders bevorzugt zusätzlich zur Vorderkammertiefe kann auch die Linsendicke aus einer individuellen Messung zur Verfügung gestellt werden. Dadurch entfällt die Notwendigkeit, diesen Parameter mit Modelldaten oder Schätzparametern zu belegen ($df_2 = 5 + 4 + 3$). Ansonsten gilt das oben bereits ausgeführte. Diese Ausführungsform ist besonders vorteilhaft, wenn ein Pachymeter eingesetzt wird, dessen Messtiefe die Erkennung der Linsenrückfläche erlaubt, nicht aber eine hinreichend sichere Bestimmung der Linsenkrümmungen.

Zusätzlich zur Vorderkammertiefe und einem Normalschnitt der Linsenvorderfläche können in einer bevorzugten Ausführungsform ein (z.B. Messung in zwei Normalschnitten) oder zwei weitere Parameter (Messung beider Hauptschnitte und der Achslage) der Linsenvorderfläche durch eine individuelle Messung erfasst werden. Diese zusätzlichen Informationen können insbesondere auf zwei Arten ausgenutzt werden:

- Aufgabe von a priori Annahmen: Es können ein bzw. zwei der ansonsten a priori getroffenen Annahmen aufgegeben und aus durch Berechnung bestimmt werden. In diesem Fall ergeben sich die Situationen $df_2$ = 6 + 3 + 3 bzw. $df_2$ = 7 + 2 + 3. So kann im ersten Fall die mittlere Krümmung der Rückfläche (bei Annahme einer astigmatismusfreien Rückfläche) und im zweiten Fall bei gegebener mittlerer Krümmung der Flächenastigmatismus (incl. Achslage) bestimmt werden. Alternativ kann in beiden Fällen auch die Linsendicke aus den Messungen bestimmt werden. Ein derartiges Vorgehen bedarf jedoch im Allgemeinen einer gewissen Umsicht, da verrauschte Messdaten leicht zu einem "Weglaufen" der freigegebenen Parameter führen können. Dadurch kann das Modell insgesamt deutlich schlechter anstatt besser werden. Eine Möglichkeit dies zu verhindern besteht darin, anatomisch sinnvolle Grenzwerte für diese Parameter vorzugeben und die Variation der Parameter auf diesen Bereich zu beschränken. Selbstverständlich können diese Grenzen auch abhängig von den gemessenen Werten vorgegeben werden.

- Verringerung der Messunsicherheit: Werden dagegen weiterhin die gleichen a priori Annahmen getroffen (vorzugsweise also $\{L_2, d_L\}$), liegen die Situationen $df_2$ = 6 + 4 + 3 bzw. $df_2$ = 7 + 4 + 3 vor, das System ist also mathematisch überbestimmt.

  Anstelle einer einfachen analytischen Bestimmung von $D_{LR}$ gemäß nachfolgender Ausführungen wird $D_{LR}$ (und gegebenenfalls der noch fehlende Parameter aus $L_1$) so bestimmt ("fit"), dass der Abstand zwischen dem sich aus den Gleichungen ergebenden $L_1$ und dem gemessenen $L_1$ (bzw. dem um den fehlenden Parameter ergänzten gemessenen $L_1$) minimal wird. Durch dieses Vorgehen kann - offensichtlich - eine Verringerung der Messunsicherheit erreicht werden.

In einer weiteren bevorzugten Implementierung werden die Vorderkammertiefe, zwei oder drei Parameter der Linsenvorderfläche und die Linsendicke individuell gemessen. Die Berechnung der übrigen Größen erfolgt dabei analog, wobei die a priori Annahme der Linsendicke durch die entsprechende Messung ersetzt werden kann.

In einer weiteren bevorzugten Implementierung werden individuelle Messungen der Vorderkammertiefe, mindestens eines Parameters der Linsenvorderfläche, der Linsendicke und mindestens eines Parameter der Linsenrückfläche bereitgestellt. Hierbei handelt es sich um eine Ergänzung der oben genannten Fälle. Die jeweiligen zusätzlich gemessenen Parameter können analog den schrittweisen Erweiterungen der obigen Abschnitte erfolgen. Diese Fälle sind besonders vorteilhaft, wenn die oben erwähnten Pachymetrieeinheiten, die in einer Ebene, zwei Ebenen oder über die ganze Fläche messen in der Messtiefe entsprechend erweitert und so präzise sind, dass sich die Krümmungsdaten hinreichend genau ermitteln lassen.

Nachfolgend wird anhand einiger Beispiele gezeigt, wie die Berechnung einzelner Parameter aus den übrigen gemessenen oder a priori festgelegten Parametern und anhand der individuellen Refraktionsdaten erfolgen kann.

Beispielsweise ist in bevorzugten Ausführungsformen eine Messung der Krümmung einer Linsenfläche in einem Normalschnitt verfügbar. Da die Rückfläche in der Praxis nicht gemessen werden kann, ohne dass auch die Vorderfläche gemessen wird, und die Messung der Vorderfläche bevorzugt stattfindet, werden nachfolgend die Gleichungen für die Fälle einer in einem Normalschnitt bekannten Krümmung der Linsenvorderfläche angegeben. Ist statt eines Normalschnittes der Linsenvorderfläche ein Normalschnitt der Linsenrückfläche gegeben (z.B. entsprechende Messungen, Modellannahmen), muss in analoger Weise mit Gl.(1b) verfahren werden. Ohne Beschränkung der Allgemeinheit legt man das Koordinatensystem so, dass der Normalschnitt in x -Richtung verläuft. In einem nächsten Schritt wertet man dann die Matrixgleichung (1a) in dem gegebenen Normalschnitt aus und löst nach $D_{LR}$ auf, und setzt diese Lösung anschließend wieder in Gl.(1a) ein zur vollständigen Angabe von $L_1$.

Setzt man die xx -Komponente von $L_1(D_{LR})$ aus Gl. (1) dem gemessenen Wert $L_{1,xx}$ gleich, erhält man für dieses Matrixelement eine in $D_{LR}$ quadratische Gleichung, deren positive Lösung dem Abstand zwischen Linsenrückfläche und Netzhaut entspricht:

$$D_{LR} = \frac{-b + \sqrt{b^2 - 4c}}{2a} \qquad (2)$$

Dabei gelten:

$$a = \tau_L(1 + \tau_L A)$$

$$b = 1 - \tau_L(\mathrm{tr}(L_2) - AB)$$

$$c = A - L_{2,xx} + \tau_L \det L_2 (1 + \tau_L A) - \tau_L A \, \mathrm{tr}(L_2)$$
$$\quad = A - L_{2,xx} + a \det L_2 - \tau_L A \, \mathrm{tr}(L_2)$$

$$(2a)$$

mit

$$A = -S_{M,L1,xx} - L_{1,xx}$$

$$B = 2 - \tau_L \operatorname{tr}(\mathbf{L}_2)$$

$$\det(\mathbf{L}_2) = L_{2,xx} L_{2,yy} - L_{2,xy}^2$$

$$\operatorname{tr}(\mathbf{L}_2) = L_{2,xx} + L_{2,yy} \tag{2b}$$

sowie

$$S_{M,L1,xx} = \frac{\tau_{CL} S'^2_{M,C,xy} + S'_{M,C,xx} \cdot \left(1 - \tau_{CL} S'_{M,C,yy}\right)}{-\tau_{CL}^2 S'^2_{M,C,xy} + \left(1 - \tau_{CL} S'_{M,C,xx}\right) \cdot \left(1 - \tau_{CL} S'_{M,C,yy}\right)}$$

$$S'_{M,C,xx} = S_{M,xx} + C_{xx} \quad \left(xy \text{ und } yy \text{ analog}\right) \tag{2c}$$

Für den Fall einer symmetrischen Linsenrückfläche ($\mathbf{L}_2 = L_{2,xx} \cdot \mathbf{1}$) vereinfacht sich dies zu:

$$D_{LR} = L_{2,xx} + \frac{L_{1,xx} + S_{M,L1,xx}}{1 - \tau_L \cdot \left(L_{1,xx} + S_{M,L1,xx}\right)} \tag{3}$$

mit $S_{M,L1,xx}$ aus Gleichung (2c).

In beiden Fällen ist es damit möglich, die Linsenvorderfläche $L_1$ zu berechnen, indem man das jeweilige gewonnene $D_{LR}$ in Gleichung (1a) einsetzt:

$$\mathbf{L}_1 = \frac{\mathbf{D}_{LR} - \mathbf{L}_2}{1 + \tau_L \cdot \left(\mathbf{D}_{LR} - \mathbf{L}_2\right)} - \frac{\mathbf{S}_M + \mathbf{C}}{1 - \tau_{CL} \left(\mathbf{S}_M + \mathbf{C}\right)} \tag{4}$$

Das Ergebnis ist naturgemäß symmetrisch ($L_{1,xy} = L_{1,yx}$) und reproduziert für die Komponente $L_{1,xx}$ den in (2b) bzw. (3) eingesetzten Wert.

In einigen bevorzugten Ausführungsformen steht eine individuelle Messung oder eine Vorgabe einer mittleren Krümmung einer Linsenfläche zur Verfügung. Diese Situation liegt beispielsweise dann vor, wenn die mittlere Krümmung der Linsenvorderfläche gemessen werden kann oder keine Messungen an den Linsenflächen durchgeführt werden können und die mittlere Krümmung einer Linsenfläche angenommen wird (z.B. aus der Literatur entnommen). Wie soeben wird auch hier das Verfahren für die Linsenvorderfläche beschrieben und ist analog auf die Linsenrückfläche übertragbar. Im diesem Fall einer gegebenen mittleren Sphäre $L_{1,ms}$ der Linsenvorderfläche sind die freien Parameter der Zylinder $L_{1,cyl}$ und die Achslage $\alpha_{L1}$. Mit $L_{1,diff} = L_{1,cyl}/2$ wird $\mathbf{L}_1$ zu

$$\mathbf{L}_1 = \begin{pmatrix} L_{1,ms} - L_{1,diff} \cdot \cos 2\alpha_{L1} & -L_{1,diff} \cdot \sin 2\alpha_{L1} \\ -L_{1,diff} \cdot \sin 2\alpha_{L1} & L_{1,ms} + L_{1,diff} \cdot \cos 2\alpha_{L1} \end{pmatrix} \tag{5}$$

Auch geht man wieder von Gl.(1a) aus. Setzt man nun die Ausdrücke für $\mathbf{L}_1$ aus den Gleichungen (5) und (1a) gleich, erhält man ein Gleichungssystem aus drei Gleichungen (die beiden Nichtdiagonalelemente sind identisch) und den drei Unbekannten $L_{1,diff}$, $\alpha_{L1}$ und $D_{LR}$. Dieses hat die physikalisch relevante Lösung

$$D_{LR} = \frac{-\bar{b} + \sqrt{\bar{b}^2 - 4\bar{a}\bar{c}}}{2\bar{a}}$$

$$L_{1diff} = \pm\sqrt{\sigma^2 + \gamma^2}$$

$$\alpha_{L1} = \frac{1}{2}\arctan(\pm\gamma, \pm\sigma) + \frac{\pi}{2}$$

(6)

mit

$$\bar{a} = \tau_L(1 + \tau_L\bar{A})$$

$$\bar{b} = 1 - \tau_L\left(\mathrm{tr}(\mathbf{L}_2) - \bar{A}B\right)$$

$$\bar{c} = \frac{1}{4}\left(\bar{A}B^2 - B\,\mathrm{tr}(\mathbf{L}_2) - \bar{a}\,\mathrm{Ast}(\mathbf{L}_2)^2\right)$$

und

$$\bar{A} = \bar{S}_{M,L1} - \bar{L}_{1,mess}$$

$$\mathrm{Ast}(\mathbf{L}_2) = \sqrt{\mathrm{tr}(\mathbf{L}_2)^2 - 4\det\mathbf{L}_2}$$

$$\gamma = \frac{2\left(-1 + \sqrt{\bar{b}^2 - 4\bar{a}\bar{c}}\right)(L_{2,xx} - L_{2,yy}) + \tau_L^2\,\mathrm{Ast}(\mathbf{L}_2)^2\left(S_{M,L1,xx} - S_{M,L1,yy}\right)}{2\tau_L^2\,\mathrm{Ast}(\mathbf{L}_2)^2}$$

$$\sigma = \frac{2\left(-1 + \sqrt{\bar{b}^2 - 4\bar{a}\bar{c}}\right)L_{2,xy} + \tau_L^2\,\mathrm{Ast}(\mathbf{L}_2)^2\,S_{M,L1,xy}}{2\tau_L^2\,\mathrm{Ast}(\mathbf{L}_2)^2}$$

(6a)

Auch dies lässt sich für den Fall einer rotationssymmetrischen Linsenrückfläche vereinfachen:

$$D_{LR} = L_2 + \frac{\bar{L}_{1,mess} + \bar{S}_{M,L1}}{1 - \tau_L \cdot \left(\bar{L}_{1,mess} + \bar{S}_{M,L1}\right)}$$

$$\mathbf{L}_1 = \left(\bar{L}_{1,mess} + \bar{S}_{M,L1}\right) \cdot 1 - \frac{\mathbf{S}_M + \mathbf{C}}{1 - \tau_{CL}(\mathbf{S}_M + \mathbf{C})}$$

(7)

wobei

$$\overline{L}_{1,mess} = \frac{D_{LR} - L_2}{1 + \tau_L \cdot \left( D_{LR} - L_2 \right)} - \overline{S}_{M,L1}$$

mit

$$\overline{S}_{M,L1} = \frac{S_{M,L1,xx} + S_{M,L1,yy}}{2}$$

Damit sind die einzelnen Elemente des Augenmodells vollständig berechenbar.

Bei den gegebenen (d.h. gemessenen bzw. angenommenen) Größen kann es sich außer um einen Hauptschnitt mit gegebener Winkelstellung bzw. der mittleren Krümmung auch um andere Parameter wie den stärksten Hauptschnitt, den schwächsten Hauptschnitt, den Zylinder und die Achslage handeln. Das Vorgehen ist in diesen Fällen analog zu den geschilderten Fällen.

Da inzwischen auch die HOA des Auges bei der Optimierung von Brillengläsern berücksichtigt werden, ist es vorteilhaft, bei der Belegung des Augenmodells auch die HOA der Hornhaut bzw. der Linse zu betrachten. Bei der Wahl von HOA für die Linse gilt generell, dass der Linsenvorder- bzw. Rückfläche HOA zugeordnet werden können, die auch den Brechungsindexverlauf innerhalb der Linse abbilden können.

Vorzugsweise wird der bisher dargestellte Formalismus insbesondere hinsichtlich der genannten Schritte 1 bis 6 auf die Mitbehandlung der HOA erweitert, indem außer den in Schritt 1 bis 6 explizit angegebenen Formeln für die Vergenzmatrizen die Berechnungsverfahren aus den Publikationen von G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" angewendet werden.

Generell ist das Vorgehen in Bezug auf das Abzählen von Freiheitsgraden sehr ähnlich wie oben ausgeführt. Liegen zur brechenden Fläche $C$ der Hornhaut und zur austretenden Wellenfront $S_M$ außer Daten zu Fehlern 2. Ordnung auch Daten über deren HOA vor (entweder aus Messungen oder aus sinnvollen Annahmen), dann kann auch die Wellenfront $S_{L1}$ mit entsprechend vielen HOA rechnerisch bestimmt werden. Dies gilt unabhängig von der Darstellungsform der HOA. Besonders bevorzugt ist allerdings die Taylorreihe, denn in dieser Form gilt exakt die Aussage: Sind zu beiden Flächen $C$ und $S_M$ HOA-Koeffizienten bis zur Ordnung n gegeben, dann lassen sich auch die entsprechenden HOA-Koeffizienten für $S_{L1}$ bis zur Ordnung $n$ daraus rechnerisch bestimmen. Weiterhin bevorzugt ist die Zernike-Basis, denn auch hier gilt eine ähnliche Aussage. Exakt ist diese allerdings nur dann, wenn alle Zernike-Koeffizienten mit einer Ordnung > $n$ verschwinden.

Vorzugsweise wird (vorab) eine Ordnung $n$ festgelegt, bis zu der alle beteiligten Flächen und Wellenfronten behandelt werden sollen. Unabhängig von der Darstellung der HOA besitzen die Wellenfronten oder Flächen dann außer den drei Komponenten für die Fehler 2.Ordnung noch $N$ Komponenten für die HOA, wobei $N$ von $n$ und i.a. von der Darstellungsform der HOA abhängt (In der Taylor- und Zernike-Zerlegung gilt $N = (n + 1)(\pi + 2)/2 - 6$ )

Entsprechend besitzt dann auch die Anpassungsbedingung anhand einer gemessenen Wellenfront, z.B. $S_{M,L1}$, nicht mehr nur die oben beschriebenen drei Komponenten, sondern insgesamt maximal $N + 3$ Komponenten. Diesen stehen dann entsprechend 3 (N + 3) + 3 = 3N + 12 Parameter gegenüber (nämlich die drei Längenparameter $d_{CL}$, $d_L$ und $d_{LR}$ (bzw. $D_{LR}$) sowie je $N + 3$ Komponenten von der Cornea $C$ und den Linsenflächen $L_1$ und $L_2$). Das bedeutet, es gilt:

$$df_n = df_n(i) + df_n(ii) + df_n(iii)$$
$$= 3N + 12$$

mit $df_n(iii) = N + 3$. Werden wieder bevorzugt die Vorderkammertiefe $d_{CL}$ und die Cornea $C$ gemessen, gilt $df_n(i) = N + 4$ und folglich $df_n(ii) = N + 5$, entsprechend der Situation $df_n = (N + 4) + (N + 5) + (N + 3)$.

Das weitere Vorgehen kann ganz analog zum oben beschriebenen durchgeführt werden.

Grundsätzlich können durch geeignete Messvorrichtungen mit einer Aberrometrieeinheit die HOA der Abbildung des Auges auf die Netzhaut in Transmission erfasst werden. Andererseits können durch eine Topographieeinheit die HOA der Hornhaut-Oberfläche in Reflexion gemessen werden. Damit stehen dann sowohl Daten der austretenden Wellenfront $S_M$ als auch eine Beschreibung der brechenden Fläche $C$ der Hornhaut, inklusive der HOA bis zu einer bestimmten Ordnung n zur Verfügung.

Im Falle einer Messung der Wellenfront $S_M$ auch für HOA liefert diese $df_n(iii) = N + 3$ Bedingungen zur Parameterberechnung. Wird wieder bevorzugt, außer der Cornea $C$ auch die Vorderkammertiefe $d_{CL}$, gemessen, gilt $df_n(i)$ = $N$ + 4 und folglich ist $df_n(ii)$ = $N$ + 5, entsprechend der Situation $df_n$ = $(N + 4) + (N + 5) + (N + 3)$

In einem solchen Fall können bei der Belegung des Modells die HOA der Linse so gewählt werden, dass bei der Propagation einer von einem Punkt der Netzhaut ausgehenden Wellenfront gemäß den Schritten 1 bis 6 in umgekehrter Reihenfolge die gemessene Wellenfront entsteht. Sind die Parameter des Augenmodell dann belegt, kann die Propagation dieser von einem Punkt der Netzhaut ausgehenden Wellenfront bis hin zur Bewertungsfläche (gemäß zumindest einiger der Schritte 1 bis 6 in umgekehrter Reihenfolge) zur Referenzwellenfront führen, die dann für einen Vergleich mit der von einem Objekt ausgehenden Wellenfront herangezogen wird.

Grundsätzlich kann analog zu dem oben mit Bezug auf WO 2013/104548 A1 beschriebenen Verfahren bei der Anpassung von $\mathbf{L}_1$ vorgegangen werden, wobei die beiden Längenparameter $d_L$ und $d_{LR}$ (bzw. $D_{LR}$) a priori festgelegt werden. Der einzige Unterschied ist jetzt, dass die Linsenvorderfläche $\mathbf{L}_1$ inklusive ihrer $N$ HOA-Parameter bis zur Ordnung $n$ an die Messungen angepasst werden können, entsprechend $df_n(iii)$ = $N + 3$ . Die durch Mangel an Messwerten unbekannte Linsenrückfläche $\mathbf{L}_2$ wird vorzugsweise inklusive der $N$ HOA-Parameter bis zur Ordnung $n$ vorab festgelegt (z.B. durch Literaturwerte über das Durchschnitts-Auge der Bevölkerung), entsprechend $df_n(ii)$ = $N + 5$. Dies geschieht insbesondere dadurch, dass man zum einen die gemessene Wellenfront $S_M$ mittels der Schritte 1,2 durch die ebenfalls gemessene Hornhaut $C$ hindurch rechnet und bis zur objektseitigen Seite der Linsenvorderfläche $L_1$ propagiert. Zum anderen rechnet man von einer gedachten punktförmigen Lichtquelle auf der Netzhaut eine Kugelwelle mittels der rückwärts durchlaufenen Schritte 6,5,4 von hinten nach vorne, indem man diese Kugelwelle an der zuvor festgelegten Linsenrückfläche $L_2$ bricht und die dann erhaltene Wellenfront von der Linsenrückfläche bis zur bildseitigen Seite der Linsenvorderfläche $L_1$ propagiert. Die so bestimmten zwei Wellenfronten $S_{L1}$ und $S'_{L1}$, die objektseitig bzw. bildseitig der Linsenvorderfläche liegen, besitzen im Allgemeinen sowohl Abbildungsfehler niedriger Ordnung als auch HOA, deren Werte sich jedoch zwischen den beiden Wellenfronten unterscheiden. Da die beiden Wellenfronten in ein- und demselben Messstrahlengang vorkommen und daher über den noch fehlenden Schritt 3 zusammenhängen müssen, kann aus dieser Differenz auf eindeutige Weise bis zur Ordnung $n$ auf die brechende Linsenvorderfläche $\mathbf{L}_1$ rückgeschlossen werden, und zwar beispielsweise durch die aus G. Esser et al.: "Derivation of the refraction equations for higher order aberrations of local wavefronts at oblique incidence", JOSA A, Vol. 27, No. 2 (2010) und aus G. Esser et al.: "Derivation of the propagation equations for higher order aberrations of local wavefronts, JOSA A, Vol. 28, No. 11 (2011)" bekannten Berechnungsmethoden.

Andererseits ist es auch möglich, analog zu dem oben mit Bezug auf WO 2013/104548 A1 beschriebenen Verfahren bei der Anpassung von $\mathbf{L}_2$ vorzugehen, wobei wiederum die beiden Längenparameter $d_L$ und $d_{LR}$ (bzw. $D_{LR}$) a priori festgelegt werden. Jetzt wird die Linsenrückfläche $\mathbf{L}_2$ inklusive ihrer HOA bis zur Ordnung $n$ an die Messungen angepasst, nachdem die Linsenvorderfläche $\mathbf{L}_1$ festgelegt worden ist. Ein Unterschied zur Anpassung von $\mathbf{L}_1$ besteht insbesondere darin, dass die gemessene Wellenfront $S_M$ den Schritten 1,2,3,4 unterzogen wird und die angenommene Wellenfront aus der punktförmigen Lichtquelle nur dem Schritt 6, und dass der zur Anpassung der Linsenrückfläche $\mathbf{L}_2$ zu erfolgende fehlende Schritt jetzt Schritt 5 ist.

Zur Durchrechnung bedient man sich dabei beispielsweise der in G. Esser et al.: "Derivation of the refraction equations for higher order aberrations of local wavefronts at oblique incidence", JOSA A, Vol. 27, No. 2 (2010) und in G. Esser et al.: "Derivation of the propagation equations for higher order aberrations of local wavefronts, JOSA A, Vol. 28, No. 11 (2011)" beschriebenen Formalismen für die Brechungs- bzw. Propagationsschritte. Insbesondere ist es sinnvoll, sich von den Abbildungsfehlern der niedrigsten Ordnung zur höchsten interessierenden Ordnung (typischerweise sechste) vorzuarbeiten.

Zur Verwendung der besagten Formalismen ist es vorteilhaft, die Wellenfronten bzw. Flächen durch die lokale Ableitung der Pfeilhöhe in Richtung der zur Ausbreitungsrichtung senkrechten Ebenen zu beschreiben. Jede Fläche oder Wellenfront, die nicht in dieser Form vorliegt, wird vorzugsweise zunächst auf diese Form gebracht. Dies kann beispielsweise durch Transformation von einer ZernikeDarstellung auf die Darstellung durch lokale Ableitungen geschehen, oder durch einen noch vorangehenden Fit einer Pfeilhöhendarstellung. Eine geeignete technische Darstellungsform von Flächen durch Taylorkoeffizienten ist beispielsweise in WO 2013/104548 A1 beschrieben.

Natürlich können analog zum obigen Vorgehen die Abweichungen (einschließlich den Abbildungsfehlern zweiter Ordnung) auch auf die Linsenvorder- und die Linsenrückfläche verteilt werden.

In einer bevorzugten Ausführungsform wird vorgeschlagen, dass zumindest einer der Längenparameter $d_L$ und $d_{LR}$ weder a-priori vorgegeben noch individuell gemessen, sondern anhand der individuellen Refraktionsdaten und der sonstigen (vorab) festgelegten Daten berechnet wird. Dazu wird insbesondere für einen der Freiheitsgrade der Linsenflächen $\mathbf{L}_1$ oder $\mathbf{L}_2$ zumindest ein Messwert oder eine Annahme bereitgestellt. Ist dies z.B. ein Messwert für die Krümmung von $\mathbf{L}_1$ in einem Normalschnitt, dann kann insbesondere $d_{LR}$ (bzw. $D_{LR}$) daraus durch Berechnung bestimmt werden.

Bezieht sich die Angabe in den Vergenzmatrizen auf die lokale Krümmung (dies entspricht der Angabe der HOA als Koeffizienten einer Zerlegung nach Taylor), werden dazu zuerst wie in oben bereits beschrieben $D_{LR}$ und die fehlenden Parameter der Linse bestimmt. Im Anschluss daran können dann mit dem Formalismus aus G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" schrittweise die HOA der Linse ausgehend von der zweiten bis zur n-ten Ordnung aufgebaut werden.

Werden dagegen die mittlere Krümmung über eine bestimmte Pupille verwendet, was beispielsweise bei der Darstellung nach Zernike der Fall ist, ist der Freiheitsgrad $D_{LR}$ ebenso festgelegt. In diesem Formalismus wäre aufgrund der Abhängigkeiten ein iteratives Vorgehen notwendig. Dies lässt sich jedoch durch eine Umrechnung zwischen den beiden Notationen vor Beginn der Berechnung vermeiden.

Grundsätzlich können durch geeignete Messvorrichtungen mit einer Aberrometrieeinheit die HOA der Abbildung des Auges auf die Netzhaut in Transmission erfasst werden. Allerdings sind solche Aberrometrieeinheiten zur Erfassung von HOA recht aufwendig und stehen nicht jedem Optiker zur Verfügung. Oft ist es mit weniger Aufwand allerdings möglich, durch eine Topographieeinheit die HOA der Hornhaut-Oberfläche in Reflexion zu messen. Damit stehen zwar noch keine Daten der austretenden Wellenfront $S_M$ aber zumindest eine Beschreibung der brechenden Fläche C der Hornhaut, inklusive der HOA bis zu einer bestimmten Ordnung n zur Verfügung.

Die Erfindung bietet die Möglichkeit, das individuelle Augenmodell zu nutzen, wenn zwar individuelle Messungen zu den HOA der Hornhaut aber keine individuellen Messungen zu den HOA des Auges vorliegen. In einer bevorzugten Implementierung wird dabei außer der Cornea $C$ auch die Vorderkammertiefe $d_{CL}$ gemessen, d.h. es gilt $df_n(i) = N + 4$. Bei Verwendung eines Autorefraktometers (d.h. keine Messung der HOA) an Stelle eines Aberrometers (auch in Kombination mit einer subjektiven Refraktion) oder der alleinigen Verwendung einer subjektiven Refraktion ohne Einsatz eines Aberrometers oder Autorefraktometers ist zwar die Vergenzmatrix $S_M$ der LOA bekannt, aber darüber hinaus liegen keine individuellen Informationen über die HOA der (Mess-Strahlengangs-)Wellenfront $S_M$ des gesamten Auges vor. Das bedeutet, genau wie beim Fall ohne HOA nur $df_n(iii) = 3$ statt $df_n(iii) = N + 3$ Berechnungsbedingungen vorliegen. Wenn man das Modell bis zur Ordnung n vollständig belegen möchte, legt man dann vorzugsweise entsprechend $df_n(ii) = 2N + 5$ statt $df_n(ii) = N + 5$ Parameter a priori fest. Dabei wird wieder bevorzugt der Fall betrachtet, dass sowohl $d_L$ als auch $d_{LR}$ zu den a priori festgelegten Parametern gehören. Damit lässt sich das Modell auf unterschiedliche Weise mit den weiteren Parametern belegen und für die Berechnung und Optimierung eines Brillenglases nutzen.

Insbesondere lässt sich dieser Fall genauso behandeln wie oben bei Vorliegen von gemessenen HOA des Auges beschrieben, wenn man Annahmen über die HOA des Auges trifft. Ein Beispiel hierfür sind an Hand eines Probandenkollektivs ermittelte oder modellbasierte Werte. Bevorzugt wird dabei eine verbleibende sphärische Aberration angenommen, da insbesondere aus T. O. Salmon und C. van de Pol: Normal-eye Zernike coefficients and root-mean-square wavefront errors, J Cataract Refract Surg, Vol. 32, Seiten 2064-2074 (2006) und aus J. Porter et al.: Monochromatic aberrations of the human eye in a large population, JOSA A, Vol. 18, No. 8 (2001) bekannt ist, dass diese im Mittel über die Population deutlich von Null verschieden ist. Die Berechnung der HOA der Linse erfolgt dann ganz analog zu dem oben beschriebenen Vorgehen mit dem einzigen Unterschied, dass die HOA-Werte für $S_M$ nicht einer individuellen Messung entnommen, sondern auf die oben genannten Annahmen gestützt werden.

Trifft man alternativ geeignete Annahmen über die HOA der Linse, d.h. legt man die HOA beider Linsenflächen $L_1$ und $L_2$ a priori fest, können beispielsweise mit Hilfe der Algorithmen aus G. Esser et al.: "Derivation of the refraction equations for higher order aberrations of local wavefronts at oblique incidence", JOSA A, Vol. 27, No. 2 (2010) und von G. Esser et al.: "Derivation of the propagation equations for higher order aberrations of local wavefronts, JOSA A, Vol. 28, No. 11 (2011)" die HOA der Wellenfront $S_M$ bis zur Ordnung n berechnet werden, indem man die Schritte 6,5,4,3,2,1 von der Netzhaut zur Cornea rückwärts durchläuft. Dabei gehen in die Berechnung von $S_M$ insbesondere auch die a priori festgelegten $d_L$ und $d_{LR}$ ein.

Für die LOA der Linsenflächen werden dabei keine über obigen Ausführungen hinausgehenden a priori-Festlegungen getroffen, da die LOA der Wellenfront $S_M$, z.B. als gemessene Vergenzmatrix $S_M$ , aus der subjektiven Refraktion, der Autorefraktormessung oder einer Kombination daraus vorliegen.

Ein bevorzugter Fall ist hierbei, dass die HOA der Linsenflächen in der verwendeten Basis gleich Null gesetzt werden. Besonders bevorzugt wird diese Annahme in Bezug auf die Taylor-Basis getroffen. Weiterhin bevorzugt wird diese Annahme in Bezug auf die Zernike-Basis getroffen. Zwar sind in keiner Basis die HOA von $S_M$ ein direktes Abbild der HOA von $C$, weil die beteiligten Propagationen in jedem Fall auch HOA einführen, der Vorteil verschwindender HOA der Linsenflächen besteht aber in der Verminderung des Rechenaufwandes aufgrund vieler verschwindender Terme.

Alternativ können auch modellbasierte Werte für die HOA der Linsenflächen gewählt werden. Dies gilt insbesondere für sphärische Aberrationen, da insbesondere aus T. O. Salmon und C. van de Pol: Normal-eye Zernike coefficients and root-mean-square wavefront errors, J Cataract Refract Surg, Vol. 32, Seiten 2064-2074 (2006) und aus J. Porter et al.: Monochromatic aberrations of the human eye in a large population, JOSA A, Vol. 18, No. 8 (2001) bekannt ist, dass die sphärische Aberration der Linse im Mittel über die Population deutlich von Null verschieden ist. Dabei können diese unabhängig von den gemessenen Daten oder in Abhängigkeit von gemessenen Daten (z.B. Refraktionswerte, sphärische Aberration der Hornhaut) gewählt werden.

Selbst wenn weder ein Topograph noch ein Aberrometer verwendet werden, also keine individuellen Messdaten zu HOAs vorliegen, können dennoch modellbasierte Annahmen über die HOA der Hornhaut, der Linse bzw. das Auge getroffen und bei der Belegung des Augenmodells verwendet werden. Die angenommen Werte können dabei auch an Hand entsprechender Modelle in Abhängigkeit von gemessenen Daten (z.B. Refraktionswerte, Ergebnisse der Topometrie- oder Autofraktometermessung) gewählt werden. Beispiele für die genaue Berechnung wurden bereits weiter oben beschrieben, wobei an Stelle der gemessenen Werte für die HOAs die entsprechenden Annahmen treten. Dies gilt auch wieder insbesondere für sphärische Aberrationen, da diese im Mittel über die Population deutlich von Null verschieden ist. Dabei kann diese unabhängig von den gemessenen Daten oder in Abhängigkeit von gemessenen Daten (z.B. Refraktionswerte, Ergebnisse der Topometrie- oder Autorefraktometermessung) gewählt und der Hornhaut, einer der beiden Linsenflächen oder Kombinationen zugeordnet werden.

Die vorliegende Erfindung bietet die Möglichkeit, durch Messungen von bzw. Annahmen über $L1$ und $L_2$ auf $S_M$ zu schließen. Man erhält also ohne aberrometrische Messungen sinnvolle Werte für die HOA von $S_M$. Dazu müssen auch keine genaue Kenntnisse über die Längenparameter $d_L$ und $d_{LR}$ (bzw. $D_{LR}$) vorliegen, so dass sich der Formalismus auch ohne die in Abschnitt 3 beschriebene Berechnung von $d_{LR}$ nutzen lässt. Im Gegensatz zu den Fehlern zweiter Ordnung der Wellenfront $S_M$ hängen die HOA von $S_M$ nämlich nur so schwach von den Längenparametern $d_L$ und $d_{LR}$ (bzw. $D_{LR}$) ab, dass zur Anpassung der HOA von $S_M$ die Wahl der a priori festzulegenden Werte für $d_L$ und $d_{LR}$ - im Rahmen des physiologisch sinnvollen Bereichs - nur wenig Einfluss hat und folglich auch Standardparameter verwendet werden können.

Eine Anwendung dieser Methode liegt darin, auch ohne individuelle aberrometrischen Messungen (z.B. auf Basis von Topographiemessungen) Brillenglasoptimierungen unter Berücksichtigung der HOA des Augen wie der DNEye Optimierung vorgenommen werden können.

Die Auswertung der Aberrationen während des Berechnungs- bzw. Optimierungsverfahrens kann an unterschiedlichen Stellen im Strahlverlauf vorgenommen werden, d.h. die Bewertungsfläche kann an unterschiedlichen Positionen vorgesehen werden. Anstatt an der Netzhaut oder an der Linsenrückfläche kann eine Bewertung der abbildenden Wellenfront auch schon an einer weiter vorne im Modellauge liegenden Fläche erfolgen. Dazu wird innerhalb des Modellauges eine Referenzwellenfront **R** definiert, die dann beispielsweise bei der Glasoptimierung verwendet wird. Diese Referenzwellenfront hat dabei die Eigenschaft, dass sie bei weiterer Propagation durch das Auge bis zur Retina zu einem punktförmigen Bild führt. Entsprechend kann die Referenzwellenfront durch Rückpropagation einer Wellenfront, die auf der Netzhaut in einen Punkt zusammenläuft, von der Netzhaut bis zur Position der Referenzwellenfront bestimmt werden. Da beispielsweise die gemessene Wellenfront $S_M$ genau die Wellenfront ist, die aus einer punktförmigen Lichtquelle auf der Netzhaut hervorgeht, kann stattdessen auch diese ins Augeninnere bis zur Position der Referenzwellenfront propagiert werden.

Mathematisch betrachtet sind beide Vorgehensweisen äquivalent und führen auf die gleichen Formeln für die Referenzwellenfront. Im Folgenden wird zur Ableitung der entsprechenden Referenzwellenfronten jeweils der Weg gewählt, der mit weniger Propagationsschritten auskommt und eine einfachere Darstellung ermöglicht. Nachfolgend wird beispielhaft nur die Behandlung der Komponenten des Defokus und des Astigmatismus beschrieben. Eine Ausdehnung auf HOA und die Verwendung der subjektiven Refraktion ist jedoch ebenfalls möglich und vorteilhaft.

Bei der Berücksichtigung von HOA können diese analog zur Berechnung der HOA gemäß untenstehender Ausführungen durch Brechung (G. Esser et al.: "Derivation of the refraction equations for higher order aberrations of local wavefronts at oblique incidence", JOSA A, Vol. 27, No. 2 (2010)) und Propagation (G. Esser et al.: "Derivation of the propagation equations for higher order aberrations of local wavefronts", JOSA A, Vol. 28, No. 11 (2011)) erfolgen.

Da die Wellenfrontpropagation ein nichtlinearer Vorgang ist, führt eine Brillenglasoptimierung, die eine abbildende Wellenfront durch Vergleich mit einer Referenzwellenfront bewertet, im allgemeinen zu verschiedenen Ergebnissen, je nachdem, an welcher Fläche innerhalb des Auges dieser Vergleich stattfindet.

In einer bevorzugten Ausführungsform wird lediglich auf den allerletzten Schritt (insbesondere Schritt 6b), also die Propagation von der AP zur Netzhaut verzichtet. Die einfallende Wellenfront wird also nach der Brechung an der Linsenrückfläche nur bis zur AP simuliert (also Berechnung von $S_{AP}$ gemäß eingangs genanntem Schritt 6a) und dort mit einer Referenzwellenfront $R_{AP}$ verglichen. Diese zeichnet sich dabei dadurch aus, dass sie bei der Propagation zur Netzhaut dort ein punktförmiges Bild ergibt. Gemäß dem oben Gesagten ist die Vergenzmatrix dieser Wellenfront gerade

$$\mathbf{R}_{AP} = \mathbf{D}_{AP} = \mathbf{D}_{LR}^{(b)} = \frac{1}{\tau_{LR}^{(b)}}\mathbf{1} = \frac{1}{\tau_{LR} - \tau_{LR}^{(a)}}\mathbf{1} = \frac{1}{1/D_{LR} - d_{LR}^{(a)}/n_{LR}}\mathbf{1}$$

mit dem aus Gleichung (2) bzw. (3) bestimmten $D_{LR}$ sowie dem negativen (akkommodationsabhängigen) Wert $d_{LR}^{(a)} < 0$, dessen Betrag den Abstand zwischen der Linsenrückfläche und der AP beschriebt.

In einer weiterhin bevorzugten Ausführungsform wird außerdem auf den vorletzten Schritt, insgesamt also die Propagation von der Linsenrückfläche zur Netzhaut verzichtet. Die einfallende Wellenfront wird also nur bis nach der Brechung an der Linsenrückfläche simuliert (also Berechnung von $S'_{L2}$ gemäß eingangs genanntem Schritt 5) und dort mit einer Referenzwellenfront $R'_{L2}$ verglichen. Diese zeichnet sich dabei dadurch aus, dass sie bei der Propagation zur Netzhaut dort ein punktförmiges Bild ergibt. Gemäß dem oben Gesagten ist die Vergenzmatrix dieser Wellenfront gerade

$$\mathbf{R}'_{L2} = \mathbf{D}'_{L2} = D_{LR} \cdot \mathbf{1}$$

mit dem aus Gleichung (2) bzw. (3) bestimmten $D_{LR}$.

Eine weitere Vereinfachung ergibt sich, wenn man den Vergleich vor die Brechung durch die Linsenrückfläche legt. In diesem Fall muss die einfallende Wellenfront nur bis $S_{L2}$ gemäß obigem Schritt 4 simuliert, also berechnet werden. Dazu wird analog zu $S'_{L2}$ eine Referenzwellenfront $R_{L2}$ definiert, die nach der Brechung an der Linsenrückfläche und der Propagation zur Netzhaut dort ein punktförmiges Bild ergibt. Diese bestimmt sich zu

$$\mathbf{R}_{L2} = \mathbf{R}'_{L2} - \mathbf{L}_2 = D_{LR} \cdot \mathbf{1} - \mathbf{L}_2$$

mit dem aus Gleichung (2) bzw. (3) bestimmten $D_{LR}$ und dem aus der Literatur bzw. aus Messungen bekanntem $L_2$.

Im Fall einer rotationssymmetrischen Linsenrückfläche vereinfacht sich dies zu

$$\mathbf{R}_{L2} = \left( D_{LR} - L_{2,xx} \right) \cdot \mathbf{1}$$

Insbesondere soweit die Linsendicke ebenfalls der Literatur entnommen wird, bietet es sich in einer weiteren bevorzugten Ausführungsform an, als nächsten Vereinfachungsschritt auf die Propagation durch die Linse zu verzichten und den Vergleich hinter der Brechung durch die Linsenvorderfläche auszuführen. In Weiterführung des oben Gesagten verwendet man dazu vorzugsweise eine Referenzwellenfront $R'_{L1}$, die aus $R_{L2}$ durch Rückwärtspropagation um die Linsendicke entsteht und folgende Vergenzmatrix besitzt:

$$\mathbf{R}'_{L1} = \mathbf{R}_{L2} / (\mathbf{1} + \tau_L \mathbf{R}_{L2})$$

mit dem aus Gleichung (2) bzw. (3) bestimmten $D_{LR}$ und dem aus der Literatur bzw. aus Messungen bekannten $\tau_L = d_L/n_L$ sowie der aus Gl.(6) bzw. (7) bestimmten Vergenzmatrix $\mathbf{R}_{L2}$.

Im Fall einer rotationssymmetrischen Linsenrückfläche vereinfacht sich dies zu

$$\mathbf{R}'_{L1} = \frac{D_{LR} - L_{2,xx}}{1 + \tau_L \cdot \left( D_{LR} - L_{2,xx} \right)} \cdot \mathbf{1}$$

Wie bei obigen Modellen gilt auch hier, dass selbst wenn die Betrachtung vor den letzten Schritten stattfindet und - je nach Notation - die Größe $D_{LR}$ nicht explizit vorkommt, diese Größe dennoch zusammen mit $d_L$ und $L_2$ zumindest implizit eingeht, da sie zusammen die Verteilung der Wirkung $L_1$ in der Linsenvorderfläche steuern.

Eine noch weitere Vereinfachung ergibt sich, wenn man den Vergleich vor die Brechung durch die Linsenvorderfläche legt. In diesem Fall muss die einfallende Wellenfront nur bis $S_{L1}$ gemäß Schritt 2 simuliert werden. Dazu wird analog zu $R'_{L1}$ eine Referenzwellenfront $R_{L1}$ definiert, die nach der Brechung an der Linsenvorderfläche und den weiteren Schritten an der Netzhaut zu einem Punkt konvergiert. Diese kann man entweder durch die Brechung von $R'_{L1}$ an $L_1$ berechnen oder direkt aus der Brechung der gemessenen Wellenfront $S_M$ an der Hornhaut $C$ und einer anschließenden Propagation um $d_{CL}$ bestimmen. In beiden Fällen erhält man

$$\mathbf{R}_{L1} = \frac{\mathbf{S}_M + \mathbf{C}}{1 - \tau_{CL} \cdot \left( \mathbf{S}_M + \mathbf{C} \right)}$$

Darin gehen die Größen $D_{LR}$, $d_L$ und $L_2$ nun nicht mehr ein, es ist also ausreichend, $S_M$, $C$ und $d_{CL}$ zu kennen.

Mit relativ wenig Rechenaufwand verbunden ist eine Ausführungsform, in der der Vergleich nach der Brechung an der Hornhaut durchgeführt wird. In diesem Fall werden nur noch $S_M$ und $C$ berücksichtigt:

$$\mathbf{R}'_C = \mathbf{S}_M + \mathbf{C}$$

Eine weitere sehr effiziente Möglichkeit ist der Positionierung der Bewertungsfläche an der Austrittspupille des Modellauges. Diese liegt vorzugsweise vor der Linsenrückfläche.

Weitere Aspekte

In den folgenden Absätzen werden, soweit nicht ausdrücklich etwas anderes erwähnt ist, Aspekte beschrieben, die sowohl für den ersten als auch für den zweiten Ansatz der Erfindung relevant sind:
Insbesondere werden nachfolgend zusammenfassend nochmals beispielhaft kommerziell erhältliche Geräte genannt, mit denen für die Erfindung notwendige oder bevorzugte Parametermessungen durchgeführt werden können. Sämtliche hier aufgeführten Geräte sind beispielsweise auch in M. Kaschke et al. "Optical Devices in Ophthalmology and Optometry", Wiley-VCH (2014) beschrieben:

- Form der Hornhautvorderfläche: Die Form der Hornhautvorderfläche kann mit Keratographen (z.B. Placido-Disk Keratograph ATLAS 9000 von Zeiss, Small-Target Keratograph E300 von Medmont und Placido-Disk Einheit des Galilei G2 von Ziemer) ermittelt werden. In den Fällen, in denen lediglich die Krümmungen ermittelt und genutzt werden, ist auch der Einsatz von Keratometern (z.B. manuelles Helmholtz-Littmann Keratometer von Zeiss, manuelles Javal-Schiötz Keratometer von Haag-Streit und automatische elektrooptische Keratometrieeinheit des IOL Masters von Zeiss) möglich.

- Form der Linsenvorder- und Rückfläche: Die Form der Linsenflächen kann in einem Schnitt oder dreidimensional mit Scheimpflugkameras (z.B. Pantacam von Oculus, SL-45 von Topcon und Galilei G2 von Ziemer) und OCTs (z.B. IOL Master von 500 von Zeiss, SL-OCT von Heidelberg und Visante OCT von Zeiss) gemessen werden.

- Abstand zwischen den beschriebenen Flächen: Abstände zwischen den drei genannten Flächen können sowohl mit einigen der oben genannten Scheimpflugkameras und OCTs als auch mit dem Lenstar LS900 von Haag-Streit gemessen werden. Zwar könnten einige dieser Geräte auch genutzt werden, um den Abstand zwischen diesen Flächen und der Netzhaut zu messen. Allerdings sind solche Messungen meist sehr aufwendig und können im Rahmen der vorliegenden Erfindung gerade vermieden werden. Hierzu wird beispielhaft auf R. B. Rabbetts "Bennett & Rabbetts' Clinical Visual Optics", Butterworth Heinemann Elsevier Health Sciences (2007) verwiesen.

- Brechungsindices der beteiligten Medien: Auf eine Nennung von Geräten, mit denen die Brechungsindices der beteiligten Medien gemessen werden können, kann hier verzichtet werden, da diese Werte vorzugsweise der Literatur entnommen werden. Hierzu wird beispielhaft auf R. B. Rabbetts "Bennett & Rabbetts' Clinical Visual Optics", Butterworth Heinemann Elsevier Health Sciences (2007) verwiesen.

- Abbildungsfehler höherer bzw. niedriger Ordnung des Auges: Abbildungsfehler des Auges können mit Aberrometern (z.B. iProfiler von Zeiss und KR-1W von Topcon auf Basis von Schack-Hartmann Sensoren sowie OPD-Scan III von Nidek auf Basis der dynamischen Siaskopie) gemessen werden. Bei einer Betrachtung der Abbildungsfehler niedriger Ordnung ist die Verwendung von Autorefraktometern (z.B. RM-8900 von Topcon und KW-2000 von Kowa) ausreichend.

**Bezugszeichenliste**

10    Hauptstrahl
12    Auge
14    erste Fläche des Brillenglases (Vorderfläche)
16    zweite Fläche des Brillenglases (Rückfläche)
18    Hornhautvorderfläche
20    Augenlinse

Die vorliegende Offenbarung betrifft ferner die folgenden zur Erfindung gehörenden Aspekte:

Aspekt 1: Computerimplementiertes Verfahren zum Ermitteln relevanter individueller Parameter zumindest eines Auges eines Brillenträgers für die Berechnung oder Optimierung eines Brillenglases für das zumindest eine Auge des Brillenträgers umfassend die Schritte:

- Bereitstellen von individuellen Refraktionsdaten des zumindest einen Auges des Brillenträgers; und
- Festlegen eines individuellen Augenmodells, in welchem zumindest

  -- eine Form einer Hornhautvorderfläche (18) eines Modellauges (12);
  -- ein Hornhaut-Linsen-Abstand;
  -- Parameter der Linse des Modellauges; und
  -- ein Linsen-Netzhaut-Abstand

derart anhand von individuellen Messwerten für das Auge des Brillenträgers und/oder von Standardwerten und/oder anhand der bereitgestellten individuellen Refraktionsdaten festlegt werden, dass das Modellauge (12) die bereitgestellten individuellen Refraktionsdaten aufweist, wobei zumindest das Festlegen des Linsen-Netzhaut-Abstands durch Berechnen erfolgt.

Aspekt 2: Verfahren nach Aspekt 1, wobei das Festlegen der Form der Hornhautvorderfläche (18) des Auges (12) anhand von individuellen Messungen zumindest teilweise entlang der Hauptschnitte der Hornhaut des zumindest einen Auges erfolgt.

Aspekt 3: Verfahren nach Aspekt 1 oder 2, wobei das Festlegen der Form der Hornhautvorderfläche (18) des Auges (12) anhand von individuellen Messungen der Hornhaut-Topographie des zumindest einen Auges erfolgt.

Aspekt 4: Verfahren nach einem der vorangegangenen Aspekte, wobei das Festlegen des Hornhaut-Linsen-Abstandes anhand von individuellen Messwerten für den Hornhaut-Linsen-Abstand erfolgt.

Aspekt 5: Verfahren nach einem der vorangegangenen Aspekte, wobei das Festlegen der Parameter der Linse des Modellauges ein Festlegen folgender Parameter umfasst:

  -- Form der Linsenvorderfläche;
  -- Linsendicke; und
  -- Form der Linsenrückfläche.

Aspekt 6: Verfahren nach Aspekt 5, wobei das Festlegen der Linsendicke und der Form der Linsenrückfläche anhand von vorgegebenen Standardwerten erfolgt, und wobei das Festlegen der Form der Linsenvorderfläche umfasst:

- Bereitstellen von Standardwerten für eine mittlere Krümmung der Linsenvorderfläche; und
- Berechnen der Form der Linsenvorderfläche unter Berücksichtigung der bereitgestellten individuellen Refraktionsdaten.

Aspekt 7: Verfahren nach Aspekt 5, wobei das Festlegen der Form der Linsenvorderfläche umfasst:

- Bereitstellen eines individuellen Messwerts einer Krümmung in einem Normalschnitt der Linsenvorderfläche.

Aspekt 8: Verfahren nach Aspekt 7, wobei das Festlegen der Linsendicke und der Form der Linsenrückfläche anhand von Standardwerten erfolgt, und wobei das Festlegen der Form der Linsenvorderfläche umfasst:

- Berechnen der Form der Linsenvorderfläche unter Berücksichtigung der bereitgestellten individuellen Refraktionsdaten.

Aspekt 9: Verfahren nach einem der Aspekte 1 bis 4, wobei das Festlegen der Parameter der Linse des Modellauges ein Festlegen einer optischen Wirkung der Linse umfasst.

Aspekt 10: Verfahren nach einem der Aspekte 1 bis 9, außerdem umfassend:

- Anzeigen des berechneten Linsen-Netzhaut-Abstands.

Aspekt 11: Verfahren nach einem der Aspekte 1 bis 10, außerdem umfassend:

- Ermitteln einer Augenlänge des Modellauges unter Berücksichtigung des berechneten Linsen-Netzhaut-Abstands; und
- Anzeigen der ermittelten Augenlänge.

Aspekt 12: Computerimplementiertes Verfahren zum Berechnen oder Optimieren eines Brillenglases für zumindest ein Auge eines Brillenträgers umfassend:

- ein Verfahren zum Ermitteln relevanter individueller Parameter des zumindest einen Auges des Brillenträgers gemäß einem der Aspekte 1 bis 11;
- Vorgeben einer ersten Fläche (14) und einer zweiten Fläche (16) für das zu berechnende bzw. optimierende Brillenglas;
- Ermitteln des Verlaufs eines Hauptstrahls (10) durch zumindest einen Durchblickspunkt (i) zumindest einer zu berechnenden oder optimierenden Fläche (14; 16) des Brillenglases in das Modellauge (12);
- Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront;
- iteratives Variieren der zumindest einen zu berechnenden oder optimierenden Fläche (14; 16) des Brillenglases bis die ausgewertete Aberration einer vorgegebenen Sollaberration entspricht.

Aspekt 13: Verfahren nach Aspekt 12, wobei die Bewertungsfläche zwischen der Hornhautvorderfläche und der Netzhaut, vorzugsweise zwischen der Linse und der Netzhaut des Modellauges liegt.

Aspekt 14: Verfahren nach Aspekt 12, wobei die Bewertungsfläche an der Austrittspupille des Modellauges liegt.

Aspekt 15: Vorrichtung zum Ermitteln relevanter individueller Parameter zumindest eines Auges eines Brillenträgers für die Berechnung oder Optimierung eines Brillenglases für das zumindest eine Auge des Brillenträgers umfassend:

- eine Datenschnittstelle zum Bereitstellen von individuellen Refraktionsdaten des zumindest einen Auges (12) des Brillenträgers; und
- ein Modellierungsmodul zum Festlegen eines individuellen Augenmodells, welches zumindest

-- eine Form einer Hornhautvorderfläche (18) eines Modellauges (12);
-- einen Hornhaut-Linsen-Abstand;
-- Parameter der Linse des Modellauges; und
-- einen Linsen-Netzhaut-Abstand;

derart anhand von individuellen Messwerten für das Auge des Brillenträgers und/oder von Standardwerten und/oder anhand der bereitgestellten individuellen Refraktionsdaten festlegt werden, dass das Modellauge (12) die bereitgestellten individuellen Refraktionsdaten aufweist, wobei zumindest das Festlegen des Linsen-Netzhaut-Abstands durch Berechnen erfolgt.

Aspekt 16: Vorrichtung nach Aspekt 15, wobei das Modellierungsmodul ausgelegt ist zum Ermitteln einer Augenlänge des Modellauges unter Berücksichtigung des berechneten Linsen-Netzhaut-Abstands.

Aspekt 17: Vorrichtung nach Aspekt 15 oder 16, außerdem umfassend:

- eine Anzeigeeinrichtung zum Anzeigen des berechneten Linsen-Netzhaut-Abstands und/oder der ermittelten Augenlänge.

Aspekt 18: Vorrichtung nach einem der Aspekte 15 bis 17, welche als Aberrometer und/oder als Topograf ausgebildet ist.

Aspekt 19: Vorrichtung zum Berechnen oder Optimieren eines Brillenglases für zumindest ein Auge eines Brillenträgers umfassend:

- eine Vorrichtung zum Ermitteln relevanter individueller Parameter des zumindest einen Auges des Brillenträgers gemäß einem der Aspekte 15 bis 18;
- eine Flächenmodelldatenbank zum Vorgeben einer ersten Fläche (14) und einer zweiten Fläche (16) für das zu berechnende bzw. optimierende Brillenglas;
- ein Hauptstrahlermittlungsmodule zum Ermitteln des Verlaufs eines Hauptstrahls (10) durch zumindest einen Durchblickspunkt (i) zumindest einer zu berechnenden oder optimierenden Fläche (14; 16) des Brillenglases in das Modellauge (12);
- ein Auswertemodul zum Auswerten einer Aberration einer entlang des Hauptstrahls aus einer auf die erste Fläche des Brillenglases auftreffenden sphärischen Wellenfront resultierenden Wellenfront an einer Bewertungsfläche im Vergleich zu einer in einem Punkt auf der Netzhaut des Augenmodells konvergierenden Wellenfront; und
- ein Optimierungsmodul iteratives Variieren der zumindest einen zu berechnenden oder optimierenden Fläche (14; 16) des Brillenglases bis die ausgewertete Aberration einer vorgegebenen Sollaberration entspricht.

Aspekt 20: Computerprogrammerzeugnis, welches Programmcode enthält, der ausgelegt ist, wenn geladen und ausgeführt auf einem Computer, ein Verfahren zum Ermitteln relevanter individueller Parameter zumindest eines Auges eines Brillenträgers gemäß einem der Aspekte 1 bis 11 und/oder ein Verfahren zum Berechnen oder Optimieren eines Brillenglases gemäß einem der Aspekte 12 bis 14 durchzuführen.

Aspekt 21: Verfahren zum Herstellen eines Brillenglases umfassend:

Berechnen oder Optimieren eines Brillenglases nach dem Verfahren zum Berechnen oder Optimieren eines Brillenglases gemäß einem der Aspekte 12 bis 14; und
Fertigen des so berechneten oder optimierten Brillenglases.

Aspekt 22: Vorrichtung zum Herstellen eines Brillenglases umfassend:

Berechnungs- oder Optimierungsmittel, welche ausgelegt sind, das Brillenglas nach einem Verfahren zum Berechnen oder Optimieren eines Brillenglases gemäß einem der Aspekte 12 bis 14 zu berechnen oder zu optimieren; und
Bearbeitungsmittel, welche ausgelegt ist, das Brillenglas gemäß dem Ergebnis der Berechnung bzw. Optimierung zu bearbeiten.

Aspekt 23: Computerimplementiertes Verfahren zum Ermitteln individueller Aberrationsdaten zumindest eines Auges eines Brillenträgers umfassend:

- Bereitstellen einer gemessenen Hornhauttopographie des zumindest einen Auges des Brillenträgers;
- Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und
- Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

Aspekt 24: Verfahren nach Aspekt 23, wobei das Ermitteln der individuellen Abbildungseigenschaften der Hornhaut des Auges ein Ermitteln von Werten der Abbildungsfehler höherer Ordnung der Hornhaut, $HOA_C$, umfasst, und wobei das Ermitteln der Abbildungsfehler des Auges ein Ermitteln der Abbildungsfehler höherer Ordnung des Auges, $HOA_{Auge}$, gemäß $HOA_{Auge} = HOA_C + \Delta HOA_{C,Auge}$ mit einer Verschiebung höherer Ordnung, $\Delta HOA_{C,Auge}$, insbesondere mit $\Delta HOA_{C,Auge} = 0$, umfasst.

Aspekt 25: Verfahren nach Aspekt 23 oder 24, wobei das Ermitteln der individuellen Abbildungseigenschaften der Hornhaut des Auges ein Ermitteln von Refraktionswerten niedriger Ordnung der Hornhaut, $LOA_C$, umfasst, und wobei das Ermitteln der Abbildungsfehler des Auges ein Ermitteln von Abbildungsfehlern niedriger Ordnung des Auges, $LOA_{Auge}$, gemäß $LOA_{Auge} = LOA_C + \Delta LOA_{C,Auge}$ mit einer Verschiebung niedriger Ordnung, $\Delta LOA_{C,Auge}$ umfasst.

Aspekt 26: Verfahren nach Aspekt 25, wobei das Ermitteln von Refraktionswerten niedriger Ordnung der Hornhaut, $LOA_C$ ein Ermitteln astigmatischer Anteile der Refraktion der Hornhaut, $LOA_{C,J0}$ und $LOA_{C,J45}$ umfasst, und wobei das Ermitteln von Abbildungsfehlern niedriger Ordnung des Auges, $LOA_{Auge}$, ein Ermitteln astigmatischer Anteile der Abbildungsfehler niedriger Ordnung des Auges, $LOA_{Auge,J0}$ und $LOA_{Auge,J45}$, gemäß $LOA_{Auge,J0} = LOA_{C,J0}$ und $LOA_{Auge,J45} = LOA_{C,J45}$ umfasst.

Aspekt 27: Verfahren nach Aspekt 25 oder 26, wobei das Ermitteln von Refraktionswerten niedriger Ordnung der Hornhaut, $LOA_C$ ein Ermitteln eines sphärischen Anteils der Refraktion der Hornhaut, $LOA_{C,M}$ umfasst, und wobei das Ermitteln von Abbildungsfehlern niedriger Ordnung des Auges, $LOA_{Auge}$, ein Ermitteln eines sphärischen Anteils der Abbildungsfehler niedriger Ordnung des Auges, $LOA_{Auge,M}$ gemäß $LOA_{Auge,M} = LOA_{C,M} - LOA_{C,M,Std}$ mit einem vorgegebenen Standardwert $LOA_{C,M,Std}$ umfasst.

Aspekt 28: Verfahren nach Aspekt 25 oder 26, wobei das Ermitteln von Refraktionswerten niedriger Ordnung der Hornhaut, $LOA_C$ ein Ermitteln eines sphärischen Anteils der Refraktion der Hornhaut, $LOA_{C,M}$ umfasst, und wobei das Ermitteln von Abbildungsfehlern niedriger Ordnung des Auges, $LOA_{Auge}$, ein Ermitteln eines sphärischen Anteils der Abbildungsfehler niedriger Ordnung des Auges, $LOA_{Auge,M}$, gemäß $LOA_{Auge,M} = LOA_{C,M} + \Delta LOA_{C,Auge,M}(LOA_{C,M})$ mit einer linearen Funktion $\Delta LOA_{C,Aug,M}(LOA_{C,M}) = \Delta LOA_{C,Auge,M}(LOA_{C,M,Std}) + \alpha(LOA_{C,M} -LOA_{C,M,Std})$ mit einem vorgegebenen Standardwert $LOA_{C,M,Std}$ und einem vorgegebenen Wert $\alpha$, vorzugsweise im Bereich $5 < \alpha < 15$, umfasst.

Aspekt 29: Verfahren nach Aspekt 23 oder 24, wobei das Ermitteln der Abbildungsfehler des Auges ein Ermitteln von Abbildungsfehlern niedriger Ordnung des Auges, $LOA_{Auge}$, anhand von individuellen Refraktionsmessungen an dem zumindest einen Auge umfasst.

Aspekt 30: Verfahren nach einem der vorangegangenen Aspekte 23 bis 29, wobei das Ermitteln von Abbildungsfehlern des Auges umfasst:

- Festlegen eines individuellen Augenmodells, in welchem eine Form einer Hornhautvorderfläche (18) eines Modellauges (12) der gemessenen Hornhauttopographie entspricht und in welchem außerdem zumindest

    -- ein Hornhaut-Linsen-Abstand;
    -- Parameter einer Linse des Modellauges; und
    -- ein Linsen-Netzhaut-Abstand

derart anhand von individuellen Messwerten für das Auge des Brillenträgers und/oder von Standardwerten und/oder anhand der ermittelten Abbildungsfehler niedriger Ordnung des Auges festgelegt werden, dass das Modellauge (12) die ermittelten Abbildungsfehler niedriger Ordnung des Auges aufweist; und
- Ermitteln einer Aberration einer nach einer Brechung an der Hornhautvorderfläche des Modellauges und einer Propagation durch das Modellauge in einem Punkt auf der Netzhaut konvergierenden Wellenfront.

Aspekt 31: Verfahren nach Aspekt 30, wobei das Festlegen der Parameter der Linse des Modellauges ein Festlegen folgender Parameter umfasst:

    -- Form der Linsenvorderfläche;
    -- Linsendicke; und
    -- Form der Linsenrückfläche.

Aspekt 32: Verfahren nach Aspekt 30 oder 31, wobei das Festlegen von Parametern der Linse des Modellauges, insbesondere das Festlegen der Linsendicke, und/oder des Linsen-Netzhaut-Abstands anhand von vorgegebenen Standardwerten erfolgt.

Aspekt 33: Verfahren nach einem der Aspekte 30 bis 32, wobei das Festlegen von Parametern der Linse des Modellauges, insbesondere der Form der Linsenvorderfläche und/oder der Linsenrückfläche anhand von vorgegebenen Standardwerten für die Abbildungsfehler höherer Ordnung der jeweiligen Fläche erfolgt.

Aspekt 34: Verfahren nach Aspekt 33, wobei die Standardwerte der Abbildungsfehler höherer Ordnung der Linsenvorderfläche und/oder der Linsenrückfläche auf null gesetzt werden.

Aspekt 35: Verfahren nach Aspekt 30 oder 31, wobei das Ermitteln der individuellen Abbildungseigenschaften der Hornhaut des Auges ein Ermitteln von Refraktionswerten niedriger Ordnung der Hornhaut, $LOA_C$, umfasst und wobei das Festlegen des Linsen-Netzhaut-Abstands und/oder von Parametern der Linse des Modellauges, insbesondere der Linsendicke und/oder der Form der Linsenvorderfläche und/oder der Linsenrückfläche, anhand der ermittelten Refraktionswerte niedriger Ordnung der Hornhaut, $LOA_C$, erfolgt.

Aspekt 36: Verfahren nach Aspekt 30 oder 31, wobei das Ermitteln der Abbildungsfehler des Auges ein Ermitteln von Abbildungsfehlern niedriger Ordnung des Auges, $LOA_{Auge}$, umfasst und wobei das Festlegen des Linsen-Netzhaut-Abstands und/oder von Parametern der Linse des Modellauges, insbesondere der Linsendicke und/oder der Form der Linsenvorderfläche und/oder der Linsenrückfläche, anhand der ermittelten Abbildungsfehler niedriger Ordnung des Auges, $LOA_{Auge}$, erfolgt.

Aspekt 37: Computerimplementiertes Verfahren zum Ermitteln optimierter sphärozylindrischer Werte für zumindest ein Auge eines Brillenträgers, wobei das Verfahren umfasst:

- Ermitteln von subjektiven sphärozylindrischen Refraktionswerten;
- Ermitteln von objektiven sphärozylindrischen Refraktionswerten, welches umfasst:

    -- Bereitstellen individueller Aberrationsdaten, welche mittels eines Verfahrens nach einem der Aspekte 23 bis 36 ermittelt wurden;
    -- Ermitteln einer Referenzwellenfront an einer Bewertungsfläche anhand der bereitgestellten individuellen Aberrationsdaten des Auges;
    -- Vorgeben einer Startvorgabe für eine zu optimierende Wellenfront, welche zu optimierende, objektive sphärozylindrische Refraktionswerte beschreibt, an der Bewertungsfläche;
    -- Ermitteln einer Differenzwellenfront aus der zu optimierenden Wellenfront und der Referenzwellenfront;
    -- Bewerten der Differenzwellenfront anhand einer vorgestimmten Metrik;
    -- Ermitteln der zu optimierenden Wellenfront derart, dass die Bewertung der Differenzwellenfront vorbestimmte Sollkriterien erfüllt; und
    -- Ermitteln der objektiven sphärozylindrischen Refraktionswerte aus der ermittelten zu optimierenden Wellenfront; und

- Ermitteln der optimierten sphärozylindrischen Werte als gewichteter Mittelwert aus den ermittelten subjektiven sphärozylindrischen Refraktionswerten und den ermittelten objektiven sphärozylindrischen Refraktionswerten.

Aspekt 38: Computerimplementiertes Verfahren zum Berechnen oder Optimieren eines Brillenglases für zumindest ein Auge eines Brillenträgers umfassend:

- Bereitstellen individueller Aberrationsdaten, welche mittels eines Verfahrens nach einem der Aspekte 23 bis 36 ermittelt wurden;
- Ermitteln einer Referenzaberration an einer Bewertungsfläche anhand der bereitgestellten individuellen Aberrationsdaten des Auges;
- Vorgeben einer ersten Fläche (14) und einer zweiten Fläche (16) für das zu berechnende bzw. optimierende Brillenglas;
- Ermitteln des Verlaufs eines Hauptstrahls (10) durch zumindest einen Durchblickspunkt (i) zumindest einer zu berechnenden oder optimierenden Fläche (14; 16) des Brillenglases;
- Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration;
- iteratives Variieren der zumindest einen zu berechnenden oder optimierenden Fläche (14; 16) des Brillenglases bis die ausgewertete Aberration einer vorgegebenen Sollaberration entspricht.

Aspekt 39: Verfahren nach Aspekt 38, wobei die Bewertungsfläche an der Scheitelpunktkugel liegt.

Aspekt 40: Computerimplementiertes Verfahren zum Berechnen oder Optimieren eines Brillenglases für zumindest ein Auge eines Brillenträgers umfassend:

- Ermitteln optimierter sphärozylindrischer Werte für zumindest ein Auge eines Brillenträgers auf Basis individueller Aberrationsdaten, welche mittels eines Verfahrens nach einem der Aspekte 23 bis 36 ermittelt wurden, insbesondere mittels eines Verfahrens nach Aspekt 37;
- Festlegen bzw. Ermitteln einer Kombination aus Vorder- und Rückfläche als Startfläche bzw. auf Basis der ermittelten optimierten sphärozylindrischen Werte; und
- vorzugsweise Modifizieren der festgelegten bzw. ermittelten Vorderfläche und/oder Rückfläche auf Basis der Basis der ermittelten optimierten sphärozylindrischen Werte.

Aspekt 41: Vorrichtung zum Ermitteln individueller Aberrationsdaten zumindest eines Auges eines Brillenträgers umfassend:

- eine Datenschnittstelle zum Bereitstellen einer gemessenen Hornhauttopographie des zumindest einen Auges des Brillenträgers;
- ein Hornhautauswertemodul zum Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und
- ein Berechnungsmodul zum Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

Aspekt 42: Vorrichtung zum Berechnen oder Optimieren eines Brillenglases für zumindest ein Auge eines Brillenträgers umfassend:

- eine Datenschnittstelle zum Bereitstellen individueller Aberrationsdaten, welche mittels eines Verfahrens nach einem der Aspekte 23 bis 36 ermittelt wurden;
- ein Modellierungsmodul zum Ermitteln einer Referenzaberration an einer Bewertungsfläche anhand der bereitgestellten individuellen Aberrationsdaten des Auges;
- eine Flächenmodelldatenbank zum Vorgeben einer ersten Fläche (14) und einer zweiten Fläche (16) für das zu berechnende bzw. optimierende Brillenglas;
- ein Hauptstrahlermittlungsmodul zum Ermitteln des Verlaufs eines Hauptstrahls (10) durch zumindest einen Durchblickspunkt (i) zumindest einer zu berechnenden oder optimierenden Fläche (14; 16) des Brillenglases;
- ein Auswertemodul zum Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration; und
- ein Optimierungsmodul zum iterativen Variieren der zumindest einen zu berechnenden oder optimierenden Fläche (14; 16) des Brillenglases bis die ausgewertete Aberration einer vorgegebenen Sollaberration entspricht.

Aspekt 43: Brillenglas, welches durch ein Verfahren gemäß einem der Aspekte 38 bis 40 und/oder mittels einer Vorrichtung gemäß Aspekt 42 berechnet oder optimiert ist.

Aspekt 44: Computerprogrammerzeugnis, welches Programmcode enthält, der ausgelegt ist, wenn geladen und ausgeführt auf einem Computer, ein Verfahren zum Ermitteln individueller Aberrationsdaten zumindest eines Auges eines Brillenträgers gemäß einem der Aspekte 23 bis 36 und/oder ein Verfahren zum Ermitteln optimierter sphärozylindrischer Werte für zumindest ein Auge eines Brillenträgers gemäß Aspekt 37 und/oder ein Verfahren zum Berechnen oder Optimieren eines Brillenglases gemäß einem der Aspekte 38 bis 40 durchzuführen.

Aspekt 45: Verfahren zum Herstellen eines Brillenglases umfassend:

Berechnen oder Optimieren eines Brillenglases nach dem Verfahren zum Berechnen oder Optimieren eines Brillenglases gemäß einem der Aspekte 38 bis 40; und
Fertigen des so berechneten oder optimierten Brillenglases.

Aspekt 46: Vorrichtung zum Herstellen eines Brillenglases umfassend:

Berechnungs- oder Optimierungsmittel, welche ausgelegt sind, das Brillenglas nach einem Verfahren zum Berechnen oder Optimieren eines Brillenglases gemäß einem der Aspekte 38 bis 40 zu berechnen oder zu optimieren; und
Bearbeitungsmittel, welche ausgelegt ist, das Brillenglas gemäß dem Ergebnis der Berechnung bzw. Optimierung zu bearbeiten.

Aspekt 47: Brillenglas, welches durch ein Verfahren gemäß Aspekt 45 und/oder mittels einer Vorrichtung gemäß Aspekt 46 hergestellt wurde.

**Patentansprüche**

1. Computerimplementiertes Verfahren zum Ermitteln individueller Aberrationsdaten zumindest eines Auges eines Brillenträgers für die Berechnung oder Optimierung und Herstellung eines Brillenglases für das zumindest eine Auge des Brillenträgers umfassend:

   - Bereitstellen einer gemessenen Hornhauttopographie des zumindest einen Auges des Brillenträgers;
   - Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und
   - Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

2. Verfahren nach Anspruch 1, wobei das Ermitteln der individuellen Abbildungseigenschaften der Hornhaut des Auges ein Ermitteln von Werten der Abbildungsfehler höherer Ordnung der Hornhaut, $HOA_C$, umfasst, und wobei das Ermitteln der Abbildungsfehler des Auges ein Ermitteln der Abbildungsfehler höherer Ordnung des Auges, $HOA_{Auge}$, gemäß $HOA_{Auge} = HOA_C + \Delta HOA_{C,Auge}$ mit einer Verschiebung höherer Ordnung, $\Delta HOA_{C,Auge}$, insbesondere mit $\Delta HOA_{C,Auge} = 0$, umfasst.

3. Verfahren nach Anspruch 1 oder 2, wobei das Ermitteln der individuellen Abbildungseigenschaften der Hornhaut des Auges ein Ermitteln von Refraktionswerten niedriger Ordnung der Hornhaut, $LOA_C$, umfasst, und wobei das Ermitteln der Abbildungsfehler des Auges ein Ermitteln von Abbildungsfehlern niedriger Ordnung des Auges, $LOA_{Auge}$, gemäß $LOA_{Auge} = LOA_C + \Delta LOA_{C,Auge}$ mit einer Verschiebung niedriger Ordnung, $\Delta LOA_{C,Auge}$ umfasst, und/oder

   wobei optional das Ermitteln von Refraktionswerten niedriger Ordnung der Hornhaut, $LOA_C$, umfasst:

   ein Ermitteln astigmatischer Anteile der Refraktion der Hornhaut, $LOA_{C,J0}$ und $LOA_{C,J45}$, wobei optional das Ermitteln von Abbildungsfehlern niedriger Ordnung des Auges, $LOA_{Auge}$, ein Ermitteln astigmatischer Anteile der Abbildungsfehler niedriger Ordnung des Auges, $LOA_{Auge,J0}$ und $LOA_{Auge,J45}$, gemäß $LOA_{Auge,J0} = LOA_{C,J0}$ und $LOA_{Auge,J45} = LOA_{C,J45}$ umfasst,
   und/oder
   ein Ermitteln eines sphärischen Anteils der Refraktion der Hornhaut, $LOA_{C,M}$, wobei optional das Ermitteln von Abbildungsfehlern niedriger Ordnung des Auges, $LOA_{Auge}$, ein Ermitteln eines sphärischen Anteils der Abbildungsfehler niedriger Ordnung des Auges, $LOA_{Auge,M}$,

   gemäß $LOA_{Auge,M} = LOA_{C,M} - LOA_{C,M,Std}$ mit einem vorgegebenen Standardwert $LOA_{C,M,Std}$ umfasst,
   oder
   gemäß $LOA_{Auge,M} = LOA_{C,M} + \Delta LOA_{C,Auge,M} (LOA_{C,M})$ mit einer linearen Funktion

   $\Delta LOA_{C,Auge,M}(LOA_{C,M}) = \Delta LOA_{C,Auge,M}(LOA_{C,M,Std}) + \alpha(LOA_{C,M} - LOA_{C,M,Std})$ mit einem vorgegebenen Standardwert $LOA_{C,M,Std}$ und einem vorgegebenen Wert $\alpha$, vorzugsweise im Bereich $5 < \alpha < 15$,

   umfasst.

4. Verfahren nach Anspruch 1 oder 2, wobei das Ermitteln der Abbildungsfehler des Auges ein Ermitteln von Abbildungsfehlern niedriger Ordnung des Auges, $LOA_{Auge}$, anhand von individuellen Refraktionsmessungen an dem zumindest einen Auge umfasst.

5. Verfahren nach einem der vorangegangenen Ansprüche 1 bis 4, wobei das Ermitteln von Abbildungsfehlern des Auges umfasst:

   - Festlegen eines individuellen Augenmodells, in welchem eine Form einer Hornhautvorderfläche (18) eines Modellauges (12) der gemessenen Hornhauttopographie entspricht und in welchem außerdem zumindest

     -- ein Hornhaut-Linsen-Abstand;
     -- Parameter einer Linse des Modellauges; und

-- ein Linsen-Netzhaut-Abstand

derart anhand von individuellen Messwerten für das Auge des Brillenträgers und/oder von Standardwerten und/oder anhand der ermittelten Abbildungsfehler niedriger Ordnung des Auges festgelegt werden, dass das Modellauge (12) die ermittelten Abbildungsfehler niedriger Ordnung des Auges aufweist; und
- Ermitteln einer Aberration einer nach einer Brechung an der Hornhautvorderfläche des Modellauges und einer Propagation durch das Modellauge in einem Punkt auf der Netzhaut konvergierenden Wellenfront,

wobei optional das Festlegen der Parameter der Linse des Modellauges ein Festlegen folgender Parameter umfasst:

-- Form der Linsenvorderfläche;
-- Linsendicke; und
-- Form der Linsenrückfläche,

und/oder
wobei optional das Festlegen von Parametern der Linse des Modellauges, insbesondere das Festlegen der Linsendicke, und/oder des Linsen-Netzhaut-Abstands anhand von vorgegebenen Standardwerten erfolgt,
und/oder
wobei optional das Festlegen von Parametern der Linse des Modellauges, insbesondere der Form der Linsenvorderfläche und/oder der Linsenrückfläche anhand von vorgegebenen Standardwerten für die Abbildungsfehler höherer Ordnung der jeweiligen Fläche erfolgt, wobei optional die Standardwerte der Abbildungsfehler höherer Ordnung der Linsenvorderfläche und/oder der Linsenrückfläche auf null gesetzt werden.

6. Verfahren nach Anspruch 5, wobei das Ermitteln der individuellen Abbildungseigenschaften der Hornhaut des Auges ein Ermitteln von Refraktionswerten niedriger Ordnung der Hornhaut, $LOA_C$, umfasst und wobei das Festlegen des Linsen-Netzhaut-Abstands und/oder von Parametern der Linse des Modellauges, insbesondere der Linsendicke und/oder der Form der Linsenvorderfläche und/oder der Linsenrückfläche, anhand der ermittelten Refraktionswerte niedriger Ordnung der Hornhaut, $LOA_C$, erfolgt,
oder
wobei das Ermitteln der Abbildungsfehler des Auges ein Ermitteln von Abbildungsfehlern niedriger Ordnung des Auges, $LOA_{Auge}$, umfasst und wobei das Festlegen des Linsen-Netzhaut-Abstands und/oder von Parametern der Linse des Modellauges, insbesondere der Linsendicke und/oder der Form der Linsenvorderfläche und/oder der Linsenrückfläche, anhand der ermittelten Abbildungsfehler niedriger Ordnung des Auges, $LOA_{Auge}$, erfolgt.

7. Computerimplementiertes Verfahren zum Ermitteln optimierter sphärozylindrischer Werte für zumindest ein Auge eines Brillenträgers, wobei das Verfahren umfasst:

- Ermitteln von subjektiven sphärozylindrischen Refraktionswerten;
- Ermitteln von objektiven sphärozylindrischen Refraktionswerten, welches umfasst:

-- Bereitstellen individueller Aberrationsdaten, welche mittels eines Verfahrens nach einem der Ansprüche 1 bis 6 ermittelt wurden;
-- Ermitteln einer Referenzwellenfront an einer Bewertungsfläche anhand der bereitgestellten individuellen Aberrationsdaten des Auges;
-- Vorgeben einer Startvorgabe für eine zu optimierende Wellenfront, welche zu optimierende, objektive sphärozylindrische Refraktionswerte beschreibt, an der Bewertungsfläche;
-- Ermitteln einer Differenzwellenfront aus der zu optimierenden Wellenfront und der Referenzwellenfront;
-- Bewerten der Differenzwellenfront anhand einer vorgestimmten Metrik;
-- Ermitteln der zu optimierenden Wellenfront derart, dass die Bewertung der Differenzwellenfront vorbestimmte Sollkriterien erfüllt; und
-- Ermitteln der objektiven sphärozylindrischen Refraktionswerte aus der ermittelten zu optimierenden Wellenfront; und

- Ermitteln der optimierten sphärozylindrischen Werte als gewichteter Mittelwert aus den ermittelten subjektiven sphärozylindrischen Refraktionswerten und den ermittelten objektiven sphärozylindrischen Refraktionswerten.

8. Computerimplementiertes Verfahren zum Berechnen oder Optimieren eines Brillenglases für zumindest ein Auge eines Brillenträgers umfassend:

- Bereitstellen individueller Aberrationsdaten, welche mittels eines Verfahrens nach einem der Ansprüche 1 bis 6 ermittelt wurden;
- Ermitteln einer Referenzaberration an einer Bewertungsfläche anhand der bereitgestellten individuellen Aberrationsdaten des Auges;
- Vorgeben einer ersten Fläche (14) und einer zweiten Fläche (16) für das zu berechnende bzw. optimierende Brillenglas;
- Ermitteln des Verlaufs eines Hauptstrahls (10) durch zumindest einen Durchblickspunkt (i) zumindest einer zu berechnenden oder optimierenden Fläche (14; 16) des Brillenglases;
- Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration;
- iteratives Variieren der zumindest einen zu berechnenden oder optimierenden Fläche (14; 16) des Brillenglases bis die ausgewertete Aberration einer vorgegebenen Sollaberration entspricht,

wobei optional die Bewertungsfläche an der Scheitelpunktkugel liegt.

9. Computerimplementiertes Verfahren zum Berechnen oder Optimieren eines Brillenglases für zumindest ein Auge eines Brillenträgers umfassend:

- Ermitteln optimierter sphärozylindrischer Werte für zumindest ein Auge eines Brillenträgers auf Basis individueller Aberrationsdaten, welche mittels eines Verfahrens nach einem der Ansprüche 1 bis 6 ermittelt wurden, insbesondere mittels eines Verfahrens nach Anspruch 7;
- Festlegen bzw. Ermitteln einer Kombination aus Vorder- und Rückfläche als Startfläche bzw. auf Basis der ermittelten optimierten sphärozylindrischen Werte; und
- vorzugsweise Modifizieren der festgelegten bzw. ermittelten Vorderfläche und/oder Rückfläche auf Basis der Basis der ermittelten optimierten sphärozylindrischen Werte.

10. Vorrichtung zum Ermitteln individueller Aberrationsdaten zumindest eines Auges eines Brillenträgers umfassend:

- eine Datenschnittstelle zum Bereitstellen einer gemessenen Hornhauttopographie des zumindest einen Auges des Brillenträgers;
- ein Hornhautauswertemodul zum Ermitteln individueller Abbildungseigenschaften der Hornhaut des Auges, welche zumindest Abbildungsfehler höherer Ordnung der Hornhaut, $HOA_C$, beschreiben, aus der gemessenen Hornhauttopographie; und
- ein Berechnungsmodul zum Ermitteln von Abbildungsfehlern des Auges, welche zumindest Abbildungsfehler höherer Ordnung des Auges beschreiben, derart, dass zumindest die Abbildungsfehler höherer Ordnung des Auges, $HOA_{Auge}$, unter Berücksichtigung der ermittelten individuellen Abbildungseigenschaften der Hornhaut des Auges ermittelt werden.

11. Vorrichtung zum Berechnen oder Optimieren eines Brillenglases für zumindest ein Auge eines Brillenträgers umfassend:

- eine Datenschnittstelle zum Bereitstellen individueller Aberrationsdaten, welche mittels eines Verfahrens nach einem der Ansprüche 1 bis 6 ermittelt wurden;
- ein Modellierungsmodul zum Ermitteln einer Referenzaberration an einer Bewertungsfläche anhand der bereitgestellten individuellen Aberrationsdaten des Auges;
- eine Flächenmodelldatenbank zum Vorgeben einer ersten Fläche (14) und einer zweiten Fläche (16) für das zu berechnende bzw. optimierende Brillenglas;
- ein Hauptstrahlermittlungsmodul zum Ermitteln des Verlaufs eines Hauptstrahls (10) durch zumindest einen Durchblickspunkt (i) zumindest einer zu berechnenden oder optimierenden Fläche (14; 16) des Brillenglases;
- ein Auswertemodul zum Auswerten einer Aberration einer entlang des Hauptstrahls aus einer auf die erste Fläche des Brillenglases auftreffenden sphärischen Wellenfront resultierenden Wellenfront an der Bewertungsfläche im Vergleich zur ermittelten Referenzaberration; und
- ein Optimierungsmodul zum iterativen Variieren der zumindest einen zu berechnenden oder optimierenden Fläche (14; 16) des Brillenglases bis die ausgewertete Aberration einer vorgegebenen Sollaberration entspricht.

12. Computerprogrammerzeugnis, welches Programmcode enthält, der ausgelegt ist, wenn geladen und ausgeführt auf einem Computer, ein Verfahren zum Ermitteln individueller Aberrationsdaten zumindest eines Auges eines Brillenträgers gemäß einem der Ansprüche 1 bis 6 und/oder ein Verfahren zum Ermitteln optimierter sphärozylindrischer Werte für zumindest ein Auge eines Brillenträgers gemäß Anspruch 7 und/oder ein Verfahren zum Berechnen oder Optimieren eines Brillenglases gemäß Anspruch 8 oder 9 durchzuführen.

13. Verfahren zum Herstellen eines Brillenglases umfassend:

Berechnen oder Optimieren eines Brillenglases nach dem Verfahren zum Berechnen oder Optimieren eines Brillenglases gemäß Anspruch 8 oder 9; und
Fertigen des so berechneten oder optimierten Brillenglases.

14. Vorrichtung zum Herstellen eines Brillenglases umfassend:

Berechnungs- oder Optimierungsmittel, welche ausgelegt sind, das Brillenglas nach einem Verfahren zum Berechnen oder Optimieren eines Brillenglases gemäß Anspruch 8 oder 9 zu berechnen oder zu optimieren; und
Bearbeitungsmittel, welche ausgelegt ist, das Brillenglas gemäß dem Ergebnis der Berechnung bzw. Optimierung zu bearbeiten.

15. Brillenglas, welches durch ein Verfahren gemäß Anspruch 8 oder 9 und/oder mittels einer Vorrichtung gemäß Anspruch 11 berechnet oder optimiert ist,
und/oder.
welches durch ein Verfahren gemäß Anspruch 13 und/oder mittels einer Vorrichtung gemäß Anspruch 14 hergestellt wurde.

FIG 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10313275
- WO 2008089999 A1
- WO 2013104548 A1
- DE 102007032564 A1

**In der Beschreibung aufgeführte Nicht-Patentliteratur**

- **ROLAND ENDERS.** Die Optik des Auges und der Sehhilfen. Optische Fachveröffentlichung GmbH, 1995, 25
- **DIEPES, BLENDOWSKE.** Optik und Technik der Brille. Optische Fachveröffentlichung GmbH, 2002, 47
- **G. ESSER ; W. BECKEN ; W. MÜLLER ; P. BAUMBACH ; J. ARASA ; D. UTTENWEILER.** Derivation of the Refraction Equations for Higher Order Aberrations of Local Wavefronts at Oblique Incidence. *JOSA A,* 2010, vol. 27 (2), 218-37
- **G. ESSER ; W. BECKEN ; W. MÜLLER ; P. BAUMBACH ; J. ARASA ; D. UTTENWEILER.** Derivation of the Propagation Equations for Higher Order Aberrations of Local Wavefronts. *JOSA A,* 2011, vol. 28 (12), 2442-58
- **G. ESSER et al.** Derivation of the refraction equations for higher order aberrations of local wavefronts at oblique incidence. *JOSA A,* 2010, vol. 27 (2
- **VON G. ESSER et al.** Derivation of the propagation equations for higher order aberrations of local wavefronts. *JOSA A,* 2011, vol. 28 (11
- **G. ESSER et al.** Derivation of the propagation equations for higher order aberrations of local wavefronts. *JOSA A,* 2011, vol. 28 (11
- **T. O. SALMON ; C. VAN DE POL.** Normal-eye Zernike coefficients and root-mean-square wavefront errors. *J Cataract Refract Surg,* 2006, vol. 32, 2064-2074
- **J. PORTER et al.** Monochromatic aberrations of the human eye in a large population. *JOSA A,* 2001, vol. 18 (8
- **M. KASCHKE et al.** Optical Devices in Ophthalmology and Optometry. Wiley-VCH, 2014
- **R. B. RABBETTS.** Bennett & Rabbetts' Clinical Visual Optics. Butterworth Heinemann Elsevier Health Sciences, 2007